# EUROPEAN PATENT APPLICATION

(11) **EP 3 705 580 A1**
(43) Date of publication of application: **09.09.2020**
(21) Application number: 18874137.5
(22) Date of filing: 02.11.2018
(51) Int. Cl.: C12Q 1/06, C12Q 1/68, G01N 37/00, C12N 15/09

(54) **INTRA-ORAL INSPECTION METHOD USING INFORMATION FOR BACTERIA GROUPS ASSOCIATED WITH CLINICAL INDICES**

(30) Priority: 02.11.2017 JP 2017212488
(71) Applicant: Mitsubishi Chemical Corporation, Tokyo 100-8251 (JP)
(72) Inventor: TOGAWA Naoyuki, Tokyo 100-8251 (JP); HARA Ai, Tokyo 100-8251 (JP); MURAKAMI Shinya, Suita-shi Osaka 565-0871 (JP); NOZAKI Takenori, Suita-shi Osaka 565-0871 (JP)
(74) Representative: Hoffmann Eitle
(86) International application number: PCT/JP2018/040917
(87) International publication number: WO 2019/088272

(57) **Abstract**

An intraoral examination method for determining the state of periodontal disease is provided. The method is an intraoral examination method for measuring a signal intensity of a nucleic acid from an oral bacterial group present in an oral sample, calculating an abundance of the bacterial group from a measured value of the signal intensity, and determining the state of periodontal disease using the obtained calculated value as an index, wherein the abundance of the bacterial group shows a correlation between a bacterial load of a bacterial species that increases as a periodontal pocket value increases and a bacterial load of a bacterial species that decreases as a periodontal pocket value increases.

## Description

### Technical Field

The present invention relates to an intraoral examination method for determining the state of periodontal disease by using information on a bacterial group related to clinical indexes.

### Background Art

Periodontal disease has an aspect of a bacterial infection that involves a plurality of bacteria, and an aspect of a multifactorial disease that progresses with the involvement of causative bacteria (bacterial factors), immunity (host factors), and lifestyle habits. Periodontopathic bacteria are involved in the onset of the disease.

Porphyromonas gingivalis, Tannerella forsythensis, Treponema denticola, Campylobacter rectus, Fusobacterium nucleatum, Prevotella intermedia, Aggregatibacter actinomycetemcomitans, and the like have been reported as periodontopathic bacteria (Non Patent Literature 1 and 2). Of these, three bacterial species, namely Porphyromonas gingivalis, Tannerella forsythensis, and Treponema denticola, are called "red complex" and are regarded as important causative bacteria of chronic periodontitis. It is known that the presence of the "red complex" increases the malignancy of periodontal disease, and the bacteria that consist of the "red complex" are considered to be clinically important bacteria.

In addition to the above, Aggregatibacter actinomycetemcomitans has been reported as a causative bacterium of invasive periodontitis, and Prevotella intermedia has been reported as adolescent or gestational periodontitis (Non Patent Literature 1 and 2).

As a conventional bacterial test for periodontal disease, it has been reported that there is a relationship between the total value of three types of "red complex" bacteria in plaque or saliva and the state (progression degree) of periodontal disease. Specifically, it is determined when the bacterial count of at least one of Porphyromonas gingivalis, Tannerella forsythensis, and Treponema denticola with respect to the total bacterial count is less than 0.5%, the degree of relationship is "low," when it is 0.5% or more and less than 5%, the degree of relationship is "medium," and when it is 0.5% or more, the degree of relationship is "high" (Non Patent Literature 3).

As a method for detecting periodontopathic bacteria and calculating the bacterial count, for example, methods using a culture method, real-time PCR, a next-generation sequencer, and a DNA microarray have been reported. More specifically, there is also a report of individually detecting the number of Porphyromonas gingivalis cells and/or Tannerella forsythensis (formerly Bacteroides forsythus) cells in saliva using a real-time PCR method (Patent Literature 1,3, and 4).

In addition, a T-RFLP method has also been reported, in which the genomic DNA of the bacterial flora is recovered, treated with a restriction enzyme, and the bacterial flora is recognized from the information of the fragmented DNA using the degree of pattern similarity as an index (Patent Literature 5). The report identifies patterns from the bacterial flora which correlate with dental clinical indexes. However, information on the specific bacterial count of each individual bacterium was not included, and no further interpretation was reached. The T-RFLP method can represent the composition of a bacterial community as a peak pattern and can easily perform comparative analysis of a plurality of samples. On the other hand, since each peak is not necessarily derived from one bacterial species, the bacterial community composition is difficult to understand (Non Patent Literature 4).

In recent years, the use of next-generation sequencers has been mentioned as a method for understanding the composition of a bacterial group. For example, Patent Literature 9 provides a method for testing salivary bacterial flora by randomly determining the base sequences of 16S ribosomal RNA genes of bacterial flora in a saliva specimen collected from a human subject. This test method proposes a method for detecting inflammatory bowel disease by saliva. As a result of UniFrac analysis, the healthy group and the CD patient group can be distinguished, while on the other hand, the t-test is repeated for each individual bacterium to perform a significant difference test, which makes the analysis process questionable.

In addition, as a method for detecting the bacterial loads of all bacteria, i.e., a method for detecting the total bacterial load, there is a report of using a universal primer that selects a highly conserved region between microorganisms (Patent Literature 6 to 8). In an example using a DNA microarray, there is a report that 20 types of oral bacteria were detected by setting one set of universal primers in the PCR step of sample preparation. (Non Patent Literature 5 to 8).

Up to now, many examples have been reported, in which the bacterial count of at least one "red complex" bacterium is measured and used as an index of periodontal disease severity. However, since periodontal disease is a disease caused by a plurality of bacteria, only the same information as that obtained by the periodontal pocket measurement was obtained in the measurement of a limited variety of malignant bacteria. In addition, regarding indexes for determining therapeutic effects of periodontal disease, the therapeutic effects are basically determined based on clinical information obtained through the experience of dentists, and in most cases, information on the bacterial flora has not been confirmed.

Furthermore, there was no index of periodontal disease deterioration in the early stage of periodontal disease. Therefore, periodontal disease has already progressed by the time that many patients notice that they have periodontal disease, and even if they start treatment after they notice the symptoms of periodontal disease, the treatment is not effective in many cases. Therefore, there was also a need for an effective method for predicting the deterioration of periodontal disease. The following are specific examples.
"Supportive periodontal therapy (SPT)," which is continuous professional care after periodontal treatment, is an essential treatment for keeping a "stable condition" and maintaining a favorable prognosis of periodontal treatment. Currently, the criteria for transition to SPT in clinical settings are probing pocket depth (PPD), bleeding on probing (BOP), and the like. Meanwhile, if there is an index for predicting the progress after the transition to SPT in the future, it will be especially useful in determining a treatment plan, but no clear criteria have been established at present (Non Patent Literature 9).

Under such circumstances, as an attempt to set criteria, a method using a combination of alanine aminotransferase (ALT) and the proportion of the bacterial count of P. gingivalis with respect to the total bacterial count of saliva and a method for measuring the proportion of P. gingivalis in saliva and the serum antibody titer against P. gingivalis during re-evaluation of treatment in combination have been examined (Non Patent Literature 10). Further, oral bacterial tests are also expected as predictive indexes (Non Patent Literature 11). For example, Haffajee et al. reported there is a relationship between the bacterial counts of A. actinomycetemcomitans and P. gingivalis and the risk of attachment loss of 2 mm or more (Non Patent Literature 1 and 2). However, as mentioned above, the number of bacterial species tested is limited, and such tests have not yet been put to practical use as predictive indexes.

### Citation List

### Patent Literature

Patent Literature 1: JP Patent No. 4252812
Patent Literature 2: JP Patent Publication (Kokai) No. 2008-206516 A
Patent Literature 3: JP Patent Publication (Kokai) No. 2004-229537 A
Patent Literature 4: WO2002/010444
Patent Literature 5: JP Patent Publication (Kokai) No. 2011-193810 A
Patent Literature 6: WO03/106676
Patent Literature 7: JP Patent Publication (Kohyo) No. 2004-504069 A
Patent Literature 8: JP Patent Publication (Kokai) No. 2007-068431 A
Patent Literature 9: JP Patent No. 5863035

### Non Patent Literature

Non Patent Literature 1: Socransky, S. S. et al. J ClinMicrobiol, 37, 1426-30, 1999
Non Patent Literature 2: "Concept of Periodontal Treatment Using Bacterial Test" (in Japanese), edited by Igaku Joho-sha, Ltd.: Masato Minabe, Toshiaki Yoshino, p. 3, published in June 2005
Non Patent Literature 3: "Clinical Guidelines for Antibacterial Therapy in Patients with Periodontal Disease" (in Japanese), edited by the Japanese Society of Periodontology
Non Patent Literature 4: "Human Health and Diseases Controlled by Indigenous Bacterial Flora" (in Japanese), edited by YODOSHA CO., LTD.: Hiroshi Ohno, Syohei Hattori, p. 97, published in March 2014
Non Patent Literature 5: Eberhard, J. et al. Oral Microbiol Immunol 2008; 23: 21-8
Non Patent Literature 5: Shang, S et al. Pediatric Research 2005; 58: 143-148
Non Patent Literature 6: Topcuoglu, N. et al. J Clin Pediatr Dent 2013; 38: 155-60
Non Patent Literature 7: Topcuoglu, N. et al. Anaerobe2015; 35: 35-40
Non Patent Literature 8: Henne, K. et al. J Oral Microbiol 2014; 6: 25874
Non Patent Literature 9: Guidelines by the Japanese Society of Periodontology, "JSP Clinical Practice Guideline for the Periodontal Treatment, 2015"
Non Patent Literature 10: "Predicting Periodontitis Progression from Salivary Bacterial Test and Serum Antibody Titer Test" (in Japanese): Journal of the Japanese Society of Periodontology, vol. 58 (2016) No. 4, pp. 254-258
Non Patent Literature 11: "Possibility of Bacterial Test in Periodontal Disease" (in Japanese): Journal of the Japanese Society of Periodontology, vol. 55 (2013), No. 4, pp. 294-299
Non Patent Literature 12: Haffajee AD, Socransky SS. Microbial etiological agents of destructive periodontal diseases. Periodontol 2000. 1994 Jun; 5:78-111. Review.

### Summary of Invention

### Technical Problem

As described above, currently, there is still insufficiency in information for determining predictive indexes and treatment policy of periodontal disease deterioration. Therefore, an object of the present invention is to provide a method for determining the state of periodontal disease and therapeutic effects of periodontal disease by detecting and quantifying oral bacteria in detail by a simple method, and the like.

### Solution to Problem

As a result of intensive studies to solve the above problems, the present inventors found that the state of periodontal disease can be determined by determining the abundance ratio between specific bacteria (groups) regarding the bacteria present in an oral sample. This has led to the completion of the present invention. Further, the present inventors found that pathological conditions with the same periodontal pocket value can be further subdivided and classified by collectively measuring major oral bacterial groups (including a periodontal disease-related bacterial group and an indigenous bacterial group) in plaque and creating a model for determining deterioration of pathological conditions based on the abundance ratio of the periodontal disease-related bacterial group and the indigenous bacterial group.

Furthermore, the present inventors found that therapeutic effects on periodontal disease, progress of periodontal disease, and the like can be determined by obtaining the abundance ratio between specific bacteria (groups) present in an oral sample. This has also led to the completion of the present invention.

Specifically, the present invention is as follows.
[1] An intraoral examination method for measuring a signal intensity of a nucleic acid from an oral bacterial group present in an oral sample, calculating an abundance of the bacterial group from a measured value of the signal intensity, and determining a state of periodontal disease using the obtained calculated value as an index, wherein
   an abundance ratio of bacterial groups shows a correlation between a bacterial load of a bacterial species that increases as a periodontal pocket value increases and a bacterial load of a bacterial species that decreases as a periodontal pocket value increases.
[2] The method according to [1], wherein the state of periodontal disease is determined by comparing the obtained calculated value with a cut-off value of the abundance ratio of bacterial groups.
[3] The method according to [1] or [2], wherein the abundance ratio of bacterial groups is a ratio of the bacterial load of the bacterial species that increases as the periodontal pocket value increases and the bacterial load of the bacterial species that decreases as the periodontal pocket value increases.
[4] The method according to [2] or [3], wherein the cut-off value is determined based on an ROC curve created from a calculated value obtained by calculating the abundance ratio of bacterial groups from the measured value of the signal intensity of the nucleic acid from the oral bacterial group present in the oral sample for standardization.
[5] The method according to any of [1] to [4], wherein the abundance ratio of bacterial groups shows a correlation between the bacterial load of Fusobacterium nucleatum species and the bacterial load of the bacterial species that decreases as the periodontal pocket value increases.
[6] The method according to any one of [1] to [5], wherein the following (a) and (b) are used as the abundance ratio of bacterial groups:
   (a) a correlation between the bacterial load of the bacterial species that increases as the periodontal pocket value increases (including at least one bacterial species other than Fusobacterium nucleatum species) and the bacterial load of the bacterial species that decreases as the periodontal pocket value increases; and
   (b) a correlation between the bacterial load of Fusobacterium nucleatum species and the bacterial load of the bacterial species that decreases as the periodontal pocket value increases.
[7] The method according to any one of [1] to [6], wherein the bacterial species that increases as the periodontal pocket value increases is at least one selected from the group consisting of Porphyromonas gingivalis, Tannerella forsythia, Treponema denticola, Campylobacter gracilis, Campylobacter rectus, Campylobacter showae, Fusobacterium nucleatum subsp. vincentii, Fusobacterium nucleatum subsp. polymorphum, Fusobacterium nucleatum subsp. animalis, Fusobacterium nucleatum subsp. nucleatum, Fusobacterium periodonticum, Prevotella intermedia, Streptococcus constellatus, Aggregatibacter actinomycetemcomitans, Eikenella corrodens, Filifactor alocis, Porphyromonas endodontalis, Eubacterium nodatum, Eubacterium saphenum, Treponema medium, and Selenomonas sputigena.
[8] The method according to any one of [1] to [7], wherein bacterial species that decreases as the periodontal pocket value increases is at least one selected from the group consisting of Prevotella nigrescens, Campylobacter concisus, Capnocytophaga gingivalis, Capnocytophaga ochracea, Capnocytophaga sputigena, Streptococcus gordonii, Streptococcus intermedius, Streptococcus mitis, Streptococcus mitis bv 2, Actinomyces odontolyticus, Veillonella parvula, Actinomyces naeslundii II, Selenomonas noxia, Prevotella denticola, Prevotella melaninogenica, Gemella sanguinis, Eubacterium sulci, Corynebacterium matruchotii, Rothia mucilaginosa, Porphyromonas catoniae, Solobacterium moorei, Neisseria flavescens, Prevotella loescheii, Megasphaera micronuciformis, Actinomyces graevenitzii, Veillonella atypica, Prevotella pallens, Prevotella shahii, Porphyromonas pasteri, Veillonella rogosae, Alloprevotella spp. (A. rava, OT 308), Rothia dentocariosa, Granulicatella adiacens, Streptococcus salivarius, Haemophilus parainfluenzae, and Streptococcus parasanguinis.
[9] The method according to any one of [5] to [8], wherein the Fusobacterium nucleatum species is at least one selected from the group consisting of Fusobacterium nucleatum subsp. vincentii, Fusobacterium nucleatum subsp. polymorphum, Fusobacterium nucleatum subsp. animalis, and Fusobacterium nucleatum subsp. nucleatum.
[10] An intraoral examination method, characterized by measuring a signal intensity of a nucleic acid from an oral bacterial group present in an oral sample, calculating an abundance of the bacterial group from a measured value of the signal intensity, and correlating the obtained calculated value with a state or progression of periodontal disease or therapeutic effects on periodontal disease.
[11] The method according to [10], wherein an abundance ratio of bacterial groups and a state of periodontal disease are correlated by calculating a ratio of a bacterial species that increases as a periodontal pocket value increases to a bacterial species that decreases as a periodontal pocket value increases and/or a bacterial species that increases as a periodontal pocket value decreases.
[12] The method according to [10] or [11], wherein an abundance ratio of bacterial groups and progression of periodontal disease are correlated by calculating a ratio of a bacterial species belonging to the genus Fusobacterium that increases as a periodontal pocket value increases to a bacterial species that decreases as a periodontal pocket value increases and/or a bacterial species that increases as a periodontal pocket value decreases.
[13] The method according to any one of [10] to [12], wherein an abundance ratio of bacterial groups and therapeutic effects on periodontal disease are correlated by comparing the following values of (a) and/or (b) before and after periodontal treatment:
   (a) a ratio of a bacterial species that increases as a periodontal pocket value increases and a bacterial species that decreases as a periodontal pocket value increases and/or a bacterial species that increases as a periodontal pocket value decreases; and
   (b) a ratio of a bacterial species belonging to the genus Fusobacterium that increases as a periodontal pocket value increases and a bacterial species that decreases as a periodontal pocket value increases and/or a bacterial species that increases as a periodontal pocket value decreases.
[14] The method according to [12] or [13], wherein the bacterial species belonging to the genus Fusobacterium is at least one selected from the group consisting of Fusobacterium nucleatum subsp. vincentii, Fusobacterium nucleatum subsp. polymorphum, Fusobacterium nucleatum subsp. animalis, Fusobacterium nucleatum subsp. nucleatum, and Fusobacterium periodonticum.
[15] The method according to any one of [11] to [14], wherein the bacterial species that increases as a periodontal pocket value increases is at least one selected from the group consisting of Porphyromonas gingivalis, Tannerella forsythia, Treponema denticola, Campylobacter gracilis, Campylobacter rectus, Campylobacter showae, Fusobacterium nucleatum subsp. vincentii, Fusobacterium nucleatum subsp. polymorphum, Fusobacterium nucleatum subsp. animalis, Fusobacterium nucleatum subsp. nucleatum, Fusobacterium periodonticum, Prevotella intermedia, Streptococcus constellatus, Aggregatibacter actinomycetemcomitans, and Eikenella corrodens, and wherein the bacterial species that decreases as the periodontal pocket value increases and/or bacterial species that increases as the periodontal pocket value decreases is at least one selected from the group consisting of Prevotella nigrescens, Campylobacter concisus, Capnocytophaga gingivalis, Capnocytophaga ochracea, Capnocytophaga sputigena, Streptococcus gordonii, Streptococcus intermedius, Streptococcus mitis, Streptococcus mitis bv 2, Actinomyces odontolyticus, Veillonella parvula, Actinomyces naeslundii II, and Selenomonas noxia.
[16] The method according to any one of [12] to [15], wherein the bacterial species belonging to the genus Fusobacterium is at least one selected from the group consisting of Fusobacterium nucleatum subsp. vincentii, Fusobacterium nucleatum subsp. polymorphum, Fusobacterium nucleatum subsp. animalis, Fusobacterium nucleatum subsp. nucleatum, and Fusobacterium periodonticum, and wherein the bacterial species that decreases as the periodontal pocket value increases and/or bacterial species that increases as the periodontal pocket value decreases is at least one selected from the group consisting of Prevotella nigrescens, Campylobacter concisus, Capnocytophaga gingivalis, Capnocytophaga ochracea, Capnocytophaga sputigena, Streptococcus gordonii, Streptococcus intermedius, Streptococcus mitis, Streptococcus mitis bv 2, Actinomyces odontolyticus, Veillonella parvula, Actinomyces naeslundii II, and Selenomonas noxia.

### Advantageous Effects of Invention

According to the present invention, a large number of oral bacteria (including periodontal disease-related bacteria and indigenous bacteria) can be collectively detected and quantified, and the state of periodontal disease can be subdivided so as to be determined as compared with conventional methods. Further, according to the present invention, the state of periodontal disease, the therapeutic effects on periodontal disease, the progression of periodontal disease can be determined, and the pathological conditions having the same periodontal pocket size can be further subdivided and classified. In addition, it is possible to determine the therapeutic effects and the stable condition without the periodontal pocket value. Furthermore, in the future, it will be also possible to improve the discriminant model performance by replacing bacterial species used for determination.

### Brief Description of Drawings

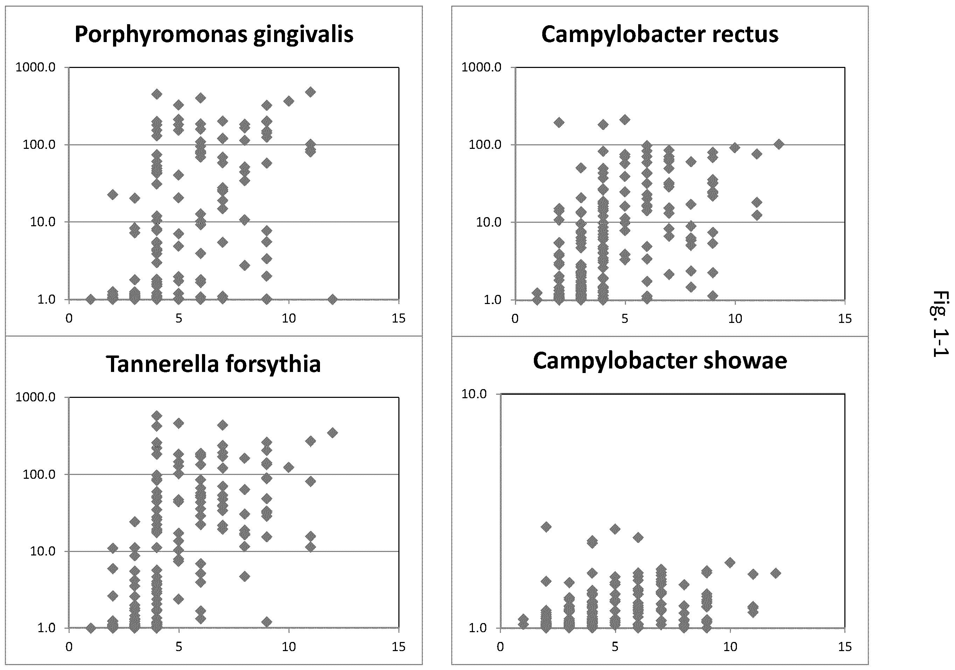

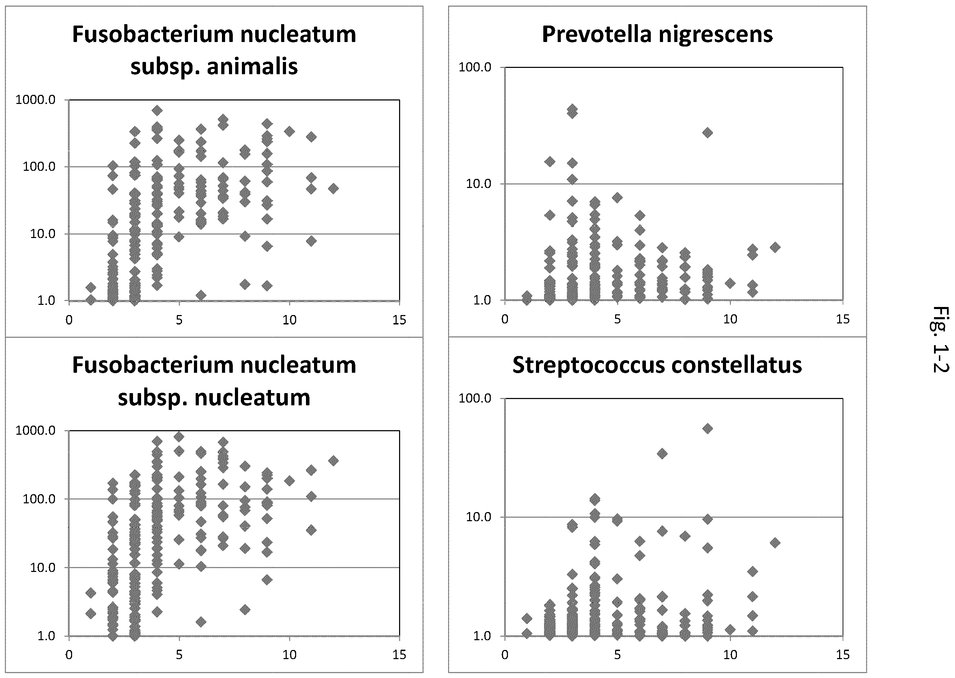

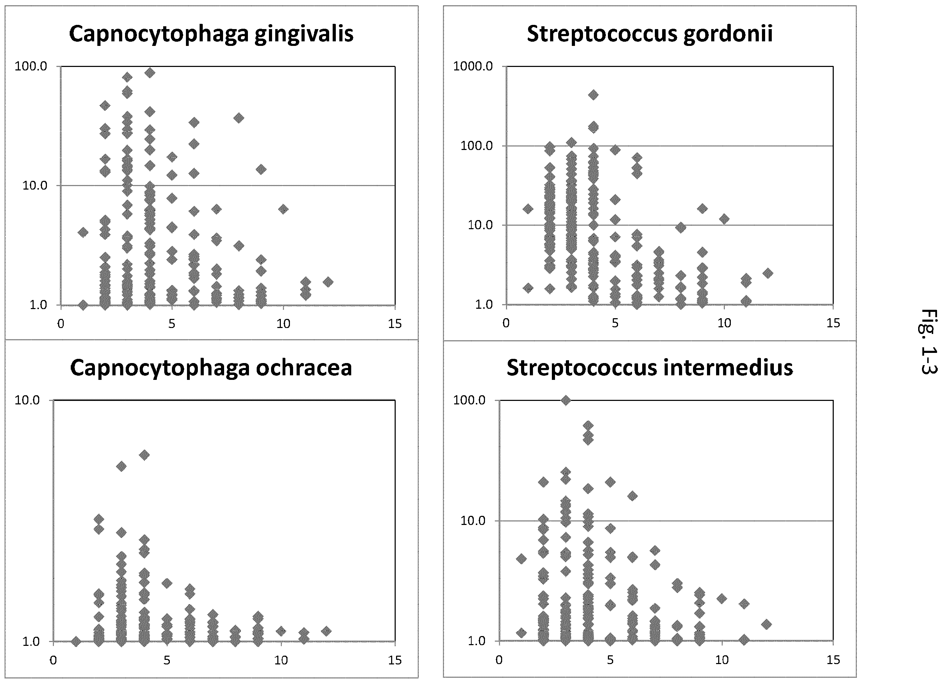

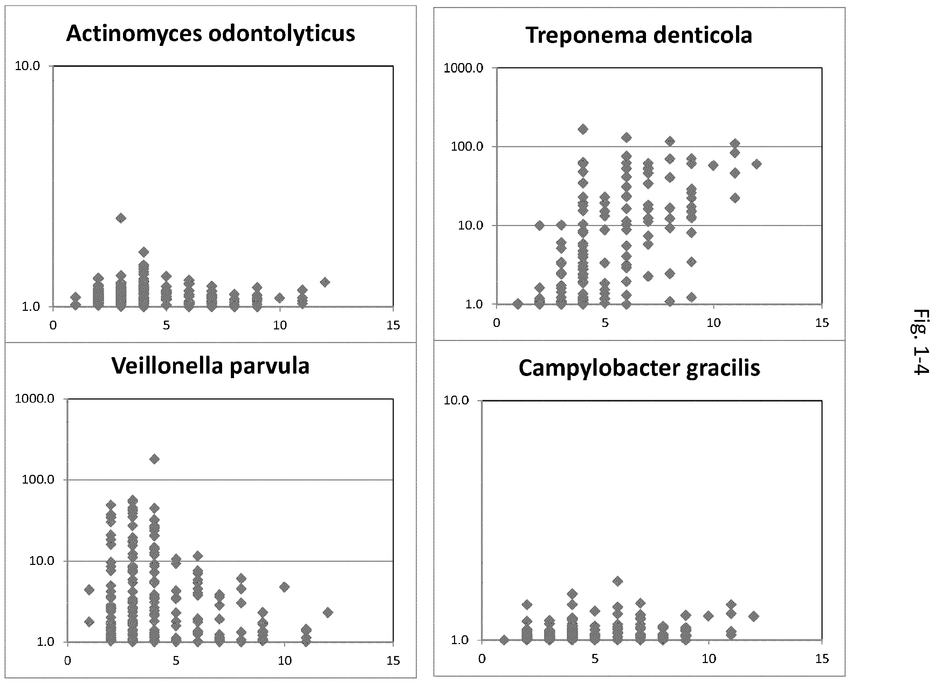

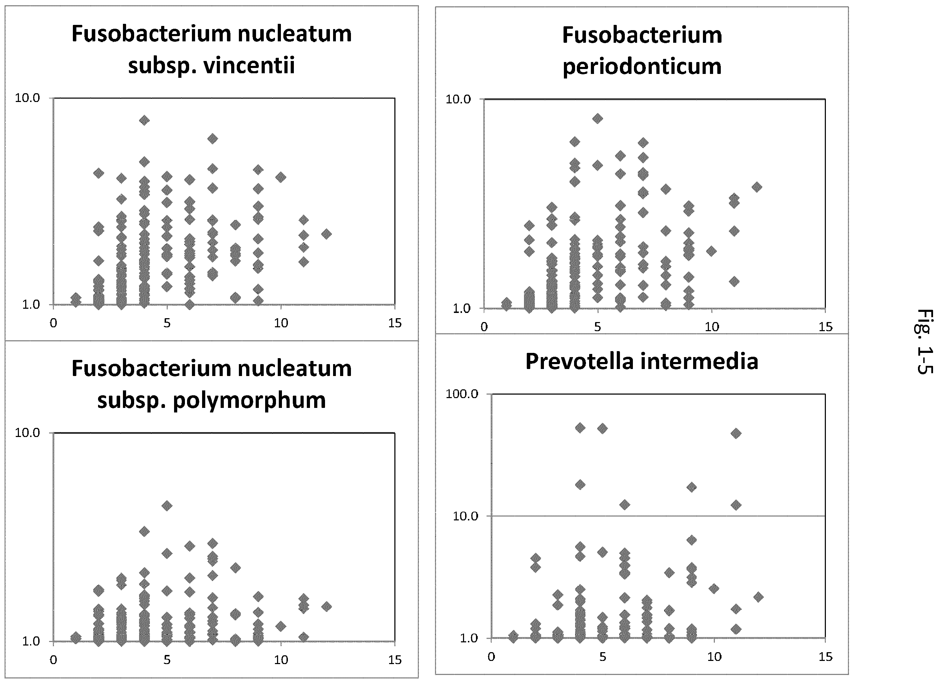

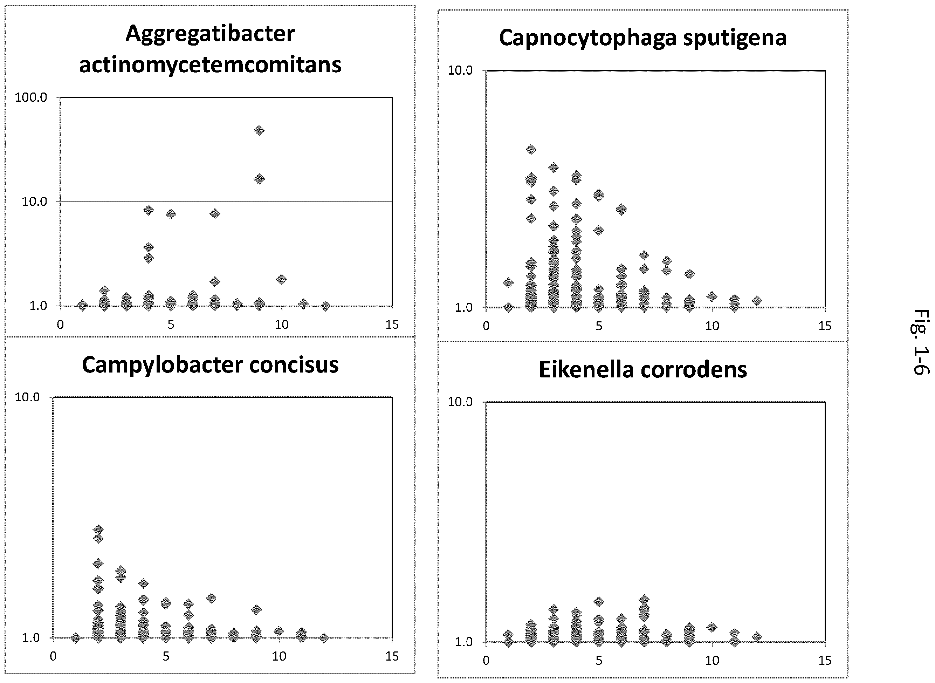

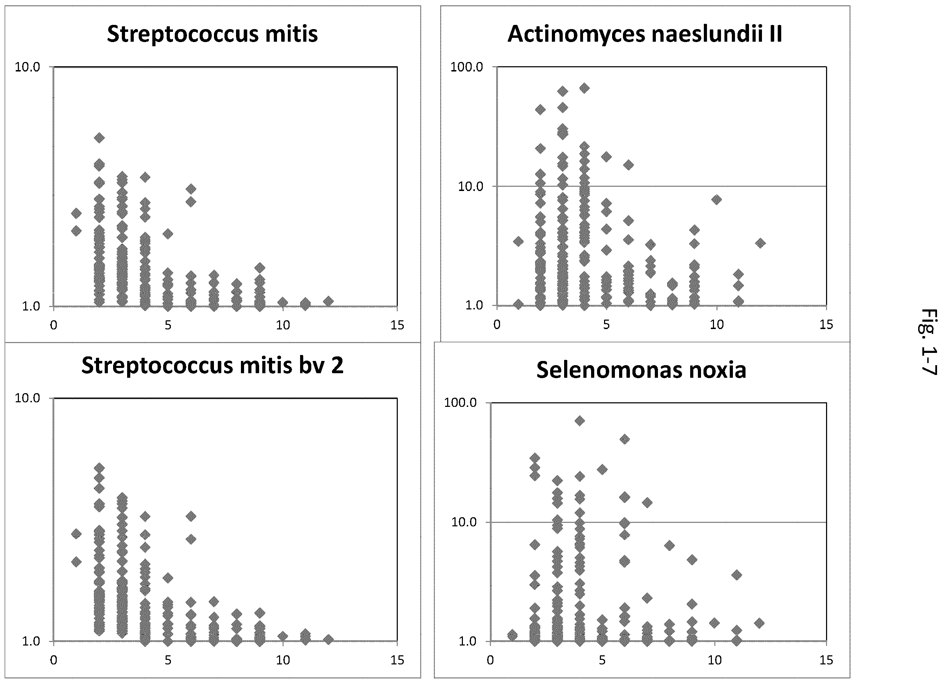

[Figure 1-1] Figure 1-1 is a scatter diagram of DNA chip measurement data (SN ratio) of subgingival plaque collected from 220 male and female subjects in their 20s to 70s before treatment of periodontal disease (vertical axis) and the periodontal pocket depth (Pd) (horizontal axis). Each diagram was described for individual 28 types of bacteria mounted on a DNA chip.
[Figure 1-2] Figure 1-2 is a scatter diagram of DNA chip measurement data (SN ratio) of subgingival plaque collected from 220 male and female subjects in their 20s to 70s before treatment of periodontal disease (vertical axis) and the periodontal pocket depth (Pd) (horizontal axis). Each diagram was described for individual 28 types of bacteria mounted on a DNA chip.
[Figure 1-3] Figure 1-3 is a scatter diagram of DNA chip measurement data (SN ratio) of subgingival plaque collected from 220 male and female subjects in their 20s to 70s before treatment of periodontal disease (vertical axis) and the periodontal pocket depth (Pd) (horizontal axis). Each diagram was described for individual 28 types of bacteria mounted on a DNA chip.
[Figure 1-4] Figure 1-4 is a scatter diagram of DNA chip measurement data (SN ratio) of subgingival plaque collected from 220 male and female subjects in their 20s to 70s before treatment of periodontal disease (vertical axis) and the periodontal pocket depth (Pd) (horizontal axis). Each diagram was described for individual 28 types of bacteria mounted on a DNA chip.
[Figure 1-5] Figure 1-5 is a scatter diagram of DNA chip measurement data (SN ratio) of subgingival plaque collected from 220 male and female subjects in their 20s to 70s before treatment of periodontal disease (vertical axis) and the periodontal pocket depth (Pd) (horizontal axis). Each diagram was described for individual 28 types of bacteria mounted on a DNA chip.
[Figure 1-6] Figure 1-6 is a scatter diagram of DNA chip measurement data (SN ratio) of subgingival plaque collected from 220 male and female subjects in their 20s to 70s before treatment of periodontal disease (vertical axis) and the periodontal pocket depth (Pd) (horizontal axis). Each diagram was described for individual 28 types of bacteria mounted on a DNA chip.
[Figure 1-7] Figure 1-7 is a scatter diagram of DNA chip measurement data (SN ratio) of subgingival plaque collected from 220 male and female subjects in their 20s to 70s before treatment of periodontal disease (vertical axis) and the periodontal pocket depth (Pd) (horizontal axis). Each diagram was described for individual 28 types of bacteria mounted on a DNA chip.
[Figure 2] Figure 2 is a scatter diagram of the bacterial group balance indexes (15 species of the "positively correlated bacteria" group and 13 species of the "negatively correlated bacteria" group) (vertical axis) and the periodontal pocket depth (Pd) (horizontal axis). The figure shows data for 220 subjects.
[Figure 3] Figure 3 is a histogram of the bacterial group balance indexes (LOG10 conversion) of 1-mm to 3-mm periodontal pocket (non-disease group and definition) and 5-mm or more periodontal pocket (disease group and definition) (vertical axis) and the frequency (horizontal axis).
[Figure 4] Figure 4 is a graph showing the results of ROC analysis of balance indexes.
[Figure 5] Figure 5 is a radar chart showing the SN ratio of each species in a sample with a periodontal pocket value of 4 mm and a balance index (LOG10) value of 1.516648. The axes from the center to the outside are the balance indexes.
[Figure 6] Figure 6 is a scatter diagram of the bacterial group balance indexes (2 species of "progression index bacteria" and 13 species of "negatively correlated bacteria") (vertical axis) and the periodontal pocket depth (Pd) (horizontal axis). The figure shows data for 220 subjects.
[Figure 7] Figure 7 is a histogram of the bacterial group balance indexes (LOG10 conversion) of 1-mm to 3-mm periodontal pocket (non-disease group and definition) and 5-mm or more periodontal pocket (disease group and definition) (vertical axis) and the frequency (horizontal axis).
[Figure 8] Figure 8 is a graph showing the results of ROC analysis of balance indexes.
[Figure 9] Figure 9 is a radar chart showing the SN ratio of each species in a sample with a periodontal pocket value of 4 mm and a balance index (LOG10) value of 0.883. The axes from the center to the outside are the balance indexes.
[Figure 10] Figure 10 is a chart showing the results of determining and subdividing the state of periodontal disease.
[Figure 11-1] Figure 11-1 is a radar chart showing the SN ratio of each bacterium in a sample in the subdivided state. The axes from the center to the outside are the balance indexes.
[Figure 11-2] Figure 11-2 is a radar chart showing the SN ratio of each bacterium in a sample in the subdivided state. The axes from the center to the outside are the balance indexes.
[Figure 11-3] Figure 11-3 is a radar chart showing the SN ratio of each bacterium in a sample in the subdivided state. The axes from the center to the outside are the balance indexes.
[Figure 12] Figure 12 is a graph showing the results of ROC analysis of balance indexes.
[Figure 13] Figure 13 is a graph showing the results of ROC analysis of balance indexes.
[Figure 14] Figure 14 is a scatter diagram of balance indexes before and after treatment, balance indexes, and periodontal pocket depth
[Figure 15] Figure 15 is a scatter diagram of balance indexes before and after treatment, balance indexes, and periodontal pocket depth (created from the copy number).
[Figure 16] Figure 16 is a chart showing the relative ratio of each bacterium from a sample obtained from the next-generation sequencer analysis results.
[Figure 17] Figure 17 is a scatter diagram of balance indexes and periodontal pocket depth.
[Figure 18] Figure 18 is a histogram of data on a periodontal pocket depth of 1 to 3 mm and a periodontal pocket depth of 5 mm or more among data shown in Figure 17.
[Figure 19] Figure 19 is a graph showing the results of ROC analysis.

### Description of Embodiments

Hereinafter, the present invention will be described in detail. The scope of the present invention is not limited to these descriptions, and other than the following examples, the scope of the present invention can be appropriately modified and implemented within a range not impairing the gist of the present invention. All publications cited in the present specification, for example, prior art literature, publications, patent publications, and other patent literature are incorporated herein by reference.

The present invention relates to an intraoral examination method for measuring a signal intensity of a nucleic acid from an oral bacterial group present in an oral sample, calculating an abundance of the bacterial group from a measured value of the signal intensity, and determining the state of periodontal disease using the obtained calculated value as an index. According to the present invention, the abundance of the bacterial group shows a correlation between the bacterial load of a bacterial species that increases as the periodontal pocket value increases and the bacterial load of a bacterial species that decreases as the periodontal pocket value increases.

A specific aspect of the intraoral examination method is not limited, but the method for measuring a bacterial count in an oral sample will be described herein focusing on a method using a DNA chip.

Bacteria in the oral sample may be detected, measured, and quantified by a method other than the method using a DNA chip, for example, the invader method, the real-time PCR method, the invader PCR method, the next-generation sequencing method, or the like.

### 1. Oligonucleotide probe for detecting oral bacteria

According to the method of the present invention, a DNA chip can be used when detecting oral bacteria in an oral sample collected from a subject. For example, the following probe (a) and at least one of the following probes (b) and (c) can be mounted on the DNA chip.
(a) A probe consisting of nucleic acids that specifically and separately hybridize with the genes of detection target bacteria (or amplification products from the genes)
(b) Total load index probe consisting of nucleic acids that hybridize with all bacterial genes (or amplification products from the genes)
(c) A probe consisting of nucleic acids that specifically and separately hybridize with one or more types of absolute load indexes

In addition, in general, a DNA chip is a general term for a substrate on which probes are arranged. Further, names such as DNA chip and DNA microarray used herein are not distinguished from each other and are synonymous.

### (1) Oral bacteria subjected to measurement

In the examination method of the present invention, oral bacteria subjected to measurement are not limited. However, bacteria belonging to the following can be detection target bacteria: the genera Porphyromonas, Tannerella, Treponema, Prevotella, Campylobacter, Fusobacterium, Streptococcus, Aggregatibacter, Capnocytophaga, Eikenella, Actinomyces, Veillonella, and Selenomonas, and further the genera Pseudomonas, Haemophilus, Klebsiella, Serratia, Moraxella, Eubacterium, Parvimonas, Filifactor, Alloprevotella, Solobacterium, Rothia, Peptostreptococcus, Gemella, Corynebacterium, Neisseria, Granulicatella, and Megasphaera; and the genera of the phylum SR1.

More specifically, for example, the detection target bacteria are preferably the following bacteria 1 species which are currently thought to be associated with periodontal disease and caries: Porphyromonas gingivalis, Tannerella forsythia, Treponema denticola, Campylobacter gracilis, Campylobacter rectus, Campylobacter showae, Fusobacterium nucleatum subsp. vincentii, Fusobacterium nucleatum subsp. polymorphum, Fusobacterium nucleatum subsp. animalis, Fusobacterium nucleatum subsp. nucleatum, Fusobacterium periodonticum, Prevotella intermedia, Prevotella nigrescens, Streptococcus constellatus, Aggregatibacter actinomycetemcomitans, Campylobacter concisus, Capnocytophaga gingivalis, Capnocytophaga ochracea, Capnocytophaga sputigena, Eikenella corrodens, Streptococcus gordonii, Streptococcus intermedius, Streptococcus mitis, Streptococcus mitis bv 2, Actinomyces odontolyticus, Veillonella parvula, Actinomyces naeslundiiII, and Selenomonas noxia; and further Streptococcus sanguis, Actinomyces viscosus, Streptococcus pyogenes, Streptococcus pneumoniae, Streptococcus mutans, Eubacterium nodatum, Parvimonas micra, Filifactor alocis, Streptococcus sobrinus, Porphyromonas pasteri, Veillonella atypica, Haemophilus parainfluenzae, Alloprevotella spp. (A. rava, OT 308), Streptococcus parasanguinis, Actinomyces israelii, Prevotella pallens, Prevotella loescheii, Prevotella histicola, Solobacterium moorei, Prevotella melaninogenica, Selenomonas sputigena, Rothia dentocariosa, Rothia mucilaginosa, Veillonella rogosae, Peptostreptococcus stomatis, Prevotella denticola, Porphyromonas endodontalis, Streptococcus salivarius, Actinomyces graevenitzii, Treponema medium, Treponema socranskii, Gemella sanguinis, Porphyromonas catoniae, Corynebacterium matruchotii, Eubacterium saphenum, Neisseria flavescens, Granulicatella adiacens, Eubacterium sulci, Megasphaera micronuciformis, Prevotella shahii, and SR1 sp. OT 345. More preferably, the bacteria are bacterial species with a clear increase/decrease in relation to pathological conditions.

For the purpose of detecting the condition in the oral cavity, for example, bacterial species which clearly increases and decreases together with the periodontal pocket value can be mentioned.

According to the present invention, the "periodontal pocket value" refers to the value of periodontal pocket depth (Pd). The term "periodontal pocket depth" (Pd) refers to the distance from the gingival margin to the tip of a periodontal probe when the probe is inserted into the pocket. Pd is digitized in units of 1 mm. The term "periodontal probe" as used herein means a pocket measuring instrument (perio probe).

The increase/decrease is not limited to an increase/decrease pattern, for example, a pattern of a bacterial group that increases as the periodontal pocket value increases or a pattern of a bacterial group that increases when the periodontal pocket value is small, and then increases, the bacterial load of which is maintained when the periodontal pocket value becomes large. More simple examples are a group of "bacterial species that increases as the periodontal pocket value increases" and a group of "bacterial species that decreases as the periodontal pocket value increases."

A group of bacterial species that increases as the periodontal pocket value increases and a group of bacterial species that decreases as the periodontal pocket value increases can be confirmed by a tool capable of measuring the bacterial load (or a measured amount that is proportional to the bacterial load such as the SN ratio). The tool is not particularly limited, and for example, a DNA chip can be used.

When a DNA chip is used for confirmation, an oral sample is measured with the DNA chip, and then, a correlation coefficient between the periodontal pocket value and the bacterial load of each bacterium or the measured amount such as the SN ratio is calculated. Thus, the bacteria can be classified and identified as a bacterial group having a positive correlation coefficient and a bacterial group having a negative correlation coefficient. The absolute value of the correlation coefficient for these bacteria is preferably 0.02 or more, more preferably 0.1 or more, still more preferably 0.2 or more, particularly preferably 0.4 or more, and most preferably 0.6 or more when the number of measurements is 40 or more.

When using the experimental error-corrected data to determine the state of periodontal disease, the experimental error-corrected data are used for classification of bacterial groups as well.

Bacterial species that increase as the periodontal pocket value increases (hereinafter sometimes referred to as "positively correlated bacteria") are bacteria that increase with the deterioration of periodontal disease. Known examples of such species are Porphyromonas gingivalis, Tannerella forsythia, and Treponema denticola, which are used in existing periodontal disease bacterial tests.

The bacterial species that increases as the periodontal pocket value increases is at least one selected from the group consisting of Porphyromonas gingivalis, Tannerella forsythia, Treponema denticola, Campylobacter gracilis, Campylobacter rectus, Campylobacter showae, Fusobacterium nucleatum subsp. vincentii, Fusobacterium nucleatum subsp. polymorphum, Fusobacterium nucleatum subsp. animalis, Fusobacterium nucleatum subsp. nucleatum, Fusobacterium periodonticum, Prevotella intermedia, Streptococcus constellatus, Aggregatibacter actinomycetemcomitans, Eikenella corrodens, Filifactor alocis, Porphyromonas endodontalis, Eubacterium nodatum , Eubacterium saphenum, Treponema medium, and Selenomonas sputigena.

Of these, bacteria that increase when the periodontal pocket value is small, and then increases, the bacterial load of which is maintained when the periodontal pocket value becomes large are sometimes referred to as "progression index bacteria." "Progression index bacteria" are thought to play a role in connecting the "bad bacteria" and "good bacteria" described below and serve as a pre-stage index of periodontal disease deterioration. Specific examples of "progression index bacteria" include Fusobacterium nucleatum species.

The Fusobacterium nucleatum species is at least one selected from the group consisting of Fusobacterium nucleatum subsp. vincentii, Fusobacterium nucleatum subsp. polymorphum, Fusobacterium nucleatum subsp. animalis, and Fusobacterium nucleatum subsp. nucleatum.

Meanwhile, a bacterial group that increases as the periodontal pocket value increases may be hereinafter referred to as "bad bacteria." Specific examples of "bad bacteria" include bacterial species other than the Fusobacterium nucleatum species among bacterial species that increases as the periodontal pocket value increases. For example, at least one selected from the group consisting of Porphyromonas gingivalis, Tannerella forsythia, Treponema denticola, Campylobacter gracilis, Campylobacter rectus, Campylobacter showae, Fusobacterium periodonticum, Prevotella intermedia, Streptococcus constellatus, Aggregatibacter actinomycetemcomitans, Eikenella corrodens, Filifactor alocis, Porphyromonas endodontalis, Eubacterium nodatum, Eubacterium saphenum, Treponema medium, and Selenomonas sputigena can be mentioned.

Examples of bacterial species that decreases as the periodontal pocket value increases (hereinafter sometimes referred to as "negatively correlated bacteria") include some of bacterial species belonging to the genera Streptococcus, Actinomyces, Veillonella, and the like.

These include: (i) bacterial species that decreases as the periodontal pocket value increases (i.e., deterioration of periodontal disease); (ii) bacterial species that increases as the periodontal pocket value decreases (improvement of periodontal disease); or both (i) and (ii) above. Bacterial species that decreases as the periodontal pocket value increases may be hereinafter sometimes referred to as "good bacteria."

Examples of bacterial species that decreases as the periodontal pocket value increases and/or bacterial species that increases as the periodontal pocket value decreases include at least one selected from the group consisting of Prevotella nigrescens, Campylobacter concisus, Capnocytophaga gingivalis, Capnocytophaga ochracea, Capnocytophaga sputigena, Streptococcus gordonii, Streptococcus intermedius, Streptococcus mitis, Streptococcus mitis bv 2, Actinomyces odontolyticus, Veillonella parvula, Actinomyces naeslundii II, Selenomonas noxia, Prevotella denticola, Prevotella melaninogenica, Gemella sanguinis, Eubacterium sulci, Corynebacterium matruchotii, Rothia mucilaginosa, Porphyromonas catoniae, Solobacterium moorei, Neisseria flavescens, Prevotella loescheii, Megasphaera micronuciformis, Actinomyces graevenitzii, Veillonella atypica, Prevotella pallens, Prevotella shahii, Porphyromonas pasteri, Veillonella rogosae, Alloprevotella spp. (A. rava, OT 308), Rothia dentocariosa, Granulicatella adiacens, Streptococcus salivarius, Haemophilus parainfluenzae, and Streptococcus parasanguinis.

### (2) Probe (a)

In the present invention, oligo DNA that can be used as a probe (a) can be hybridized with a base sequence of a bacterial-specific region (a region having a base sequence that changes depending on the bacterial type) of the base sequence of a nucleic acid from an oral bacterium. Here, the nucleic acid may be any of DNA and RNA including chromosomal DNA and plasmid DNA, and is not limited, but chromosomal DNA is preferable. Specifically, an oligonucleotide used as a probe in the present invention is capable of hybridizing with the base sequence of the 16S rRNA gene in the oral bacterial chromosomal DNA.

It is preferable that probes that can be used in the present invention are designed by selecting a region having a base sequence specific to a different type of oral bacterium to be detected and designing a base sequence of the region. In general, in designing a probe, in addition to selecting a specific region, it is necessary that the melting temperature (Tm) is uniform and that a secondary structure is difficult to form.

The specific base sequence corresponding to each oral bacterial species can be found by means of, for example, performing multiple alignment and designing probes in different regions between species. The algorithm for alignment is not particularly limited, but as a more specific analysis program, for example, a program such as ClustalX1.8 can be used. The parameters used for the alignment may be executed in the default state of each program, but can be adjusted as appropriate according to the type of program.

Meanwhile, the probe specificity probe may be a specificity that collectively detects bacteria of the same genus based on the genus-level specificity or may be a specificity that can be detected at the individual species level. Probes can be appropriately determined according to the bacterial detection purpose. Depending on the level of specificity of detection, the bacterial species that can be detected may be limited to one specific species or may be taken as the sum (total) at the genus level.

### (3) Probe (b)

A total load index probe is a probe for capturing all bacteria in a specimen that can be amplified with a specific primer pair. In detecting bacteria, it is especially important to detect the total bacterial load from the viewpoints of the proportion of target bacteria with respect to the entire bacteria including non-detection target bacteria and the overall abundance of bacteria present in a specimen. The non-detection target bacteria can be understood as the sum (total) of bacteria of known types which are known to be present but may not be detected, and bacteria of unknown types which are unknown to be present.

In order to detect the total bacterial load, for example, it is possible to measure the total bacterial load independently of a DNA chip. Meanwhile, the simplicity of the operation is improved by mounting a probe, which is an index of the total bacterial load, in the DNA chip. Regarding probes, a base sequence common to many types of bacterial species may be used from the base sequences amplified by the primer pair. When such a sequence cannot be found, a plurality of relatively common sequences may be designed and comprehensively determined to be used as the total load index probe. The total load index probe is preferably a probe that hybridizes with a nucleic acid from a bacterium contained in a specimen, specifically, a probe that hybridizes with a base sequence common in a plurality of types of bacteria to be detected from the base sequence amplified by the specific primer pair.

The total load index usually increases because it represents the total amount of amplification products specific to individual species. Therefore, the signal intensity of interest may exceed the range of detectable signal intensities. In order to prevent such a situation, it is desirable to limit the amount of a specimen used for hybridization. Alternatively, when designing a probe, for example, the Tm value of the probe is lowered. Specifically, it is conceivable to reduce the GC content or shorten the probe sequence length itself.

Further, at the time of hybridization, it is possible to reduce the signal intensity by adding a nucleic acid that competitively acts on the hybridization between the amplified nucleic acid and the total load index probe. Examples of such a nucleic acid include a nucleic acid having a sequence which is wholly or partially the same as that of the total load index probe, or a nucleic acid which wholly or partially has a complementary sequence of the total load index probe.

### (4) Probe (c)

An absolute load index probe is a probe that hybridizes only with an nucleic acid corresponding to an absolute load index. In the present specification, the absolute load index is an index indicating the amount of a nucleic acid that is added to a specimen in a fixed amount before an amplification reaction or a hybridization reaction. The absolute load index refers to a nucleic acid that can be surely amplified by a normal amplification reaction, and serves as a so-called positive control. Therefore, when a probe specific to the absolute load index is mounted on a DNA chip, it can be confirmed from the detection results whether the amplification reaction, hybridization, or the like has been appropriately performed.

In a case in which the absolute load index is added before an amplification reaction, it is necessary to add a specific primer pair for the absolute load index to a reaction solution. In some cases, it is also possible to commonly amplify the probe with a primer pair for bacteria. Further, in order to achieve detection independently of other detection targets by hybridization, it is necessary to select a base sequence having low similarity to both the detection target bacteria and non-detection target bacteria.

When one type of absolute load index is set, if the amplification efficiency or hybridization efficiency is slightly increased or decreased, the correction coefficient can be calculated by comparing the signal intensities of the absolute load index. When comparing data of a plurality of DNA chips, the signal intensities after correction with the correction coefficient may be compared.

Here, specific examples of the probes (a), (b), and (c) can be exemplified in Table 1.

Table 1 shows examples of probes specific to individual bacteria (SEQ ID NOS: 1 to 33).

An example of the total load index probe is set forth in SEQ ID NO: 34.

An example of the absolute load index probe is set forth in SEQ ID NO: 35.

An example of the absolute load index is set forth in SEQ ID NO: 36.

**[Table 1]**

| SEQ ID NO. | | |
|---|---|---|
| 1 | Porphyromonas gingivalis probe | TTCAATGCAATACTCGTATC |
| 2 | Tannerella forsythia probe | CACGTATCTCATTTTATTCC |
| 3 | Treponema denticola probe | CCTCTTCTTCTTATTCTTCAT |
| 4 | Treponema denticola probe1 | CTCTTCTTCTTATTCTTCAT |
| 5 | Campylobacter gracilis probe1 | GCCTTCGCAATAGGTATT |
| 6 | Campylobacter rectus probe | ATTCTTTCCCAAGAAAAGGA |
| 7 | Campylobacter rectus probe2 | GTCATAATTCTTTCCCAAGA |
| 8 | Campylobacter showae probe | CAATGGGTATTCTTCTTGAT |
| 9 | Fusobacterium nucleatum subsp. vincentii probe | TAGTTATACAGTTTCCAACG |
| 10 | Fusobacterium nucleatum subsp. polymorphum probe | CCAGTACTCTAGTTACACA |
| 11 | Fusobacterium nucleatum subsp. animalis probe5 | TTTCTTTCTTCCCAACTGAA |
| 12 | Fusobacterium nucleatum subsp. nucleatum probe7 | TACATTCCGAAAAACGTCAT |
| 13 | Fusobacterium periodonticum probe | TATGCAGTTTCCAACGCAA |
| 14 | Prevotella intermedia probe | GGGTAAATGCAAAAAGGCA |
| 15 | Prevotella nigrescens probe | CTTTATTCCCACATAAAAGC |
| 16 | Streptococcus constellatus probe | AAGTACCGTCACTGTGTG |
| 17 | Aggregatibacter actinomycetemcomitans probe1 | GTCAATTTGGCATGCTATTA |
| 18 | Campylobacter concisus probe | CCCAAGCAGTTCTATGGT |
| 19 | Capnocytophaga gingivalis probe | TACACGTACACCTTATTCTT |
| 20 | Capnocytophaga ochracea probe | CAACCATTCAAGACCAACA |
| 21 | Capnocytophaga sputigena probe1 | TACACGTACACCTTATTCTT |
| 22 | Eikenella corrodens probe2 | CTCTAGCTATCCAGTTCAG |
| 23 | Streptococcus gordonii probe | CACCCGTTCTTCTCTTACA |
| 24 | Streptococcus gordonii probe1 | CACCCGTTCTTCTCTTAC |
| 25 | Streptococcus intermedius probe | ACAGTATGAACTTTCCATTCT |
| 26 | Streptococcus intermedius probe1 | CAGTATGAACTTTCCATTCT |
| 27 | Streotococcus mitis probe6 | TCTCCCCTCTTGCACTCA |
| 28 | Streotococcus mitis by 2 probe | TCCCCTCTTGCACTCAAGT |
| 29 | Actinomyces odontolyticus probe | AAGTCAGCCCGTACCCA |
| 30 | Veillonella parvula probe | TATTCGCAAGAAGGCCTT |
| 31 | Actinomyces naeslundii II probe | CCACCCACAAGGAGCAG |
| 32 | Selenomonas noxia probe1 | TTCGCATTAGGCACGTTC |
| 33 | Streptococcus mutans probe | CACACGTTCTTGACTTAC |
| 34 | Total load index probe | CGTATTACCGCGGCTGCTGGCAC |
| 35 | Absolute load index probe | CTATTCGACCAGCGATATCACTACGTAGGC |
| 36 | Absolute load index | GTGAGAAGCCTACACAAACGTAACGTCAGGGCTAAGACAAACGCTAAC GGTACACCCTAGATGGGAGCTTGTAGCTAGATCGCTAAGTCCTACCGA CATGTAGGCATACTCACGAAGGCAATTCCCTGAAAGCCTCGTCTTATC CCGAACTTGGCATCTGCTGATACGTCAGGTTGAACGCGTACATTTACC TGTCATGCGTGGGCCTTCTCCGAATAGCCTACGTAGTGATATCGCTGG TCGAATAGGCGGATTGCTCATAAATGCACATTGGCTAAGGCCCACGGA ACACGAATCACGTGAGATCACTTACTATTCGACGGAACTACTATACGCA CCGGGACATGCAAGTAGCGTCCCACAAGCATAAGGAACTCTATACTCG CCATCTACGCAGCTACAGGGGATACACGTATGAGCGGTTACGAAGTAA AGCCGAGATAGAGCGGTCTTTAGAGAAAAAACAGGATTAGATACCCTGG TAGTCC |

### <Primer Designing>

In the primer designing method according to the present invention, first, at least one variable region showing the diversity of bacteria to be analyzed is selected, and a highly conservative universal primer designing region is selected before and after the selected variable region to design a primer sequence. The variable region of interest includes, but is not limited to, the 16S rRNAgene present in all bacteria in the genomic sequence. Of the 16S rRNA gene, it is desirable to target the full length or one or more regions of variable regions V1 to V9. More preferably, it is desirable to target the variable regions V1 to V6. Even more preferably, it is desirable to target the variable regions V3 to V6. It is known that the variable region of the 16S rRNAgene consists of the V1 to V9 regions, which have also been specified.

To evaluate the completeness of the primers, a database that has acquired a wide range of bacterial genomic sequences is utilized. Specific examples of such a database include RDP, NCBI, KEGG, and MGDB. As an example, the designed universal primer sequence is input to "Probe Match" of the RDP database. In the list of results, the number of exact matches in "Total Search" is obtained. The closer the perfect match number is to "Total Search," the higher the coverage is. At this time, as a condition, "Type" may be selected for "Strain." In addition, "Isolates" may be selected for "Source."

When designing the absolute load index sequence and a primer sequence for amplifying the sequence, for example, it is possible to use the RNDB19ETWEEN function of software "EXCEL" (manufactured by MICROSOFT), randomly generate X integers from 1 to 4 (X is an arbitrary number), connect them to create a numerical value of X digits consisting only of numerical values 1 to 4, and replace numerical values 1 to 4 with any of A, G, C and T, thereby obtaining random sequences. For example, by replacing 1 with A, 2 with T, 3 with C, and 4 with G, a large number of random sequences based on the X bases of ATGC can be obtained.

Of these sequences, only the sequences in which the sum of G and T is the same as the sum of A and T are extracted, and the extracted sequences are searched by BLAST against a database such as NCBI's GenBank to select a sequence including few similar sequences to a biologically-derived nucleic acid.

In order to make the reaction efficiency during the amplification reaction as constant as possible, it is desirable that the base length amplified in a detection target bacterium and the amplified base length of the absolute load index do not have a large difference. For example, if the amplification product of the detection target bacterium is about 500 bp, the amplification product of the absolute load index is preferably about 300 bp to 1000 bp.

Meanwhile, in a case in which the amplified chain length is confirmed by electrophoresis after amplification, it is also possible to design an amplification product with a length different from that of the detection target bacterium and detect the amplification product from the absolute load index at a position different from the band of the detection target bacterium, thereby confirming the success or failure of the amplification reaction before hybridization.

Lastly, if the absolute load index in the specimen is excessively high in terms of concentration, competition with detection target bacteria in an amplification reaction may become intense, and there is a possibility that detection target bacteria, which should be detected, may not be detected. Therefore, it is necessary to properly adjust the concentration according to the application.

In a case in which a bacterial-derived nucleic acid and the absolute load index are amplified separately, a multiplex method using two or more pairs of primers can be applied as necessary. Conversely, if necessary, a method for allowing a common pair to compete with a primer pair can also be applied.

Examples of primer sequences are shown in Table 2. A pair of primers for bacterial amplification (SEQ ID NOS: 37 and 38) and a pair of absolute load index primers (SEQ ID NOS: 39 and 40) can be used. In addition to the above, the primers shown in Table 3 can also be used.

**[Table 2]**

| SEQ ID NO | | Remarks | |
|---|---|---|---|
| 37 | Forward primer (for bacterial amplification) | Fluorescent label at 5' | TCCTACGGGAGGCAGCAGT |
| 38 | Reverse primer (for bacterial amplification) | | CAGGGTATCTAATCCTGTTTGCTACC |
| 39 | Forward primer (for absolute load index amplification) | Fluorescent label at 5' | GAGAAGCCTACACAAACGTAACGTC |
| 40 | Reverse primer (for absolute load index amplification) | | CTCTAAAGACCGCTCTATCTCGG |

**[Table 3]**

| SEQ ID NO | Primer name | Remarks | Sequence |
|---|---|---|---|
| 41 | Cy5-Universal16S-FWD | Fluorescent label at 5' | TCCTACGGGAGGCAGCAGT |
| 42 | Cy5-Universal16S-FWD1 | Fluorescent label at 5' | TCCTACGGGAGGCAGCAG |
| 43 | Cy5-Universal16S-FWD2 | Fluorescent label at 5' | TCCTACGGGAGGCAGCA |
| 44 | Cy5-Universal16S-FWD3 | Fluorescent label at 5' | TCCTACGGGAGGCAGC |
| 45 | Cy5-Universal16S-FWD4 | Fluorescent label at 5' | CCTACGGGAGGCAGC |
| 46 | Cy5-Universal16S-FWD5 | Fluorescent label at 5' | CTACGGGAGGCAGCAG |
| 47 | Cy5-Universal16S-FWD6 | Fluorescent label at 5' | TACGGGAGGCAGCAG |
| 48 | SidneyU RVS | | AACAGGATTAGATACCCTGGTAGTCC |
| 49 | Universal RVS2 2014 | | GGTAGCAAACAGGATTAGATACCCTG |
| 50 | Universal RVS1 2016 | | CRAACAGGATTAGATACCCTG |
| 51 | Universal RVS2 2016 | | AACAGGATTAGATACCCTG |
| 52 | Universal RVS3 2016 | | AACRGGATTAGATACCC |
| 53 | Universal RVS4 2016 | | AACRGGATTAGATACCCYG |

### <Probe Designing>

When designing a probe used in the present invention, the length of the probe is not limited, but is preferably 10 bases or more, more preferably 16 to 50 bases, and still more preferably 18 to 35 bases. As long as the length of a probe is appropriate (within the above range), nonspecific hybridization (mismatch) can be suppressed and such a probe can be used for specific detection. In addition, it is preferable to also confirm Tm when designing a probe used in the present invention. Tm means the temperature at which 50% of any nucleic acid strand hybridizes with its complementary strand. In order for the template DNA or RNA and the probe to form a double strand and hybridize with each other, the temperature of hybridization needs to be optimized. Meanwhile, if the temperature is excessively lowered, a nonspecific reaction is likely to occur, and therefore, the temperature is preferably as high as possible.

Accordingly, the Tm of a nucleic acid fragment to be designed is an important factor for hybridization. Known probe design software can be used for confirmation of Tm, and examples of software usable in the present invention include Probe Quest (registered trademark; DYNACOM Co., Ltd.). The confirmation of Tm can also be performed by manually calculating without using software. In that case, a calculation formula based on the nearest neighbor method, the Wallance method, the GC% method, or the like can be used. In the probe of the present invention, the average Tm is preferably, but not limited to, about 35°C to 70°C or 45°C to 60°C. Note that other conditions that allow the probe to achieve specific hybridization include the GC content and the like, and the conditions are well known to those skilled in the art.

Further, the probe of the present invention may include an additional sequence such as a tag sequence. An example of the tag sequence is a spacer sequence such as "AAAAAAA." According to the method of the present invention, the base sequence of the nucleic acid possessed by the oral bacterium to be detected does not need to be the base sequence itself in every case, and a part of the base sequence may be mutated by deletion, substitution, insertion, or the like. Therefore, a mutated gene that hybridizes with a sequence complementary to the base sequence under stringent conditions and has a function or activity derived from each base sequence may also have the base sequence of the nucleic acid to be detected. The probe can also be designed based on the base sequence of such mutated gene.

Specifically, a probe to be designed includes the sequence of the above-described probe (a). In addition, preferable examples thereof include those including DNA capable of hybridizing with a DNA having a base sequence complementary to the above DNA under stringent conditions and detecting at least a part of the base sequence of a nucleic acid from an oral bacterium. The base sequence of such a DNA is a base sequence that is preferably at least 60%, more preferably 80% or more, still more preferably 90% or more, even more preferably 95% or more, and particularly preferably 97% or more homologous to the base sequence of the probe (a).

When the probe is actually used for detection, it is necessary to consider the stringency in hybridization. By setting the stringency to a dense degree to a certain extent, even if there are similar nucleotide sequence regions between specific regions in each nucleic acid in various oral bacteria, other different regions can be distinguished and hybridized. When the base sequences between the specific regions are almost different, the stringency can be set to a mild level.

Such stringency conditions include, for example, hybridization at 50°C to 60°C under the dense conditions and hybridization at 30°C to 40°C under the mild conditions. For hybridization conditions, examples of stringent conditions include, for example, "0.24 M Tris•HCl/0.24 M NaCl/0.05% Tween-20, 40°C," "0.24 M Tris-HCl/0.24 M NaC/0.05% Tween-20, 37°C," and "0.24 M Tris-HCl/0.24 M NaCl/0.05% Tween-20, 30°C," and examples of more stringent conditions include, for example, "0.24 M Tris•HCl/0.24 M NaCl/0.05% Tween-20, 5°C," "0.24 M Tris• HCl/0.24M NaCl/0.05% Tween-20, 55°C," and "0.06 M Tris • HCl/0.06 M NaCl/0.05% Tween-20, 60°C."

More specifically, there is also a method in which hybridization is performed by adding a probe and keeping it at 50°C for 1 hour or more, and then washing it in 0.24 M Tris·HCl/0.24 M NaCl/0.05% Tween-20 four times for 20 minutes at 50°C, and washing it once with 0.24 M Tris·HCl/0.24 M NaCl at 50°C for 10 minutes at the end. By increasing the temperature during hybridization or washing, more stringent conditions can be set. A person skilled in the art can set the conditions by considering various conditions such as the probe concentration, the probe length, and the reaction time, in addition to the conditions such as the salt concentration of buffer and the temperature. For the detailed procedure of the hybridization method, "Molecular Cloning, A Laboratory Manual 4th ed." (Cold Spring Harbor Press (2012), "Current Protocols in Molecular Biology" (John Wiley & Sons (1987-1997)), and the like can be referred to.

In addition, the nucleotide constituting the probe used in the present invention may be any of DNA, RNA, or PNA, and may be a hybrid of two or more types of DNA, RNA and PNA. For example, the probe can be prepared by, for example, chemical synthesis based on a usual oligonucleotide synthesis method (purification is carried out by HPLC or the like). It is also possible to use a chemically modified terminal or intermediate of the above nucleotide.

### 2. DNA Chip for Detecting Oral Bacterial Gene Used for Measuring Oral Bacterial Load

As described above, a DNA chip can be used in the method of the present invention, and the DNA chip has a plurality of the various oligonucleotide probes described in the above section 1 which are arranged on a substrate serving as a support. As the form of the substrate serving as a support, any form such as a flat plate (e.g., a glass plate, resin plate, or silicon plate), a rod shape, beads, or the like can be used. When a flat plate is used as the support, predetermined probes can be fixed on the flat plate at predetermined intervals by type (e.g., the spotting method; see Science 270, 467-470 (1995) or the like). It is also possible to successively synthesize predetermined probes by type at specific positions on a flat plate (e.g., the photolithography method; see Science 251, 767-773 (1991) or the like).

Other preferable support forms include those using hollow fibers. When using hollow fibers as the support, a DNA chip obtained by fixing a predetermined probe to each hollow fiber by type, bundling and fixing all the hollow fibers, and then repeating cutting in the longitudinal direction of the fibers (hereinafter, referred to as "fiber type DNA chip") can be preferably exemplified. This microarray can be described as a type of microarray prepared by immobilizing nucleic acids on a through-hole substrate, and is also called a so-called "through-hole type DNA chip" (see JP Patent No. 3510882).

The method of fixing the probes to the support is not limited, and any binding mode may be used. Further, fixation of the probes is not limited to direct fixation to the support. For example, the support may be coated in advance with a polymer such as polylysine and the probes may be fixed to the treated support. Furthermore, when a tubular body such as a hollow fiber is used as the support, the tubular body can be configured to hold a gel-like material and a probe can be fixed to the gel-like material. Hereinafter, a fiber type DNA chip, which is one aspect of the DNA chip, will be described in detail. This DNA chip can be produced through, for example, the following steps (i) to (iv).
(i) A step of producing an array by three-dimensionally arranging a plurality of hollow fibers such that the longitudinal directions of the hollow fibers are the same direction
(ii) A step of producing a block body by embedding the array
(iii) A step of introducing a gel precursor polymerizable solution containing an oligonucleotide probe into the hollow portion of each hollow fiber of the block body to carry out a polymerization reaction and holding the gel-like material containing the probe in the hollow portion
(iv) A step of thinning the block body by cutting it in the direction intersecting the longitudinal direction of the hollow fiber

The material used for the hollow fiber is not limited, but for example, materials described in JP Patent Publication (Kokai) No. 2004-163211 A and the like are preferable.

The hollow fibers are three-dimensionally arranged such that their lengths in the longitudinal direction are the same (step (i)).
Examples of the arrangement method include a method for arranging a plurality of hollow fibers in parallel on a sheet-like material such as an adhesive sheet at predetermined intervals to form a sheet and winding the sheet in a spiral shape (see JP Patent Publication (Kokai) No. 11-108928 A (1999)) and a method in which two perforated plates provided with a plurality of holes at predetermined intervals are overlapped such that the holes match, hollow fibers are allowed to pass through those holes, and the two perforated plates are opened with an interval and temporarily fixed, and then, a curable resin material is filled around each hollow fiber between the two porous plates for curing (see JP Patent Publication (Kokai) No. 2001-133453 A). The produced array is embedded such that the arrangement is not disturbed (step (ii)).

Preferable examples of the embedding method include a method in which a polyurethane resin, an epoxy resin, or the like is poured into a gap between fibers and a method in which fibers are bonded to each other by heat fusion.

In the embedded array, a gel precursor polymerizable solution (gel forming solution) containing an oligonucleotide probe is filled in the hollow part of each hollow fiber, and a polymerization reaction is carried out in the hollow part (step (iii)). As a result, the gel-like material to which the probe is fixed can be held in the hollow portion of each hollow fiber. The gel precursor polymerizable solution is a solution containing a reactive substance such as a gel-forming polymerizable monomer, and the solution can be a gel-like material by polymerizing and crosslinking the monomer or the like. Examples of such a monomer include acrylamide, dimethylacrylamide, vinylpyrrolidone, and methylenebisacrylamide. In this case, the solution may contain a polymerization initiator or the like. After fixing the probe in the hollow fiber, the block body is cut into thin sections in a direction intersecting the longitudinal direction of the hollow fiber (preferably in a direction orthogonal thereto) (step (iv)). The thin sections thus obtained can be used as a DNA chip. The thickness of the DNA chip is preferably about 0.01 mm to 1 mm. The block body can be cut with, for example, a microtome, a laser, or the like. Preferable examples of the fiber type DNA chip described above include a DNA chip (Genopal™) manufactured by Mitsubishi Chemical Corporation.

In the fiber type DNA chip, the probes can be arranged three-dimensionally in the gel as described above such that the three-dimensional structure can be maintained. Therefore, as compared with a flat DNA chip in which a probe is bound to a surface-coated slide glass, the detection efficiency is increased, and an extremely sensitive and reproducible test can be performed. Further, the number of types of probes arranged on a DNA chip is preferably 500 types or less, preferably 250 types or less, and more preferably 100 types or less on a single DNA chip. By limiting the number (type) of probes arranged in this way to some extent, it becomes possible to detect oral bacteria of interest with higher sensitivity. The type of probe is distinguished by the base sequence. Therefore, even if probes originate from the same gene, they are specified as different types unless there is no difference between their base sequences.

### 3. Detection of Oral Bacterial Gene

According to the method of the present invention, the method for detecting the gene of an oral bacterium to measure the bacterial load thereof is, for example, a method including the following steps.
(I) A step of using, as a specimen, an oral sample collected from a subject and extracting nucleic acids in the specimen
(ii) A step of bringing the extracted nucleic acids into contact with the aforementioned oligonucleotide probe of the present invention or the DNA chip of the present invention
(Iii) A step of calculating the SN ratio or the bacterial load from the signal intensity obtained from the DNA chip

Hereinafter, the details of the detection method will be described step by step. (1) Step (i)

In this step, an oral sample collected from a subject is used as a specimen, and nucleic acids of bacteria contained in the specimen are extracted. Type of the oral sample to be collected is not particularly limited. For example, saliva, plaque (subgingival plaque and supragingival plaque), tongue coating, mouthwash, and the like can be used. Of these, plaque is preferable. In particular, subgingival plaque collected from a place where periodontal bacteria are most inhabited is more preferable.

The method for collecting an intraoral sample is not particularly limited, and it can be appropriately selected depending on the type of sample. For example, in a case in which saliva is used as an oral sample, examples the method include a method using a commercially available saliva collecting kit, a method for collecting saliva with a swab in the mouth, and a method for collecting saliva directly into a container.

In a case in which plaque is used as an oral sample, examples of the method include brushing of tooth surface and tooth with a toothbrush, tooth surface abrasion with a cotton swab, interdental brushing with an interdental brush, and the paper point method. Plaque is dissolved or suspended by soaking a toothbrush, swab, interdental brush, or paper point used for collecting plaque in sterile water, followed by, for example, stirring if necessary. The thus obtained solution or suspension may be used as a specimen. The amount of plaque to be collected is not particularly limited, and for example, the amount corresponding to one paper point is sufficient. When tongue coating is used as an oral sample, a method for rubbing the tongue with a swab can be used. Plaque is dissolved or suspended using a swab used for collecting plaque in sterile water. The thus obtained solution or suspension may be used as a specimen. The amount of tongue coating collected is not particularly limited, and for example, the amount corresponding to one swab is sufficient.

In a case in which mouthwash is used as an oral sample, a method in which a solution obtained by containing mouthwash or water in the mouth and collecting saliva together with the mouthwash or water in a container is used as a sample can be exemplified. Examples of the mouthwash include sterilized physiological saline. Next, extraction of nucleic acids from the bacteria present in the obtained oral sample is performed. The extraction method is not limited, and a known method can be used. For example, an automatic extraction method using a device, a method using a commercially available nucleic acid extraction kit, a bead disruption method, a method for extraction with phenol after proteinase K treatment, a method using chloroform, or a simple extraction method including a method for heating and dissolving a sample can be exemplified. These can be combined for treatment. In addition, it is not particularly necessary to extract nucleic acids from the specimen, and the process may proceed to the next step.

The nucleic acids obtained from the specimen may be directly brought into contact with a DNA chip or the like, or a desired base sequence region may be amplified by PCR or the like, and the amplified fragment may be brought into contact with the DNA chip or the like, without any limitation. The region to be amplified using the obtained nucleic acid as a template is a region encoding the nucleic acid region including the base sequence of the probe used in the present invention or the oligonucleotide arranged on the DNA chip. The desired region to be amplified is not limited and can be obtained by using the base sequence of a highly conserved region regardless of species of oral bacteria and amplifying a mixture of many types at once. The sequence for such amplification may be experimentally isolated and purified, and the base sequence of the isolated polynucleotide may be analyzed and determined based on the sequence. Alternatively, the sequence may be determined by *in silico* by searching a known base sequence in various databases and obtaining an alignment. The database of nucleic acids or amino acids is not particularly limited, but, for example, a Taxonomy database or the like is available at DDBJ (DNA Data Bank of Japan), EMBL (European Molecular Biology Laboratory, EMBL nucleic acid sequence data library), GenBank (Genetic sequence data bank), and NCBI (National Center for Biotechnology Information).

Specifically, the desired site to be amplified is preferably the ribosomal RNA (16S rRNA) gene in chromosomal DNA of an oral bacterium. Preferable examples of PCR primers that can be used for amplification of the region include those in Tables 2 (SEQ ID NOS: 37 and 38) and 3 (SEQ ID NOS: 41 to 53). Amplification of nucleic acids by the PCR method can be performed according to a standard method.

The nucleic acid extracted in this step and an amplified fragment thereof can be labeled appropriately and used in the detection process after hybridization. Specifically, a method for labeling an end of a PCR primer with various reporter dyes, a method for incorporating a reactive nucleotide analog in a reverse transcription reaction, a method for incorporating a biotin-labeled nucleotide, and the like can be considered. Furthermore, it is also possible to label the nucleic acid or a fragment thereof by reacting it with a fluorescent labeling reagent after preparation. As the fluorescent reagent, for example, various reporter dyes (e.g., Cy5, Cy3, VIC, FAM, HEX, TET, fluorescein, FITC, TAMRA, Texas red, and Yakima Yellow) can be used.

### (2) Step (ii)

In this step, the nucleic acid or an amplified fragment thereof obtained in step (i) is brought into contact with the probe or DNA chip used in the present invention. Specifically, a hybridization solution containing the nucleic acid or the like is prepared, and the nucleic acid or the like therein is bound (hybridized) to an oligonucleotide probe mounted on the DNA chip. The hybridization solution can be appropriately prepared by using a buffer solution such as SDS or SSC according to a standard method. The hybridization reaction can be performed by appropriately setting the reaction conditions (e.g., type of buffer solution, pH, and temperature) such that the nucleic acid or the like in the hybridization solution can hybridize with the oligonucleotide probe mounted on the DNA chip under stringent conditions. The term "stringent conditions" as used herein refers to conditions in which cross-hybridization due to similar sequences is unlikely to occur or nucleic acids cross-hybridized by similar sequences are dissociated. Specifically, it means the conditions of washing the DNA chip during the hybridization reaction or after hybridization.

For example, as for the conditions during the hybridization reaction, the reaction temperature is preferably 35°C to 70°C, more preferably 40°C to 65°C, and the hybridization time is preferably about 1 minute to 16 hours. As for the conditions of washing the DNA chip after hybridization, the washing solution composition comprises preferably 0.24 M Tris ·HCl/0.24 M NaCl/0.05% Tween-20, and the temperature during washing is preferably 35°C to 80°C or 40°C to 65°C, more preferably 45°C to 60°C. More specifically, the conditions in which the salt (sodium) concentration is 48 to 780 mM and the temperature is 37°C to 80°C are preferable, and the conditions in which the salt concentration is 97.5 to 390 mM and the temperature is 45°C to 60°C are more preferable.

After washing, the detection intensity is measured for each spot with an apparatus capable of detecting a label such as a nucleic acid bound to a probe. For example, in a case in which the nucleic acid or the like is fluorescently labeled, the fluorescence intensity can be measured by using various fluorescence detection devices such as CRBIO (manufactured by Hitachi Software Engineering Co., Ltd.), arrayWoRx (manufactured by GE Healthcare), Affymetrix 428 Array Scanner (manufactured by Affymetrix, Inc.), GenePix, (Axon Instruments), ScanArray (PerkinElmer), and Genopal Reader (Mitsubishi Chemical Corporation). With respect to these devices, in the case of a fluorescence scanner, scanning can be performed by, for example, appropriately adjusting the laser output and the sensitivity of the detection unit. In the case of a CCD camera type scanner, scanning can be performed by appropriately adjusting the exposure time. The quantification method based on the scan result is performed by quantification software. The quantification software is not particularly limited, and quantification can be performed using the average, median, or the like of the fluorescence intensities of spots. Further, upon quantification, it is preferable to make adjustments in consideration of the dimensional accuracy of the spot range of a DNA fragment or the like, using the fluorescence intensity of a spot without a probe as the background.

### (3) Step (iii)

In this step, the bacterial load of a detection target bacterium is calculated from the signal intensity obtained by the above procedure. For example, there is a method for expressing the bacterial load as the SN ratio from the ratio of the signal intensity of a probe for detecting a detection target bacterium and the signal intensity of the background. Since the signal intensity is proportional to the abundance of a bacterium, the SN ratio can be used as is for analysis when it is not necessary to calculate the copy number.

Alternatively, it is also possible to use a method in which detection is performed under a plurality of conditions by changing the chromosomal DNA concentration of each bacterium in advance, the conversion factor (calibration curve) is obtained to calculate the chromosomal DNA concentration for each bacterium based on the signal intensity obtained under each concentration condition, and the chromosomal DNA concentration is calculated from the signal intensity obtained under each condition. In this case, the results can be calculated as the bacterial copy number.

Moreover, in any case, the signal intensity and the copy number may be corrected by considering the correction coefficient for the signal intensity of each detection target bacterium on the DNA chip. The order of correction and conversion of the signal intensity/copy number does not matter.

### 4. Determination of State of Periodontal Disease

According to the present invention, a signal intensity of a nucleic acid from an oral bacterial group present in an oral sample is measured, an abundance of the bacterial group from a measured value of the signal intensity is calculated, and the state of periodontal disease is determined using the obtained calculated value as an index.

For measurement of signal strength of a nucleic acid from an oral bacterial group present in an oral sample, any tool can be used. As described in the section 3 above, a method using a DNA chip and other methods such as a method using real-time PCR and a method using the FISH method can be exemplified.

Examples of signal intensity measurement values include the SN ratio obtained from a DNA chip, the Ct value obtained by real-time PCR, and the fluorescence intensity obtained by the FISH method.

The abundance ratio of bacterial groups means a correlation between a bacterial load of a bacterial species that increases as a periodontal pocket value increases (positively correlated bacterium) and a bacterial load of a bacterial species that decreases as a periodontal pocket value increases (negatively correlated bacterium).

Examples of such a correlation include: a ratio of the sum of bacterial loads of positively correlated bacteria and the sum of bacterial loads of negatively correlated bacteria (∑ bacterial loads of positively correlated bacteria/∑ bacterial loads of negative correlated bacteria); a value obtained by subtracting the sum of bacterial loads of positively correlated bacteria from the sum of bacterial loads of negatively correlated bacteria (∑ bacterial loads of negatively correlated bacteria-∑ bacterial loads of positively correlated bacteria); a ratio of a value obtained by multiplying the sum of bacterial loads of positively correlated bacteria by a predetermined coefficient and a value obtained by multiplying the sum of bacterial loads of negatively correlated bacteria by a predetermined coefficient (Σ coefficient × bacterial loads of positively correlated bacteria/∑ coefficient × bacterial loads of negatively correlated bacteria); and a sum of a value obtained by multiplying the sum of bacterial loads of positively correlated bacteria by a predetermined positive coefficient and a value obtained by multiplying the sum of bacterial loads of negatively correlated bacteria by a predetermined negative coefficient (∑ positive coefficient × bacterial loads of positively correlated bacteria + negative coefficient × bacterial loads of negatively correlated bacteria).

When the number of species of positively correlated bacteria and the number of species of negatively correlated bacteria are different, it is preferable to correct them such that the results are calculated from the same number of bacterial species.

For example, after calculating the sum of the SN ratios of "positively correlated bacteria" groups, the average SN ratio of "positively correlated bacteria" groups is calculated by dividing by the number of types of "positively correlated bacteria" groups. Similarly, after calculating the sum of the SN ratios of "negatively correlated bacteria" groups, the average SN ratio of "negatively correlated bacteria" groups is calculated by dividing by the number of types of "negatively correlated bacteria" groups.

Lastly, by taking the ratio of the average SN ratio of "positively correlated bacteria" groups and the average SN ratio of "negatively correlated bacteria" groups, a balance index can be obtained.

It is preferable to use, as the abundance ratio of bacterial groups, the "ratio" of the bacterial load of a bacterial species that increases as the periodontal pocket value increases and the bacterial load of a bacterial species that decreases as the periodontal pocket value increases.

The calculated value of the abundance ratio thus obtained is referred to as "balance index."

The numerator and denominator for calculating the balance index are arbitrarily determined, and either one may be the denominator or numerator. For example, the denominator may be the SN ratio of a bacterial species group that decreases as the periodontal pocket value increases, and the numerator may be the SN ratio of a bacterial species group that increases as the periodontal pocket value increases. Alternatively, the denominator may be the SN ratio of a bacterial species group that increases as the periodontal pocket value increases, and the numerator may be the SN ratio of a bacterial species group that decreases as the periodontal pocket value increases.

In conventional bacterial tests, the state of periodontal disease was determined by detecting bacteria corresponding to "bad bacteria." Since these are of bacterial species that increase after the periodontal pocket has grown to a certain extent, it was possible to obtain only information after deterioration. According to the present invention, since the balance index is calculated using a bacterial group corresponding to "good bacteria" such that the state of periodontal disease is determined, a healthy state can also be determined.

A more detailed explanation is as follows.

The bacterial load of "bad bacteria" is a monotonically increasing function with respect to the periodontal pocket value while the bacterial load of "good bacteria" is a monotonically decreasing function with respect to the periodontal pocket value.

When the vertical axis shows the bacterial load of "bad bacteria" and the horizontal axis shows the periodontal pocket value, there may be no value that allows determination within a periodontal pocket value range of 0 mm to 3 mm.

Meanwhile, when the vertical axis shows the index of bacterial load of "bad bacteria"/bacterial load of "good bacteria" and the horizontal axis shows the periodontal pocket value, the inflection point appears clearly in this function, which is advantageous that determination can be performed near the point. In addition, there is a value that allows determination within a periodontal pocket value range of 0 mm to 3 mm, and a healthy state can also be determined based on this value.

Further, according to the present invention, it is preferable to determine the state of periodontal disease by comparing the balance index with a cut-off value.

The cut-off value is a value having a function as a threshold value or a reference value of the abundance ratio of bacterial groups (balance index).

A signal intensity of a nucleic acid from an oral bacterial group present in an oral sample for standardization is measured, an abundance of the bacterial group from a measured value of the signal intensity is calculated, and an ROC curve is created from the obtained calculated value (balance index). The cut-off value can be determined from this ROC curve. The cut-off value is preferably selected such that the distance from the upper left of the ROC curve in a figure is small. However, it can be appropriately changed depending on the purpose (required sensitivity and specificity).

The cut-off value can also be determined by cluster analysis in addition to the above ROC curve. More specifically, it is considered that when cluster analysis is performed by the k-means method, the optimal number of clusters is examined and determined by the elbow method, or the number of clusters is automatically output using the x-means method, for example, and then, the cut-off value is set to an index corresponding to the boundary between the clusters.

In a first aspect of creating a discriminant model, the model can be a discriminant model based on the abundance ratio of "bacterial species that increases as the periodontal pocket value increases" and the abundance of "bacterial species that decreases as the periodontal pocket value increases" (balance index)

Examples of various bacteria are as described in Item 1. above.

Examples of the bacterial species that increases as the periodontal pocket value increases are not particularly limited, but preferably include at least one of bacterial species other than the Fusobacterium nucleatum species (a "progression index bacterium"). Specifically, at least one selected from the group consisting of Porphyromonas gingivalis, Tannerella forsythia, Treponema denticola, Campylobacter gracilis, Campylobacter rectus, Campylobacter showae, Fusobacterium periodonticum, Prevotella intermedia, Streptococcus constellatus, Aggregatibacter actinomycetemcomitans, Eikenella corrodens, Selenomonas sputigena, Treponema medium, Eubacterium saphenum, Eubacterium nodatum, Porphyromonas endodontalis, Filifactor alocis, Peptostreptococcus stomatis, and Treponema socranskii is preferable, and at least one selected from the group consisting of Porphyromonas gingivalis, Tannerella forsythia, Treponema denticola, Campylobacter gracilis, Campylobacter rectus, Campylobacter showae, Fusobacterium periodonticum, Prevotella intermedia, Streptococcus constellatus, Aggregatibacter actinomycetemcomitans, Eikenella corrodens, Selenomonas sputigena, Treponema medium, Eubacterium saphenum, Eubacterium nodatum, Porphyromonas endodontalis, and Filifactor alocis is more preferable.

The bacterial species used are preferably 4 or more species, more preferably 8 or more species, even more preferably 12 or more species, and particularly preferably 14 or more species. The bacterial species used are preferably 100 or less species, more preferably 75 or less species, even more preferably 50 or less species, and particularly preferably 25 or less species.

Examples of the bacterial species that decreases as the periodontal pocket value increases are not particularly limited. Specifically, at least one selected from the group consisting of Streptococcus parasanguinis, Haemophilus parainfluenzae, Streptococcus salivarius, Granulicatella adiacens, Rothia dentocariosa, Alloprevotella spp. (A. rava, OT 308), Veillonella rogosae, Porphyromonas pasteri, Prevotella shahii, Prevotella pallens, Veillonella atypica, Actinomyces graevenitzii, Megasphaera micronuciformis, Prevotella loescheii, Neisseria flavescens, Solobacterium moorei, Porphyromonas catoniae, Rothia mucilaginosa, Corynebacterium matruchotii, Eubacterium sulci, Gemella sanguinis, Prevotella melaninogenica, Prevotella denticola, Prevotella nigrescens, Campylobacter concisus, Capnocytophaga gingivalis, Capnocytophaga ochracea, Capnocytophaga sputigena, Streptococcus gordonii, Streptococcus intermedius, Streptococcus mitis, Streptococcus mitis bv 2, Actinomyces odontolyticus, Veillonella parvula, Actinomyces naeslundii II, Selenomonas noxia, SR1 sp. OT 345, Parvimonas micra, Streptococcus sobrinus, Actinomyces israelii, and Prevotella histicola are preferable, and at least one selected from the group consisting of Streptococcus parasanguinis, Haemophilus parainfluenzae, Streptococcus salivarius, Granulicatella adiacens, Rothia dentocariosa, Alloprevotella spp. (A. rava, OT 308), Veillonella rogosae, Porphyromonas pasteri, Prevotella shahii, Prevotella pallens, Veillonella atypica, Actinomyces graevenitzii, Megasphaera micronuciformis, Prevotella loescheii, Neisseria flavescens, Solobacterium moorei, Porphyromonas catoniae, Rothia mucilaginosa, Corynebacterium matruchotii, Eubacterium sulci, Gemella sanguinis, Prevotella melaninogenica, Prevotella denticola, Prevotella nigrescens, Campylobacter concisus, Capnocytophaga gingivalis, Capnocytophaga ochracea, Capnocytophaga sputigena, Streptococcus gordonii, Streptococcus intermedius, Streptococcus mitis, Streptococcus mitis bv 2, Actinomyces odontolyticus, Veillonella parvula, Actinomyces naeslundii II, and Selenomonas noxia are more preferable.

The bacterial species used are preferably 2 or more species, more preferably 10 or more species, and even more preferably 20 or more species. The bacterial species used are preferably 100 or less species, more preferably 75 or less species, even more preferably 50 or less species, and particularly preferably 25 or less species.

The abundance of the bacterial group is calculated from the measured value of the signal intensity of a nucleic acid from the bacterial group present in an oral preparation sample for standardization with a known state of periodontal disease, and the cut-off value is obtained from the calculated value (balance index).

Thereafter, in determining a sample with an unknown state of periodontal disease, after the bacterial groups is collectively detected, the balance index is calculated in the same manner, and the calculated value is compared with the cut-off value, thereby determining the state.

According to the discriminant model of the first aspect, a sample with an unknown state of periodontal disease can be determined into two groups based on the cut-off value.

As an example, a model for determining a non-disease state and a disease state will be described.

The non-disease state and the disease state can be defined as appropriate, but the non-disease state is defined herein as having a periodontal pocket depth of 1 mm to 3 mm and the disease state is defined herein as having a periodontal pocket depth of 5 mm or more. In other words, a periodontal pocket depth of 4 mm corresponds to a state for which it is unknown whether the state is a disease state or a non-disease state.

For samples with a periodontal pocket depth of 1-3 mm and samples with a periodontal pocket depth of 5 mm or more, the signal intensities of nucleic acids from various bacterial groups were measured, and the abundance ratio of various bacterial groups was calculated from the measured value, thereby obtaining a cut-off value from the calculated value (balance index). Thereafter, a balance index was calculated in the same manner for samples with a periodontal pocket depth of 4 mm, and by comparing this with the cut-off value obtained above, it is possible to determine whether the group with a periodontal pocket depth of 4 mm, the disease state of which was unknown, is in a non-disease state (similar to the group with a periodontal pocket depth of 1 mm to 3 mm) or a disease state (similar to the group with a periodontal pocket depth of 5 mm). The method of the present invention is especially useful in that it makes it possible to determine the group with a periodontal pocket depth of 4 mm, which has been conventionally difficult to determine.

In a second aspect of creating a discriminant model, the model can be a discriminant model based on the abundance ratio of "progression index bacteria (Fusobacterium nucleatum species)" and "bacterial species that decrease as the periodontal pocket value increases." The "bacterial species that increases as the periodontal pocket value increases" in the first aspect is replaced by the Fusobacterium nucleatum species which is a "progression index bacterium."

Examples of various bacteria are as described in Item 1. above.

The Fusobacterium nucleatum species is not particularly limited. Specifically, at least one selected from the group consisting of Fusobacterium nucleatum subsp. animalis, Fusobacterium nucleatum subsp. nucleatum, Fusobacterium nucleatum subsp. vincentii, and Fusobacterium nucleatum subsp. Polymorphum is preferable, and at least one or two selected from the group consisting of Fusobacterium nucleatum subsp. animali and Fusobacterium nucleatum subsp. Nucleatum are more preferable.

Bacterial species that decreases as the periodontal pocket value increases similar to those as in the first aspect are preferably used.

As in the case of the discriminant model of the first aspect, it is possible to calculate the cut-off value, thereby determining the two groups.

According to the discriminant model of the second aspect, the state when the periodontal pocket value is small can be better captured than in the first aspect.

There is an index similar to the index of the present invention. One example is the ratio of the sum of bacterial loads of Porphyromonas gingivalis, Tannerella forsythia, and Treponema denticola which are "bad bacteria" and the bacterial load of Fusobacterium nucleatum which is a progression index bacterium. This is different from the present invention in that the index of the present invention is the ratio of the "bad bacteria" group and the "good bacteria" group. According to the index of the present invention, the course of deterioration can be determined more clearly.

A more detailed explanation is as follows.

The bacterial load of "bad bacteria" is a monotonically increasing function with respect to the periodontal pocket value while the bacterial load of "good bacteria" is a monotonically decreasing function with respect to the periodontal pocket value.

In a case in which the vertical axis shows the index of the bacterial load of "bad bacteria"/the bacterial load of "progression index bacteria" and the horizontal axis shows the periodontal pocket value, the bacterial load of "bad bacteria" is small in a sample in the healthy state, and as a result, no bad bacteria may be detected. In other words, there may be no value that allows determination within a periodontal pocket value range of 0 mm to 3 mm.

Meanwhile, when the vertical axis shows the index of bacterial load of "bad bacteria" or "progression index bacteria"/bacterial load of "good bacteria" and the horizontal axis shows the periodontal pocket value, the inflection point appears clearly in this function, which is advantageous that determination can be performed near the point. In addition, there is a value that allows determination within a periodontal pocket value range of 0 mm to 3 mm, and a healthy state can also be determined based on this value.

Further, in the present invention, the following (a) and (b) can be used in combination as the abundance ratio of the bacterial groups:
(a) a correlation between the bacterial load of the bacterial species that increases as the periodontal pocket value increases (including at least one bacterial species other than Fusobacterium nucleatum species) and the bacterial load of the bacterial species that decreases as the periodontal pocket value increases; and
(b) a correlation between the bacterial load of Fusobacterium nucleatum species and the bacterial load of the bacterial species that decreases as the periodontal pocket value increases. When a DNA chip is used, a plurality of bacterial groups can be detected at once, and thus, a plurality of balance indexes can be calculated at the same time. Therefore, it is possible to simultaneously determine the two balance indexes as axes and classify into 2 × 2 = 4 groups.

The determination of the state of periodontal disease obtained by the present invention is a determination of a state estimated from the SN ratio proportional to the bacterial count or bacterial load, and does not represent an accurate pathological condition. In other words, a dentist needs to make a diagnosis for an accurate pathological condition. However, according to the present invention, since it is possible to determine independently of the periodontal pocket value, it is possible to early detect and treat periodontal disease from a new viewpoint and to contribute to prevention of the disease.

### 5. Determination of Therapeutic Effects on Periodontal Disease

Treatment refers to the treatment commonly performed by dentists and dental hygienists at the dental site. Examples of basic periodontal treatment include plaque control (tooth brushing instruction), tartar removal (scaling and root planing), and occlusal adjustment. Another example is surgical treatment that is performed when the results of re-evaluation tests after periodontal treatment indicate that cure is not achieved because tartar is deep inside the pocket and cannot be removed. Specific examples of surgical treatment include flap surgery, periodontal tissue regeneration therapy, and plastic surgery (periodontal plastic surgery). In addition, "supportive periodontal therapy (SPT)," which is continuous professional care after periodontal treatment, is important as an essential treatment for keeping a "stable condition" and maintaining a favorable prognosis of periodontal treatment.

Therapeutic effects on periodontal disease are determined by collecting a specimen before and after periodontal disease treatment and comparing and examining data.

The most basic idea is to utilize the clinical information of the specimen and clarify the bacteria that have increased or decreased before and after the treatment, thereby making it possible to objectively determine the therapeutic effects. In addition, the bacterial data after treatment make it possible to clarify the bacteria that were hard to be reduced by the treatment, and allow specific treatment.

According to the method of the present invention, therapeutic effects on periodontal disease can be determined from a plurality of bacterial balance indexes by performing the determination described in "4. Determination of State of Periodontal Disease" above before and after treatment. In particular, the state of periodontal disease showing the same periodontal pocket value can be further classified into four categories.

In a case in which the information of the periodontal pocket value is also taken into consideration, it is possible to determine whether the condition is stable. For example, even if the periodontal pocket value is 4 mm or more, it can be considered that the condition is stable if it is determined to be "mild" by the balance index described in "4. Determination of State of Periodontal Disease" above. On the other hand, even if the periodontal pocket value is 3 mm or less, it can be determined that the treatment may be considered if it is determined to be "severe" by the balance index.

In the above description, the SN ratio of a DNA chip was used as the measured value indicating the bacterial load, but any value that can be used as a synonymous value with the SN ratio of a DNA chip is within the scope of the present invention. For example, the bacterial copy number converted from the SN ratio of a DNA chip, the bacterial copy number quantified by real-time PCR, the Ct value indicating the bacterial load, the number of reads obtained as a result of next-generation sequencing, and the relative amount percentage converted from the number of reads can be considered.

### 6. Oligonucleotide probe set

The present invention provides an oligonucleotide probe for detecting oral bacteria including DNAs of the following (a) or (b):
(a) DNAs consisting of the base sequences set forth in SEQ ID NOS: 1 to 31
(b) DNAs that are 90% or more identical to the nucleotide sequences set forth in SEQ ID NOS: 1 to 33 and hybridize with a partial nucleotide sequence of the 16S rRNAgene or its complementary strand in the chromosomal DNAs of oral bacteria.

As the probe used in the present invention, any combination of DNAs among the DNAs having 33 types of nucleotide sequences set forth in SEQ ID NOS: 1 to 33 can be used. For example, among the DNAs consisting of the nucleotide sequences set forth in SEQ ID NOS: 1 to 33, any one type of DNA may be used, two types of DNA may be used in combination, 32 types of DNA may be used in combination, and 33 types of DNA may be used in combination.

Stringency conditions for "hybridization" and the like are the same as those described above.

In addition, examples of oral bacteria to be detected include at least one bacterium belonging to any of the genera Porphyromonas, Tannerella, Treponema, Prevotella, Campylobacter, Fusobacterium, Streptococcus, Aggregatibacter, Capnocytophaga, Eikenella, Actinomyces, Veillonella, and Selenomonas as described above.

Furthermore, the present invention provides a microarray for detecting oral bacteria, in which the above-described oligonucleotide probe set is arranged. According to the present invention, as the microarray, the one described in "2. DNA Chip for Detecting Oral Bacterial Gene Used for Measuring Oral Bacterial Load" can be used.

Hereinafter, the present invention will be described in more detail with reference to the Examples below, but the present invention is not limited thereto.

### Examples

### [Example 1-1]

### Method for Determining State of Periodontal Disease

### Detection of Oral Bacteria in Subgingival Plaque Specimen

### <Preparation of Subgingival Plaque Specimen>

At the Osaka University Dental Hospital, subgingival plaque was collected from 220 male and female subjects in their 20s to 70s before treatment of periodontal disease. Two absorbent paper points (ISO Color-Coded) #40 (manufactured by DENTSPLY MAILLEFER) were inserted into periodontal pockets and placed herein for 30 seconds. Then, the paper points were put into a microtube containing 0.15 mL of sterile distilled water, and vortexed for 20 seconds. The paper points were removed with sterile forceps and frozen and stored at -20°C until detection.

### <Acquisition of Clinical Information>

The clinical information of all specimens was digitized according to the following criteria. The following four items are indexes that are widely used in dentistry.
(i) Periodontal pocket depth (Pd): Pd refers to the distance from the gingival margin to the tip of a periodontal probe when the probe is inserted into the pocket. Pd was digitized in units of 1 mm. The term "periodontal probe" as used herein means a pocket measuring instrument (perio probe).
(ii) Bleeding on probing (BOP): BOP refers to the presence or absence of bleeding when a periodontal probe is inserted into the pocket. The case in which there was no bleeding was set to 0, and the case in which there was bleeding was set to 1.
(iii) Gingival Index (GI): GI refers to the degree of gingival inflammation. The case in which there was no inflammation was set to 0, the case in which there was mild inflammation was set to 1, the case in which there was moderate inflammation was set to 2, and the case in which there was severe inflammation was set to 3.
(iv) Plaque Index (PlI): PlI refers to the amount of plaque deposition on the tooth surface adjacent to the gingiva. The case in which there was no plaque was set to 0, the case in which no plaque was visually observed, but plaque was found by probe rubbing was set to 1, the case in which plaque was visually observed was set to 2, and the case in which a large amount of plaque was observed was set to 3.

### <PCR>

All the specimens that had been frozen and stored were thawed and used as a PCR template. In order to amplify the sequence of the detection target region of 16S rRNA of an oral bacterium in each specimen, PCR was carried out under the reaction conditions with the reaction solution composition described below. PCR was performed using, as a PCR kit, Premix Ex Taq (trademark) Hot Start Version (manufactured by Takara Holdings Inc.) by GeneAmp9700 (manufactured by Applied Biosystems). As primers, primers having the following sequences were used. The forward primer used had the 5' end labeled with Cy5.
Forward primer (for bacterial amplification):
   5'-Cy5-TCCTACGGGAGGCAGCAGT-3' (SEQ ID NO: 37)
Reverse primer (for bacterial amplification):
   5'-CAGGGTATCTAATCCTGTTTGCTACC-3' (SEQ ID NO: 38)
Forward primer (for absolute load index amplification):
   5'-Cy5-GAGAAGCCTACACAAACGTAACGTC-3' (SEQ ID NO: 39)
Reverse primer (for absolute load index amplification):
   5'-CTCTAAAGACCGCTCTATCTCGG-3' (SEQ ID NO: 40)

### <Reaction Solution Composition>

### 2 × Premix Ex Taq (registered trademark)

Hot Start Version: 10 µL
   4 µM forward primer (for bacterial amplification): 1 µL
   4 µM reverse primer (for bacterial amplification): 1 µL
   4 µM forward primer (for absolute load index amplification): 1 µL
   4 µM reverse primer (for absolute load index amplification): 1 µL
Template DNA: 5 µL
Absolute load index: 1 µL
Total: 20 µL

### <Reaction Conditions>

After heating at 95°C for 1 minute, a total of 40 cycles of "dissociation: 98°C (10 sec) → annealing: 60°C (30 sec) → synthesis: 72°C (20 sec)" were performed, and the mixture was cooled at 4°C, thereby obtaining an amplification product.

### <DNA Chip: Production of DNA Chip for Detecting Oral Bacteria>

A through-hole type DNA chip was produced by a method similar to the method described in Example 2-1 of JP Patent Publication (Kokai) No. 2007-74950A (method for detecting methylated DNA and/or unmethylated DNA).

Note that as oligonucleotide probes mounted herein, probes having the sequence information shown in Table 4 were used with reference to the information on bacterial species in Non Patent Literature 1: Socransky, S. S. et al. J Clin Microbiol, 37, 1426-30, 1999.

**[Table 4]**

| SEQ ID NO | | |
|---|---|---|
| 1 | Porphyromonas gingivalis probe | TTCAATGCAATACTCGTATC |
| 2 | Tannerella forsythia probe | CACGTATCTCATTTTATTCC |
| 3 | Treponema denticola probe | CCTCTTCTTCTTATTCTTCAT |
| 5 | Campylobacter gracilis probe1 | GCCTTCGCAATAGGTATT |
| 7 | Campylobacter rectus probe2 | GTCATAATTCTTTCCCAAGA |
| 8 | Campylobacter showae probe | CAATGGGTATTCTTCTTGAT |
| 9 | Fusobacterium nucleatum subsp. vincentii probe | TAGTTATACAGTTTCCAACG |
| 10 | Fusobacterium nucleatum subsp. polymorphum probe | CCAGTACTCTAGTTACACA |
| 11 | Fusobacterium nucleatum subsp. animalis probe5 | TTTCTTTCTTCCCAACTGAA |
| 12 | Fusobacterium nucleatum subsp. nucleatum probe7 | TACATTCCGAAAAACGTCAT |
| 13 | Fusobacterium periodonticum probe | TATGCAGTTTCCAACGCAA |
| 14 | Prevotella intermedia probe | GGGTAAATGCAAAAAGGCA |
| 15 | Prevotella nigrescens probe | CTTTATTCCCACATAAAAGC |
| 16 | Streptococcus constellatus probe | AAGTACCGTCACTGTGTG |
| 17 | Aggregatibacter actinomycetemcomitans probe1 | GTCAATTTGGCATGCTATTA |
| 18 | Campylobacter concisus probe | CCCAAGCAGTTCTATGGT |
| 19 | Capnocytophaga gingivalis probe | TACACGTACACCTTATTCTT |
| 20 | Capnocytophaga ochracea probe | CAACCATTCAAGACCAACA |
| 21 | Capnocytophaga sputigena probe1 | TACACGTACACCTTATTCTT |
| 22 | Eikenella corrodens probe2 | CTCTAGCTATCCAGTTCAG |
| 23 | Streptococcus gordonii probe | CACCCGTTCTTCTCTTACA |
| 25 | Streptococcus intermedius probe | ACAGTATGAACTTTCCATTCT |
| 27 | Streptococcus mitis probe6 | TCTCCCCTCTTGCACTCA |
| 28 | Streptococcus mitis by 2 probe | TCCCCTCTTGCACTCAAGT |
| 29 | Actinomvces odontolvticus probe | AAGTCAGCCCGTACCCA |
| 30 | Veillonella parvula probe | TATTCGCAAGAAGGCCTT |
| 31 | Actinomyces naeslundii II probe | CCACCCACAAGGAGCAG |
| 32 | Selenomonas noxia probe1 | TTCGCATTAGGCACGTTC |

### <Hybridization with DNA Chip>

A hybridization solution was prepared by mixing the respective solutions as described below.

### DNA amplification product obtained after PCR 20 µL

1M Tris-HCl: 48 µL
1M NaCl: 48 µL
0.5% Tween20: 20 µL
Water: 64 µL
Total: 200 µL

The hybridization solution in an amount of 200 µL was brought into contact with the DNA chip, followed by hybridization at 50°C for 2 hours After the hybridization, the DNA chip was washed under the following conditions.

Washing with 1000 µL of 0.24 M Tris·HCl/0.24 M NaCl/0.05% Tween-20 solution for 220 seconds was repeated 12 times. Then, washing with 1000 µL of 0.24 M Tris·HCl/0.24 M NaCl for 220 seconds was repeated 4 times. After the completion of washing, each chip was transferred to a 0.24 M Tris·HCl/0.24M NaCl mixed solution at room temperature.

### <Detection>

After the washing, the fluorescence intensity of each spot of the DNA chip was measured under the following conditions using Genopal Reader (manufactured by Mitsubishi Chemical Corporation).

### <Detection Conditions>

Center excitation wavelength: 633 nm
Exposure time: 0.1, 1,4, and 40 seconds

### <Results>

The fluorescence intensity of a spot with a probe mounted thereon for a detection target bacterium was subtracted by the background value (the median of the fluorescence intensities of spots without a probe), thereby calculating the SN ratio derived from hybridization. Data of the SN ratio were obtained for each of detection target bacteria for all 220 samples.

### <Correlation between Clinical Information and Bacterial Load (SN Ratio)>

A scatter diagram of the data of the periodontal pocket depth (Pd) value and the SN ratio showing the bacterial load of each bacterium for 28 types of bacteria was prepared and shown in Figure 1 (Figures 1-1 to 1-7). The vertical axis represents the SN ratio of each bacterium, and the horizontal axis represents the pocket depth (Pd) value in Figure 1. The correlation coefficient was calculated for each of the 28 types (Table 5).

**[Table 5]**

| | Bacterial Species Name | Correlation Coefficient with Pd |
|---|---|---|
| 1 | Porphyromonas gingivalis | 0.468 |
| 2 | Tannerella forsythia | 0.412 |
| 3 | Treponema denticola | 0.516 |
| 4 | Campylobacter gracilis | 0.317 |
| 5 | Campylobacter rectus | 0.355 |
| 6 | Campylobacter showae | 0.350 |
| 7 | Fusobacterium nucleatum subsp. vincentii | 0.335 |
| 8 | Fusobacterium nucleatum subsp. polymorphum | 0.202 |
| 9 | Fusobacterium nucleatum subsp. animalis | 0.343 |
| 10 | Fusobacterium nucleatum subsp. nucleatum | 0.378 |
| 11 | Fusobacterium periodonticum | 0.386 |
| 12 | Prevotella intermedia | 0.199 |
| 13 | Prevotella nigrescens | -0.005 |
| 14 | Streptococcus constellatus | 0.199 |
| 15 | Aggregatibacter actinomycetemcomitans | 0.211 |
| 16 | Campylobacter concisus | -0.189 |
| 17 | Capnocytophaga gingivalis | -0.124 |
| 18 | Capnocytophaga ochracea | -0.101 |
| 19 | Capnocytophaga sputigena | -0.183 |
| 20 | Eikenella corrodens | 0.134 |
| 21 | Streptococcus gordonii | -0.158 |
| 22 | Streptococcus intermedius | -0.096 |
| 23 | Streptococcus mitis | -0.409 |
| 24 | Streptococcus mitis bv 2 | -0.418 |
| 25 | Actinomvces odontolvticus | -0.133 |
| 26 | Veillonella parvula | -0.190 |
| 27 | Actinomvces naeslundii II | -0.170 |
| 28 | Selenomonas noxia | -0.042 |

Next, the 28 types of bacteria were roughly classified into bacterial species that increases as the periodontal pocket value increases and bacterial species that decreases as the periodontal pocket value increases based on the positive or negative correlation coefficient. The group of "bacterial species that increases as the periodontal pocket value increases" was set to consist of 15 bacterial species which were Porphyromonas gingivalis, Tannerella forsythia, Treponema denticola, Campylobacter gracilis, Campylobacter rectus, Campylobacter showae, Fusobacterium nucleatum subsp. vincentii, Fusobacterium nucleatum subsp. polymorphum, Fusobacterium nucleatum subsp. animalis, Fusobacterium nucleatum subsp. nucleatum, Fusobacterium periodonticum, Prevotella intermedia, Streptococcus constellatus, Aggregatibacter actinomycetemcomitans, and Eikenella corrodens.

The group of "bacterial species that decreases as the periodontal pocket value increases" was set to consist of 13 bacterial species which were Prevotella nigrescens, Campylobacter concisus, Capnocytophaga gingivalis, Capnocytophaga ochracea, Capnocytophaga sputigena, Streptococcus gordonii, Streptococcus intermedius, Streptococcus mitis, Streptococcus mitis bv 2, Actinomyces odontolyticus, Veillonella parvula, Actinomyces naeslundii II, and Selenomonas noxia.

Next, the sum of the SN ratios of the group of "bacterial species that increases as the periodontal pocket value increases" was calculated using the SN ratio values of the graph shown in Figure 1, and then, the sum was divided by the number of bacterial types in the group of "bacterial species that increases as the periodontal pocket value increases," thereby calculating the average SN ratio of the group of "bacterial species that increases as the periodontal pocket value increases." Similarly, the sum of the SN ratios of the group of "bacterial species that decreases as the periodontal pocket value increases" was calculated, and then, the sum was divided by the number of bacterial types in the group of "bacterial species that decreases as the periodontal pocket value increases," thereby calculating the average SN ratio of the group of "bacterial species that decreases as the periodontal pocket value increases."

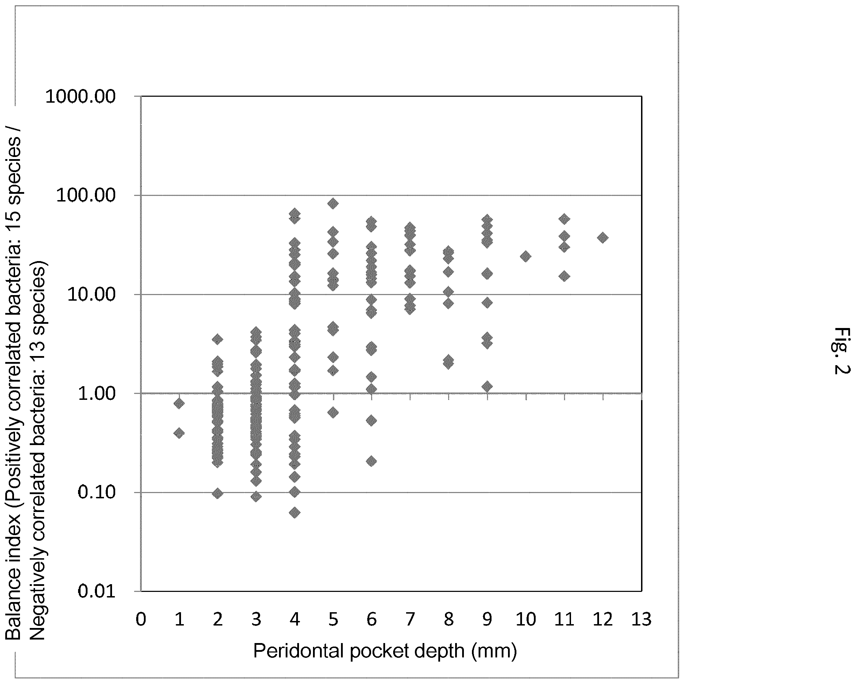

Lastly, the balance index of bacterial groups was calculated by taking the ratio of the average SN ratio of the group of "bacterial species that increases as the periodontal pocket value increases" and the average SN ratio of the group of "bacterial species that decreases as the periodontal pocket value increases." Figure 2 shows a scatter diagram in which the vertical axis represents the balance index (15 species in the group of "bacterial species that increases as the periodontal pocket value increases"/13 species in the group of "bacterial species that decreases as the periodontal pocket value increases") and the horizontal axis represents the periodontal pocket depth (Pd) value.

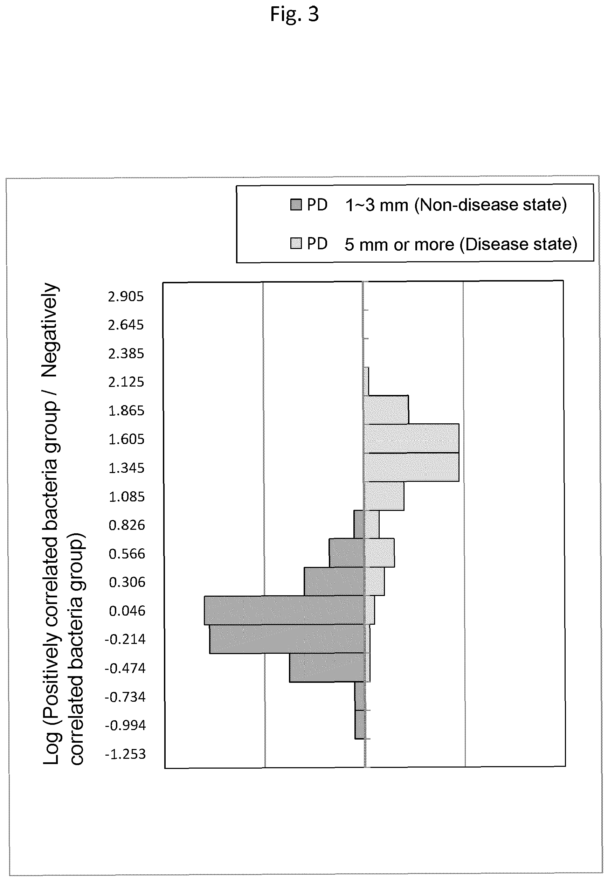

According to the present invention, the non-disease state is defined as having a periodontal pocket depth of 1 mm to 3 mm and the disease state is defined as having a periodontal pocket depth of 5 mm or more. It was decided to determine the disease state/disease state by creating a discriminant model using data with a periodontal pocket depth of 1 mm to 3 mm and data with a periodontal pocket depth of 5 mm or more from the data shown in Figure 2 and using data with a periodontal pocket depth of 4 mm as test data. A histogram was created for the data with a periodontal pocket depth of 1 mm to 3 mm and the data with a periodontal pocket depth of 5 mm or more from the data shown in Figure 2 (Figure 3). The vertical axis of Figure 3 represents a value obtained by converting the balance index of Figure 2 with LOG10. The horizontal axis represents frequency.

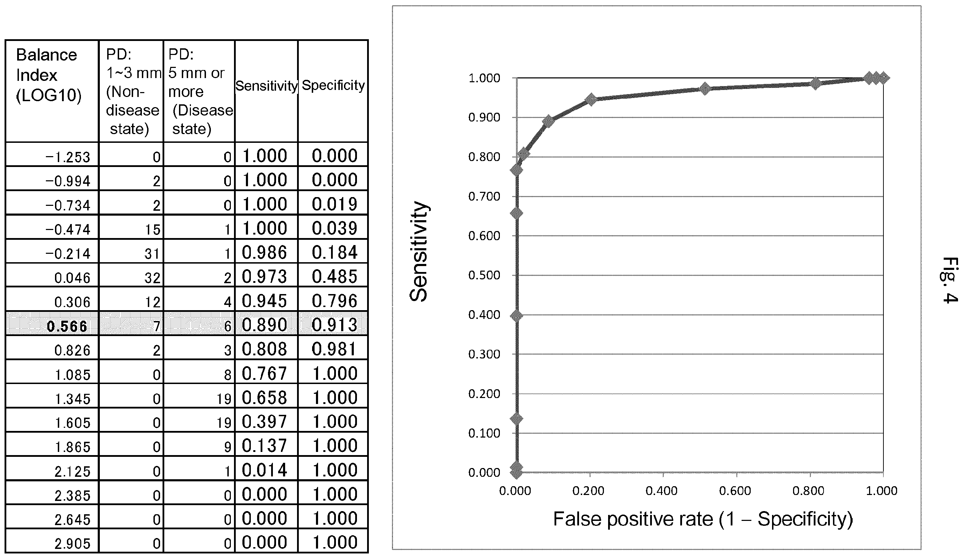

ROC analysis was performed based on the data in Figure 3 (right in Figure 4), and the point near the upper left (balance index (LOG10) = 0.566) was taken as the cut-off value. In this case, it was found from the analysis that a test was performed with a sensitivity of 0.890 and a specificity of 0.913 (left in Figure 4).

Next, using this test, data with a periodontal pocket depth (Pd) of 4 mm were determined. The 4-mm data were the data with a pocket depth of 4 mm in Figure 2, and there were data for 44 individuals.

When these data were determined with a cut-off value of 0.566, 18 subjects had a balance index (LOG10) value larger than the cut-off value (Table 6: 18 subjects from the bottom). It was determined that these subjects had a periodontal disease state as advanced as the disease state with a periodontal pocket depth of 5 mm or more.

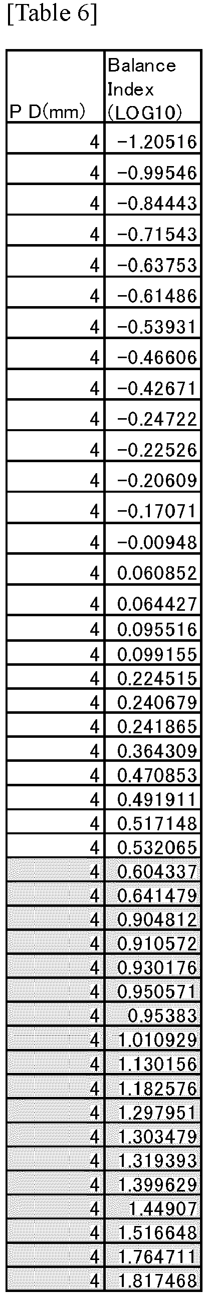

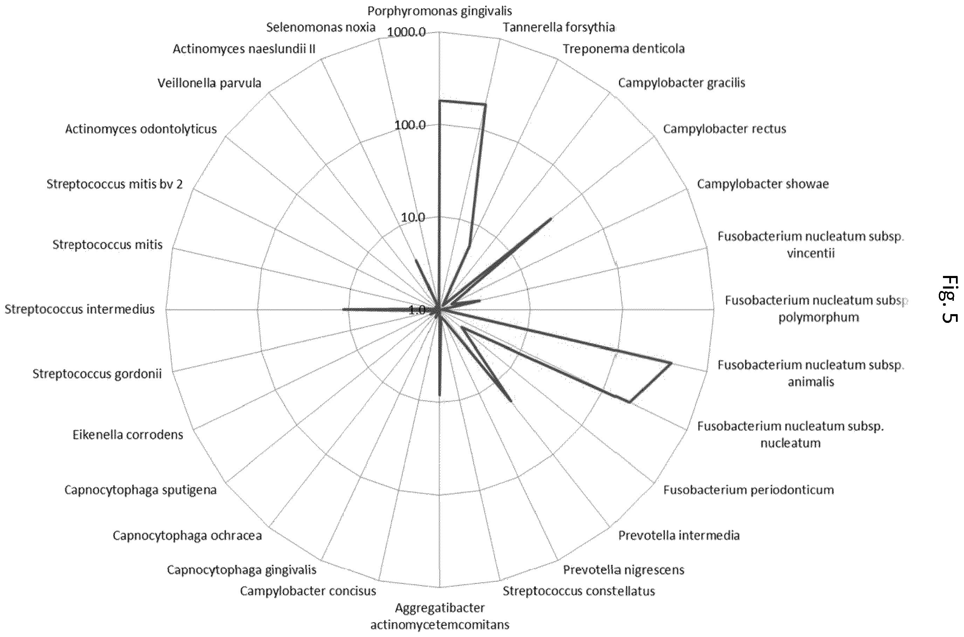

As an example of a case in which the disease state was determined to be similar to a disease state with a periodontal pocket depth of 5 mm or more, the SN ratio of each bacterium in a sample with a balance index (LOG10) value of 1.516648 is shown in Figure 5. It was confirmed from Figure 5 that there is a pattern that the SN ratio of the group of "bacterial species that increases as the periodontal pocket value increases" is greater than the SN ratio of the group of "bacterial species that decreases as the periodontal pocket value increases."

### [Example 1-2]

### Method for Determining Course of Periodontal Disease

A discriminant model of the course of periodontal disease was created using the same data as the data in Example 1.

The group of "bacterial species that increases as the periodontal pocket value increases" and the group of "bacterial species that decreases as the periodontal pocket value increases" were the same as those in Example 1.
Fusobacterium nucleatum subsp. animalis and Fusobacterium nucleatum subsp. nucleatum in the group of "bacterial species that increases as the periodontal pocket value increases" were selected as "progression indicator bacteria."

Next, the average SN ratio of the group of "progression index bacteria" was calculated by calculating the sum of SN ratios of "progression index bacteria" (Fusobacterium nucleatum subsp. animalis and Fusobacterium nucleatum subsp. nucleatum) using the SN ratio values of the graph shown in Figure 1 and dividing the calculated value by the number of bacterial types, i.e., "2." Similarly, the average SN ratio of the group of "bacterial species that decreases as the periodontal pocket value increases" was calculated.

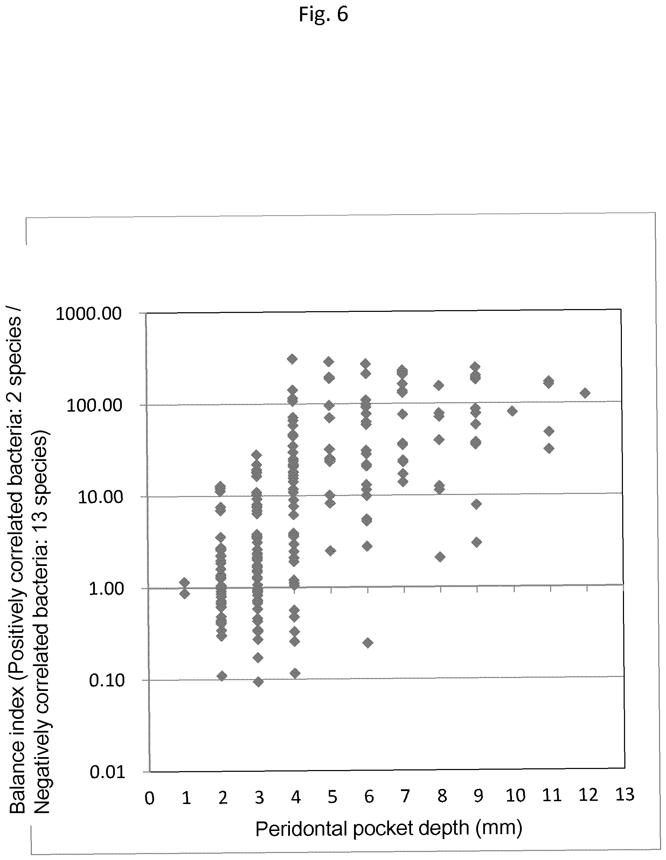

Lastly, the balance index of bacterial groups was calculated by taking the ratio of the average SN ratio of the group of "progression index bacteria" and the average SN ratio of the group of "bacterial species that decreases as the periodontal pocket value increases." Figure 6 shows a scatter diagram in which the vertical axis represents the balance index (2 species of "progression index bacteria"/13 species of the "good bacteria" group) and the horizontal axis represents the periodontal pocket depth (Pd) value.

It was decided to determine the disease state/disease state by creating a discriminant model using data with a periodontal pocket depth of 1 mm to 3 mm and data with a periodontal pocket depth of 5 mm or more from the data shown in Figure 6 and using data with a periodontal pocket depth of 4 mm as test data.

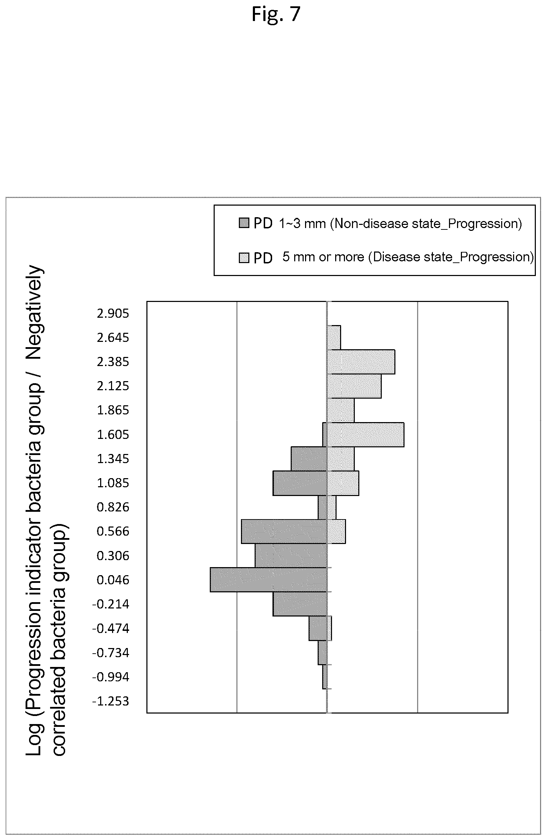

A histogram was created for the data with a periodontal pocket depth of 1 mm to 3 mm and the data with a periodontal pocket depth of 5 mm or more from the data shown in Figure 6 (Figure 7). The vertical axis of Figure 7 represents a value obtained by converting the balance index of Figure 6 with LOG10. The horizontal axis represents frequency.

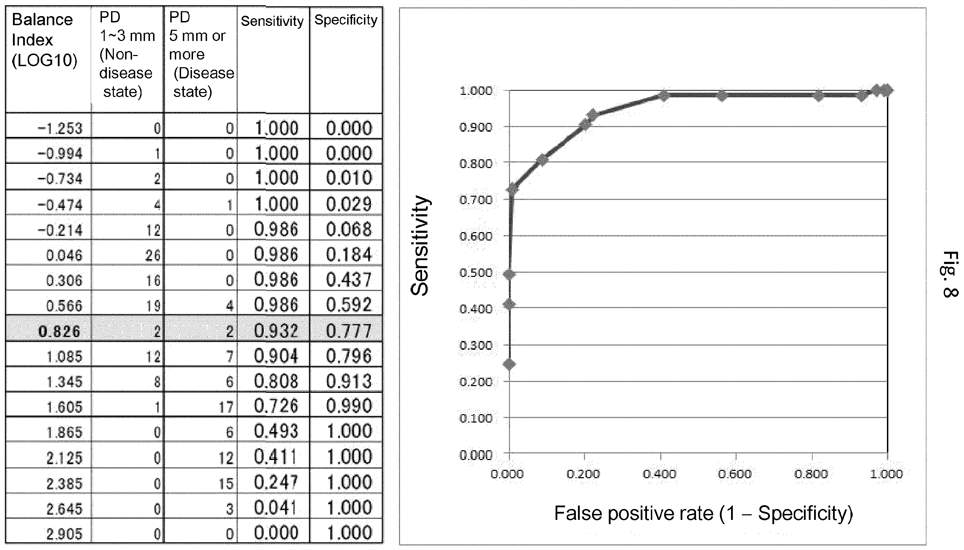

ROC analysis was performed based on the data in Figure 7 (right in Figure 8), and the point near the upper left (balance index (LOG10) = 0.826) was taken as the cut-off value. In this case, it was found from the analysis that a test was performed with a sensitivity of 0.932 and a specificity of 0.777.

Next, using this test, data with a periodontal pocket depth (Pd) of 4 mm was determined. The 4-mm data were the data with a pocket depth of 4 mm in Figure 6, and there were data for 44 individuals.

When these data were determined with a cut-off value of 0.826, 27 subjects had a balance index (LOG10) value larger than the cut-off value (Table 7: 27 subjects in colored columns (1st to 27th individuals from the bottom of Table 7). It was determined that these subjects had a periodontal disease state in the course of periodontal disease comparable to the disease state with a periodontal pocket depth of 5 mm or more.

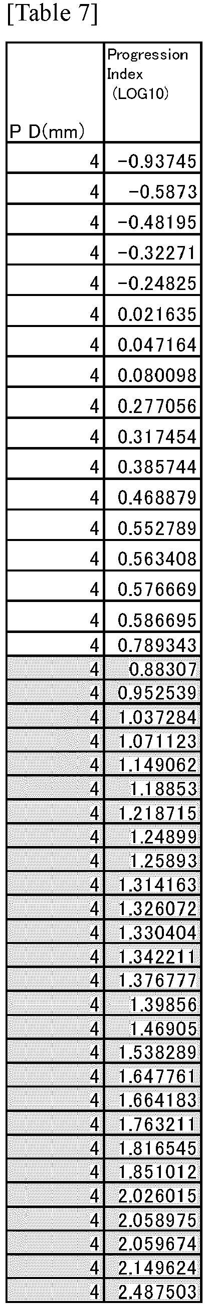

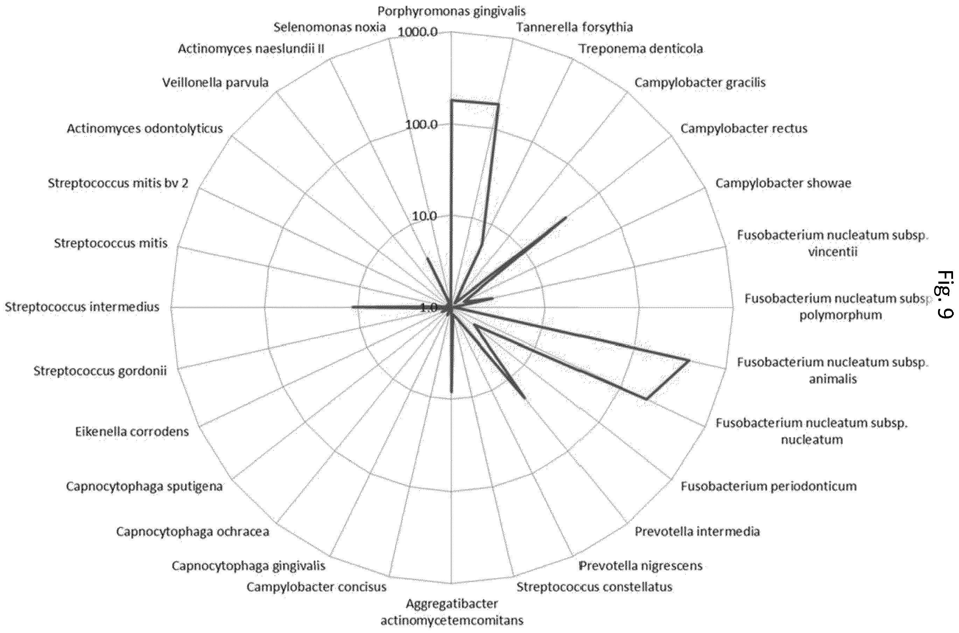

The SN ratio of each bacterium in a sample with a balance index (LOG10) value of 0.883 is shown in Figure 9 (the sample is the same as in Figure 5). It was confirmed from Figure 9 that there is a pattern that the SN ratio of the genus Fusobacterium in the group of "progression index bacteria" is greater than the SN ratio of the group of "good bacteria."

### [Example 1-3]

### Subdivision of State of Periodontal Disease

A discriminant model was created in the same manner as in Examples 1-1 and 1-2 using the data in Examples 1-1 and 1-2 except that the vertical axis represents the balance index (LOG10) in Example 1-1 and the horizontal axis represents the balance index (LOG10) in Example 1-2. As a result of determination of the site data that had been grouped together as "4-mm pocket" data so far, the data could be classified into four groups.

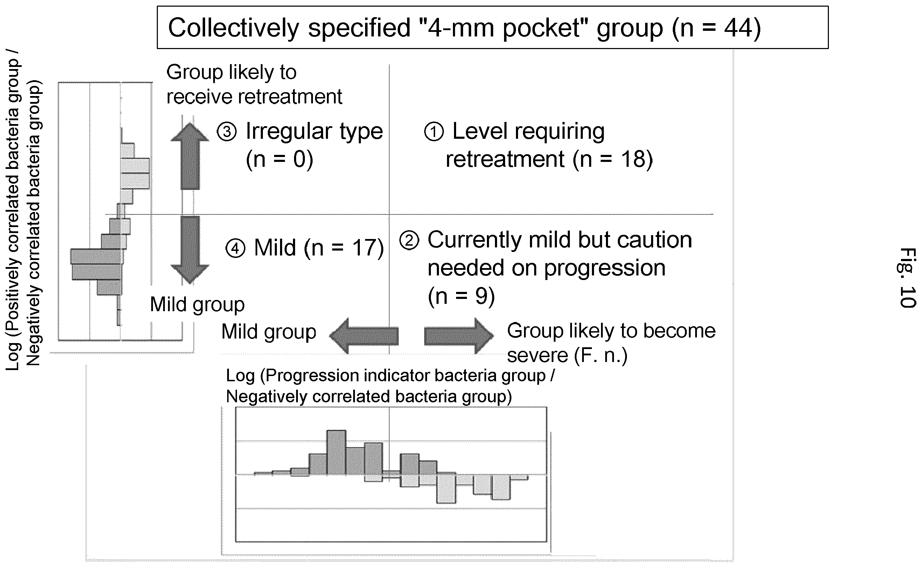

The results are summarized in Figure 10.

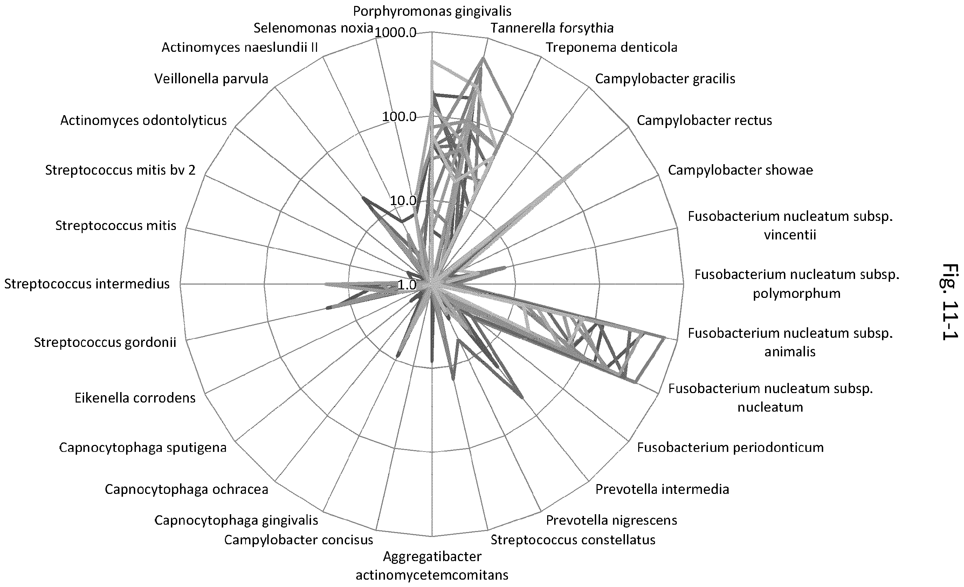

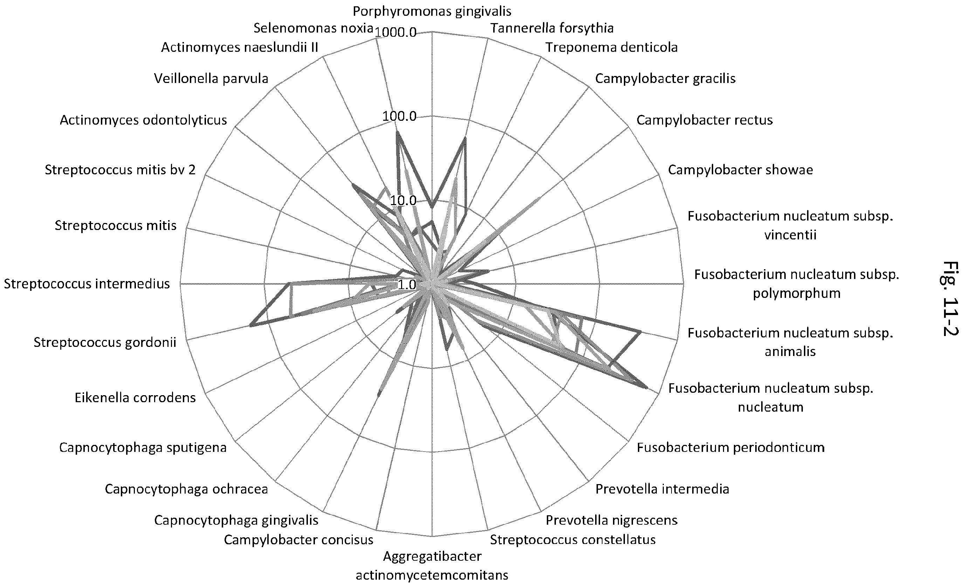

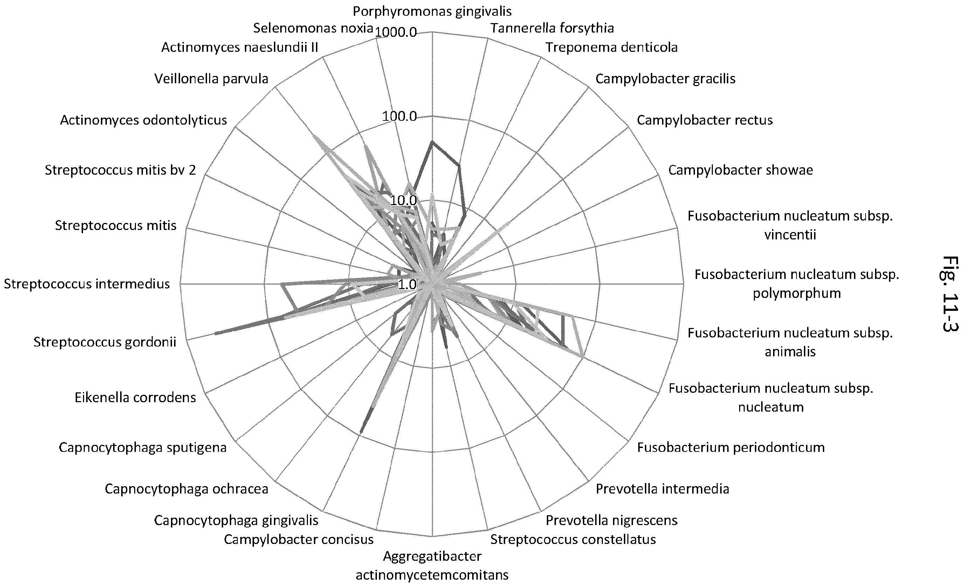

Figure 11 shows the SN ratio of each bacterium in the samples in the states of (a), (b), and (d): from the top, condition (a) (at the level requiring re-treatment: n = 18), condition (b) (currently mild but caution needed on progression: n = 19), condition (d) (mild: n = 17).

### [Example 1-4]

### Difference in Determination Ability Depending on Selected Bacteria

The 28 types of bacteria were roughly classified into bacterial species that increases as the periodontal pocket value increases and bacterial species that decreases as the periodontal pocket value increases based on the positive or negative correlation coefficient in the same manner as in Example 1-1 using data identical to those in Example 1-1.

Of these, the group of bacterial species that increases as the periodontal pocket value increases was set to consist of 5 types of bacteria known as periodontal disease-related bacteria and 1 type of bacteria of Fusobacterium nucleatum species. Specifically, 6 bacterial types, namely Porphyromonas gingivalis, Tannerella forsythia, Treponema denticola, Campylobacter rectus, Fusobacterium nucleatum subsp. nucleatum, and Prevotella intermedia, were selected.

In addition, the group of bacterial species that decreases as the periodontal pocket value increases was set to consist of 4 bacterial types with a relatively large SN ratio, namely Capnocytophaga gingivalis, Streptococcus gordonii, Streptococcus intermedius, and Veillonella parvula. In other words, data of a total of 10 bacterial species were used for creating a discriminant model.

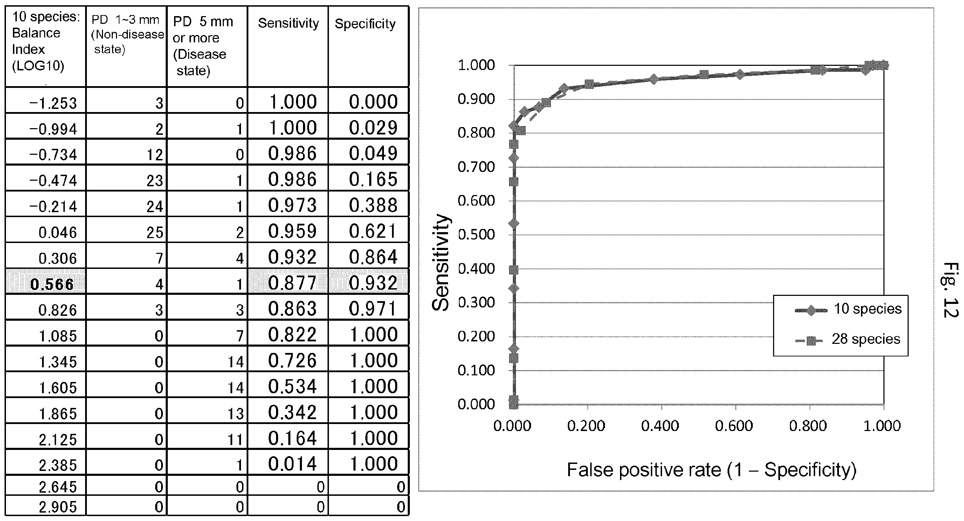

ROC analysis was performed after calculating the balance index (LOG10) in the same manner as in Example 1-1, and the results were compared with those in Example 1-1. The results are shown in Figure 12.

As a result, it was found that a test was performed for determination with a sensitivity of 0.877 and a specificity of 0.932 at a cut-off value obtained using 10 types of bacterial species (0.566) (or with a sensitivity of 0.890 and a specificity of 0.913 for 28 types of bacterial species as described above).

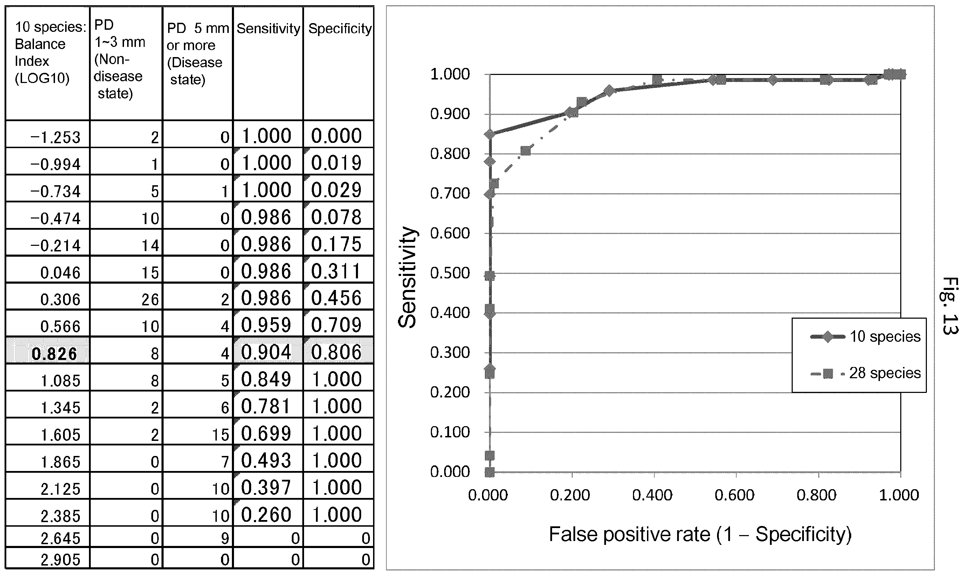

In addition, the balance index (LOG10) was calculated and ROC analysis was performed in the same manner as in Example 1-2, and comparison was made with the case of Example 1-2. The results are shown in Figure 13.

As a result, it was found that a test was performed for determination with a sensitivity of 0.904 and a specificity of 0.806 at a cut-off value obtained using 10 types of bacterial species (0.826) (or with a sensitivity of 0.932 and a specificity of 0.777 for 28 types of bacterial species as described above).

From these results, it was considered that the 28 types of bacterial species had smoother curves and the accuracy of discrimination was improved. However, even when the 10 types of bacterial species were selected, the sensitivity and specificity were not significantly lowered by selecting the bacterial species suitably.

### [Example 2-1]

### Bacterial Detection of Plaque Specimen Before and After Treatment and Determination of Therapeutic Effects on Periodontal Disease

### <Preparation of Plaque Specimen>

To compare the bacterial load of a plaque specimen before and after treatment, at the Osaka University School of Dentistry, subgingival plaque before and after treatment of periodontal disease was collected from 61 cases of males and females in their 20s to 70s. The basic periodontal treatment including tartar removal (scaling/root planing) was performed as treatment.

Two absorbent paper points (ISO Color-Coded)#40 (manufactured by DENTSPLY MAILLEFER) were inserted into periodontal pockets and placed herein for 30 seconds. Then, the paper points were put into a microtube containing 0.15 mL of sterile distilled water, and vortexed for 20 seconds. The paper points were removed with sterile forceps and frozen and stored at -20°C until detection.

### <DNA Chip: Production of DNA Chip for Detecting Oral Bacteria>

A through-hole type DNA chip was produced by a method similar to the method described in Example 1-1 of JP Patent Publication (Kokai) No. 2007-74950A (method for detecting methylated DNA and/or unmethylated DNA). As oligonucleotide probes mounted herein, probes having the sequence information shown in Table 8 were used. PCR, hybridization with a DNA chip, and detection were performed in the same manner as in Example 1-1.

**[Table 8]**

| SEQ ID NO | | |
|---|---|---|
| 1 | Porphyromonas gingivalis probe | TTCAATGCAATACTCGTATC |
| 2 | Tannerella forsythia probe | CACGTATCTCATTTTATTCC |
| 4 | Treponema denticola probe1 | CTCTTCTTCTTATTCTTCAT |
| 6 | Campylobacter rectus probe | ATTCTTTCCCAAGAAAAGGA |
| 12 | Fusobacterium nucleatum subsp. nucleatum probe7 | TACATTCCGAAAAACGTCAT |
| 14 | Prevotella intermedia probe | GGGTAAATGCAAAAAGGCA |
| 15 | Prevotella nigrescens probe | CTTTATTCCCACATAAAAGC |
| 17 | Aggregatubacter actinomycetemcomitans probe1 | GTCAATTTGGCATGCTATTA |
| 19 | Capnocytophaga gingivalis probe | TACACGTACACCTTATTCTT |
| 24 | Streptococcus gordonii probe1 | CACCCGTTCTTCTCTTAC |
| 26 | Streptococcus intermedius probe1 | CAGTATGAACTTTCCATTCT |
| 30 | Veillonella parvula probe | TATTCGCAAGAAGGCCTT |
| 33 | Streptococcus mutans probe | CACACGTTCTTGACTTAC |
| 34 | Total load index probe | CGTATTACCGCGGCTGCTGGCAC |
| 35 | Absolute load index 15 probe | CTATTCGACCAGCGATATCACTACGTAGGC |

### <Results>

### <Calculation of SN Ratio Data>

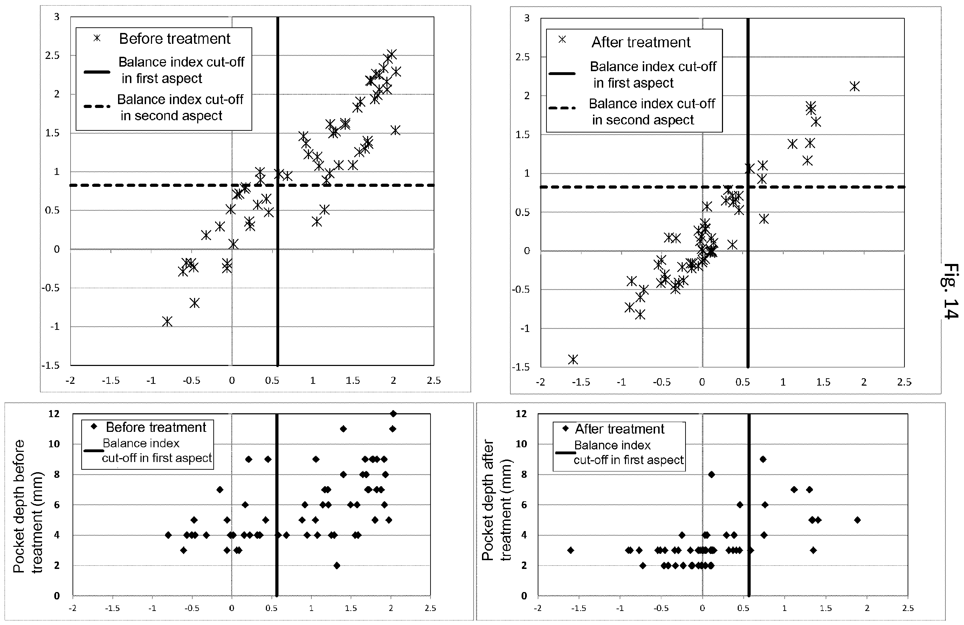

The fluorescence intensity of a spot with a probe mounted thereon for a detection target bacterium was subtracted by the background value (the fluorescence intensity of a spot without a probe), thereby calculating the SN ratio derived from hybridization. Subsequently, 10 types of bacterial species the same as those in Example 1-4, which means that the group of "bacterial species that increases as the periodontal pocket value increases" consisting of 6 bacterial species, namely Porphyromonas gingivalis, Tannerella forsythia, Treponema denticola, Campylobacter rectus, Fusobacterium nucleatum subsp. nucleatum, and Prevotella intermedia, and the group of "bacterial species that decreases as the periodontal pocket value increases" consisting of 4 bacterial species, namely Capnocytophaga gingivalis, Streptococcus gordonii, Streptococcus intermedius, and Veillonella parvula, were selected to perform determination on two axes in the same manner as in Example 1-3. The results are shown in the two graphs in the upper half of Figure 14.

The left side shows the results before treatment and the right side shows the results after treatment. When the positions of plots were observed before and after treatment, the plots entirely moved from the upper right ((a) at the level requiring retreatment) to the lower left ((d) mild), which allowed determination of effects of the treatment. Further, the two graphs in the lower half of Figure 14 are graphs of the periodontal pocket (vertical axis) and the balance index (horizontal axis). The left side is before treatment and the right side is after treatment. From these results, it was possible to determine that the disease state was stable in each plot in which the balance index did not exceed the determination value even with a periodontal pocket depth of about 4 mm after the treatment. On the other hand, it was possible to determine that each plot in which the balance index exceeded the determination value even with a periodontal pocket depth of 3 mm should be considered for treatment.

### [Example 2-2]

### <Calculation of Copy Number Data>

The fluorescence intensity of a spot with a probe mounted thereon for a detection target bacterium was subtracted by the background value (3 times the median and standard deviation of fluorescence intensities of spots without a probe), thereby calculating the signal intensity derived from hybridization. Next, the signal intensity of an absolute load index probe was compared with respect to a plurality of DNA chips, and the correction coefficient of each DNA chip was obtained, thereby making it possible to correct and compare the signal intensities of detection target bacteria. Subsequently, the bacterial load calculation coefficient determined in advance was multiplied, and the bacterial load of each detection target was calculated by the genome copy number. The calculation coefficient for each bacterial load was obtained as a coefficient for back-calculating each bacterial load from the signal intensity of each bacterium by measuring the signal intensity when detecting the genomic DNA from each bacterium and creating a calibration curve. Lastly, the dilution ratio of 80 detection specimens used for the PCR template was multiplied to calculate the bacterial count per paper point. By the above calculation, data on the bacterial count were obtained for each of the detection target bacteria for all 122 specimens in total. The lower detection limit at which the signal intensity was 0 or less in the initial stage was set to a copy number of 1000 uniformly. The results obtained before treatment (Table 9 (Table 9-1 and Table 9-2)) and those obtained after treatment (Table 10 (Table 10-1 and Table 10-2)) are shown.

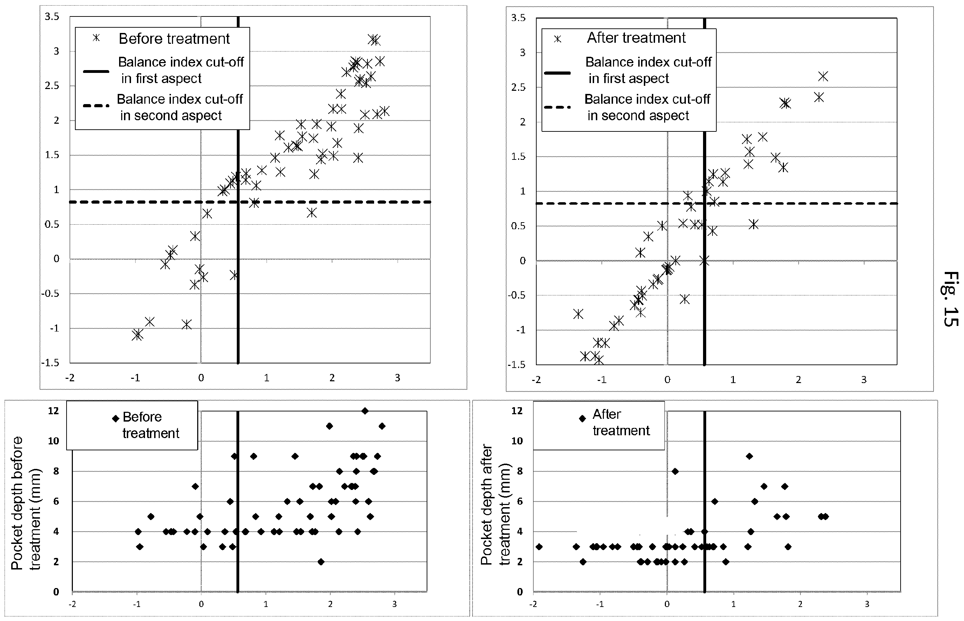

Subsequently, the same analysis as in Example 2-1 was performed using the copy number data before and after treatment. The results are shown in Figure 15. Note that the cut-off value used was the same as the analysis value for the SN ratio because a proportional relationship was observed between the SN ratio and the copy number data.

**[Table 9-1]**

| | *Porphyromonas gingivalis* | *Tannerella forsythensis* | Treponema denticola | Campylobacter rectus | *Fusobacterium nucleatum* | *Prevotella intermedia* | *Prevotella nigrescens* | *Aggregatiba cter actinomyce temcomitans* | *Capnocytophaga gingivalis* | *Streptococcus gordonii* | *Streptococcus intermedius* | *Veillonella parvula* | *Streptococcus mutans* | Total microbe |
|---|---|---|---|---|---|---|---|---|---|---|---|---|---|---|
| No. | Before treatment | Before treatment | Before treatment | Before treatment | Before treatment | Before treatment | Before treatment | Before treatment | Before treatment | Before treatment | Before treatment | Before treatment | Before treatment | Before treatment |
| 1 | 1000 | 232945 | 31886 | 52976 | 713936 | 1000 | 1000 | 1000 | 1000 | 1000 | 1000 | 1000 | 1000 | 3657180 |
| 2 | 265561 | 762466 | 24692 | 153636 | 715887 | 516627 | 1000 | 65218 | 1000 | 1000 | 1000 | 1000 | 1000 | 6431453 |
| 3 | 1000 | 240083 | 1000 | 149684 | 1497304 | 1000 | 1000 | 1000 | 1000 | 1000 | 1000 | 1000 | 1000 | 2312695 |
| 4 | 1000 | 440082 | 73401 | 131738 | 1013409 | 1000 | 1000 | 104428 | 1000 | 3940 | 1000 | 1000 | 1000 | 4305884 |
| 5 | 535727 | 73472 | 4716 | 194672 | 396431 | 1000 | 1000 | 1000 | 1000 | 1000 | 1000 | 1000 | 1000 | 1173650 |
| 6 | 1683010 | 182907 | 23328 | 194070 | 123162 | 1000 | 1000 | 1000 | 1000 | 1000 | 1000 | 1000 | 1000 | 3590443 |
| 7 | 347701 | 135772 | 81899 | 83704 | 421944 | 1000 | 1000 | 1000 | 1000 | 3947 | 1000 | 1000 | 1000 | 2294484 |
| 8 | 411329 | 175977 | 59867 | 33742 | 1424885 | 1000 | 1000 | 1000 | 1000 | 1000 | 1000 | 1000 | 1000 | 2202292 |
| 9 | 1000 | 1000 | 1000 | 3948 | 1000 | 1000 | 1000 | 1000 | 1000 | 4308 | 1000 | 1000 | 1000 | 35619 |
| 10 | 26115 | 12944 | 5377 | 15135 | 213781 | 1000 | 1000 | 1000 | 1000 | 46573 | 1000 | 1000 | 1000 | 2034312 |
| 11 | 814245 | 603351 | 208894 | 107482 | 121594 | 1000 | 1000 | 1000 | 1000 | 12675 | 1000 | 1000 | 1000 | 3944580 |
| 12 | 1000 | 1000 | 1000 | 1000 | 1000 | 1000 | 1000 | 1000 | 1000 | 44716 | 1000 | 1000 | 1000 | 213187 |
| 13 | 1086141 | 736715 | 174251 | 166797 | 705065 | 1000 | 1000 | 1000 | 1000 | 3470 | 1000 | 1000 | 1000 | 7760394 |
| 14 | 1000 | 556422 | 8209 | 245663 | 1103715 | 1000 | 1000 | 1000 | 1000 | 19608 | 28753 | 1000 | 1000 | 4741672 |
| 15 | 172662 | 558651 | 93911 | 128801 | 1634346 | 1000 | 1000 | 1000 | 1000 | 7359 | 1000 | 1000 | 1000 | 5936230 |
| 16 | 158065 | 129299 | 15937 | 13101 | 413551 | 256964 | 32970 | 1000 | 1000 | 7667 | 30877 | 1000 | 1000 | 5342257 |
| 17 | 480205 | 521133 | 10570 | 165859 | 134743 | 1000 | 1000 | 1000 | 1000 | 13231 | 1000 | 1000 | 1000 | 3642706 |
| 18 | 1000 | 1000 | 1000 | 18891 | 93640 | 1000 | 156765 | 1000 | 1000 | 28044 | 113485 | 135280 | 23921 | 2226183 |
| 19 | 1000 | 95020 | 1000 | 10119 | 599263 | 1000 | 1000 | 1000 | 1000 | 36260 | 1000 | 1000 | 1000 | 1223779 |
| 20 | 1000 | 1000 | 1000 | 1000 | 1000 | 1000 | 1000 | 1000 | 1000 | 2577 | 1000 | 1000 | 1000 | 20422 |
| 21 | 18768 | 1000 | 1000 | 1000 | 1000 | 1000 | 1000 | 1000 | 1000 | 32092 | 1000 | 1000 | 1000 | 135752 |
| 22 | 1000 | 1000 | 1000 | 1000 | 1000 | 1000 | 1000 | 1000 | 1000 | 47703 | 1000 | 1000 | 1000 | 85977 |
| 23 | 877487 | 122189 | 39817 | 21693 | 23099 | 1000 | 1000 | 1000 | 1000 | 16666 | 1000 | 1000 | 1000 | 2394924 |
| 24 | 332909 | 76062 | 4271 | 57769 | 215476 | 1000 | 1000 | 1000 | 1000 | 17243 | 1000 | 1000 | 1000 | 1393012 |
| 25 | 908848 | 295766 | 117751 | 158477 | 563999 | 371922 | 46416 | 1000 | 1000 | 12395 | 1000 | 1000 | 1000 | 6860618 |

**[Table 9-2]**

| | | | | | | | | | | | | | | |
|---|---|---|---|---|---|---|---|---|---|---|---|---|---|---|
| 26 | 1000 | 1000 | 1000 | 1000 | 14393 | 1000 | 20999 | 1000 | 1000 | 47466 | 1000 | 1000 | 1000 | 651213 |
| 27 | 2413682 | 475117 | 214242 | 236617 | 259389 | 167965 | 1000 | 1000 | 1000 | 58859 | 1000 | 1000 | 1000 | 7752034 |
| 28 | 201332 | 491201 | 136736 | 67776 | 538182 | 1000 | 1000 | 1000 | 1000 | 15457 | 18907 | 1000 | 1000 | 3793676 |
| 29 | 77522 | 79645 | 18838 | 14942 | 192067 | 1000 | 1000 | 1000 | 1000 | 37171 | 1000 | 1000 | 1000 | 660922 |
| 30 | 1000 | 27871 | 1000 | 1000 | 29023 | 1000 | 1000 | 1000 | 1000 | 1000 | 1000 | 1000 | 1000 | 94698 |
| 31 | 1000 | 1000 | 1000 | 1000 | 1000 | 1000 | 1000 | 1000 | 1000 | 29161 | 1000 | 1000 | 1000 | 589933 |
| 32 | 64663 | 1000 | 4659 | 14603 | 121718 | 1000 | 1000 | 1000 | 1000 | 2713 | 222560 | 1000 | 1000 | 385537 |
| 33 | 1000 | 1000 | 1000 | 6333 | 423142 | 1000 | 154884 | 1000 | 1000 | 55544 | 79449 | 1000 | 13693 | 4799761 |
| 34 | 28286 | 1000 | 2963 | 1000 | 19754 | 1000 | 1000 | 1000 | 1000 | 3858 | 1000 | 1000 | 1000 | 55244 |
| 35 | 848493 | 95973 | 123025 | 86279 | 347254 | 1000 | 1000 | 1000 | 1000 | 1000 | 1000 | 1000 | 1000 | 2818813 |
| 36 | 1000 | 128076 | 13848 | 37698 | 55288 | 1000 | 1000 | 1000 | 1000 | 1000 | 1000 | 1000 | 1000 | 207457 |
| 37 | 1000 | 744982 | 31716 | 128704 | 658298 | 1000 | 1000 | 1000 | 1000 | 1000 | 1000 | 1000 | 1000 | 2110398 |
| 38 | 19605 | 27673 | 3078 | 4139 | 18147 | 1000 | 1000 | 1000 | 1000 | 1000 | 1000 | 1000 | 1000 | 92010 |
| 39 | 40717 | 93993 | 1000 | 118538 | 499201 | 1000 | 1000 | 1000 | 1000 | 1000 | 1000 | 1000 | 1000 | 1091861 |
| 40 | 1043643 | 140827 | 84586 | 110624 | 510505 | 843223 | 1000 | 1000 | 1000 | 5435 | 17387 | 1000 | 1000 | 13866983 |
| 41 | 705228 | 530977 | 17342 | 62798 | 121554 | 1000 | 1000 | 102751 | 1000 | 1000 | 1000 | 1000 | 1000 | 3340179 |
| 42 | 1000 | 1000 | 1000 | 3408 | 1000 | 1000 | 1000 | 1000 | 1000 | 6312 | 1000 | 1000 | 1000 | 181657 |
| 43 | 1870070 | 365237 | 382942 | 169113 | 274399 | 1000 | 66956 | 1000 | 1000 | 37058 | 1000 | 1000 | 1000 | 15101041 |
| 44 | 1030442 | 199653 | 243434 | 11596 | 107094 | 1000 | 1000 | 1000 | 1000 | 2550 | 1000 | 1000 | 1000 | 4285411 |
| 45 | 1000 | 1000 | 1000 | 1000 | 481252 | 1000 | 1000 | 1000 | 1000 | 198848 | 1000 | 1000 | 7210 | 4648619 |
| 46 | 1000 | 1000 | 1000 | 2804 | 333650 | 1000 | 1000 | 1000 | 1000 | 42327 | 54288 | 1000 | 1000 | 3221188 |
| 47 | 1000 | 1000 | 1000 | 3444 | 14096 | 1000 | 1000 | 1000 | 1000 | 20854 | 44150 | 1000 | 1000 | 403610 |
| 48 | 63717 | 493481 | 11105 | 11110 | 165441 | 1000 | 1000 | 1000 | 1000 | 98633 | 1000 | 1000 | 1000 | 4723716 |
| 49 | 220660 | 317469 | 1000 | 69997 | 308324 | 1000 | 1000 | 1000 | 1000 | 10917 | 1000 | 1000 | 1000 | 1997360 |
| 50 | 20361 | 1000 | 1000 | 1000 | 1000 | 1000 | 1000 | 1000 | 1000 | 3841 | 1000 | 1000 | 1000 | 4927718 |
| 51 | 14677 | 1000 | 1000 | 1000 | 403135 | 1000 | 1000 | 1000 | 1000 | 99447 | 59079 | 1000 | 1000 | 1813968 |
| 52 | 1729883 | 664471 | 271488 | 69037 | 137148 | 1000 | 1000 | 1000 | 1000 | 1000 | 1000 | 1000 | 1000 | 1753017 |
| 53 | 16379 | 1000 | 2772 | 6197 | 113874 | 1000 | 1000 | 1000 | 1000 | 63657 | 1000 | 34452 | 24728 | 809996 |
| 54 | 598296 | 550571 | 90922 | 174812 | 132956 | 1000 | 1000 | 1000 | 1000 | 8277 | 1000 | 1000 | 1000 | 2166159 |
| 55 | 516451 | 1487221 | 2544 | 153695 | 980677 | 1000 | 1000 | 1000 | 1000 | 1000 | 23674 | 1000 | 1000 | 3675082 |
| 56 | 577703 | 545657 | 1000 | 103647 | 558904 | 1000 | 54027 | 1000 | 1000 | 3178 | 1000 | 1000 | 1000 | 4260745 |
| 57 | 1000 | 1000 | 1000 | 1000 | 1285818 | 1000 | 1000 | 1000 | 1000 | 185722 | 144299 | 1000 | 1000 | 2324620 |
| 58 | 92300 | 138249 | 19301 | 172096 | 684513 | 1000 | 1000 | 1000 | 1000 | 1000 | 1000 | 1000 | 1000 | 1536405 |
| 59 | 1000 | 1000 | 3812 | 1000 | 13886 | 1000 | 1000 | 1000 | 1000 | 1000 | 1000 | 1000 | 1000 | 51277 |
| 60 | 1000 | 181385 | 159871 | 116302 | 235100 | 1000 | 1000 | 1000 | 1000 | 18605 | 1000 | 1000 | 1000 | 1772727 |
| 61 | 1907552 | 471137 | 1000 | 151262 | 73808 | 248576 | 1000 | 1000 | 1000 | 7142 | 1000 | 1000 | 1000 | 6011816 |

**[Table 10-1]**

| | *Porphyromonas gingivalis* | *Tannerella forsythensis* | Treponema denticola | Campylobacter rectus | *Fusobacterium nucleatum* | *Prevotella intermedia* | *Prevotella nigrescens* | *Aggregatibacteractinomyc etemcomitans* | *Capnocytophaga gingivalis* | *Streptococcus gordonii* | *Streptococcus intermedius* | *Veillonella parvula* | *Streptococcus mutans* | Total microbe |
|---|---|---|---|---|---|---|---|---|---|---|---|---|---|---|
| No. | After treatment | After treatment | After treatment | After treatment | After treatment | After treatment | After treatment | After treatment | After treatment | After treatment | After treatment | After treatment | After treatment | After treatment |
| 1 | 1000 | 1000 | 1000 | 1000 | 1000 | 1000 | 1000 | 1000 | 1000 | 1000 | 1000 | 1000 | 1000 | 10473 |
| 2 | 1000 | 1000 | 1000 | 1000 | 1000 | 1000 | 1000 | 1000 | 1000 | 1000 | 1000 | 1000 | 1000 | 47085 |
| 3 | 1000 | 1000 | 27751 | 13967 | 146145 | 1000 | 1000 | 1000 | 1000 | 7340 | 1000 | 1000 | 1000 | 735385 |
| 4 | 1000 | 1000 | 1000 | 1000 | 1000 | 1000 | 1000 | 1000 | 1000 | 2509 | 1000 | 1000 | 1000 | 17229 |
| 5 | 1000 | 1000 | 1000 | 1000 | 1000 | 1000 | 1000 | 1000 | 1000 | 1000 | 1000 | 1000 | 5992 | 4472 |
| 6 | 371272 | 70312 | 16602 | 68576 | 228552 | 173262 | 1000 | 1000 | 1000 | 1000 | 1000 | 1000 | 1000 | 855260 |
| 7 | 1000 | 1000 | 1000 | 1000 | 1000 | 1000 | 1000 | 1000 | 1000 | 1000 | 1000 | 1000 | 1000 | 2364 |
| 8 | 1000 | 1000 | 1000 | 1000 | 1000 | 1000 | 1000 | 1000 | 1000 | 2383 | 1000 | 1000 | 1000 | 13351 |
| 9 | 1000 | 1000 | 1000 | 5996 | 1000 | 1000 | 1000 | 1000 | 1000 | 104958 | 1000 | 1000 | 1000 | 601180 |
| 10 | 24536 | 1000 | 1000 | 1000 | 1000 | 1000 | 1000 | 1000 | 1000 | 11235 | 1000 | 1000 | 1000 | 177897 |
| 11 | 1000 | 1000 | 1000 | 1000 | 1000 | 1000 | 1000 | 1000 | 1000 | 1000 | 1000 | 1000 | 1000 | 2117 |
| 12 | 1000 | 1000 | 1000 | 1245 | 1000 | 1000 | 1000 | 1000 | 1000 | 14463 | 1000 | 1000 | 1000 | 11327 |
| 13 | 1000 | 1000 | 1000 | 76471 | 64102 | 1000 | 1000 | 1000 | 1000 | 54619 | 17283 | 1000 | 1000 | 320604 |
| 14 | 22396 | 1000 | 1000 | 93363 | 91441 | 1000 | 1000 | 1000 | 4960 | 19579 | 1000 | 1000 | 1000 | 4280125 |
| 15 | 1000 | 1000 | 1000 | 1000 | 1000 | 1000 | 1000 | 1000 | 1000 | 11568 | 1000 | 1000 | 1000 | 58695 |
| 16 | 1000 | 1000 | 1000 | 1000 | 1000 | 1000 | 1000 | 1000 | 1000 | 1000 | 1000 | 1000 | 1000 | 25401 |
| 17 | 107854 | 1000 | 1000 | 8098 | 30228 | 1000 | 1000 | 1000 | 1000 | 119895 | 17648 | 190441 | 1000 | 537467 |
| 18 | 1000 | 1000 | 1000 | 1000 | 1000 | 1000 | 1000 | 1000 | 1000 | 19135 | 74222 | 1000 | 8165 | 249072 |
| 19 | 1000 | 1000 | 1000 | 1000 | 1000 | 1000 | 1000 | 1000 | 1000 | 4540 | 1000 | 1000 | 1000 | 45003 |
| 20 | 1000 | 1000 | 1000 | 1000 | 1000 | 1000 | 1000 | 1000 | 1000 | 1000 | 1000 | 1000 | 1000 | 11424 |
| 21 | 1000 | 1000 | 1000 | 1000 | 1000 | 1000 | 1000 | 1000 | 1000 | 26142 | 1000 | 1000 | 1000 | 157206 |
| 22 | 1000 | 1000 | 1000 | 1000 | 1000 | 1000 | 1000 | 1000 | 1000 | 1915 | 1000 | 1000 | 1000 | 35438 |
| 23 | 1000 | 1000 | 1000 | 2782 | 1000 | 1000 | 1000 | 1000 | 1000 | 58538 | 1000 | 1000 | 1000 | 362325 |
| 24 | 1000 | 1000 | 1000 | 1000 | 1000 | 1000 | 1000 | 1000 | 1000 | 1000 | 1000 | 1000 | 1000 | 22248 |
| 25 | 79236 | 95041 | 1000 | 28738 | 328103 | 1000 | 1000 | 1000 | 1000 | 4218 | 1000 | 1000 | 1000 | 280607 |

**[Table 10-2]**

| | | | | | | | | | | | | | | |
|---|---|---|---|---|---|---|---|---|---|---|---|---|---|---|
| 26 | 1000 | 1000 | 1000 | 1000 | 1000 | 1000 | 1000 | 1000 | 1000 | 31682 | 1000 | 1000 | 1000 | 105169 |
| 27 | 1000 | 59308 | 10631 | 109723 | 25213 | 1000 | 28226 | 1000 | 1000 | 18339 | 17142 | 1000 | 18385 | 1125398 |
| 28 | 1000 | 1000 | 1000 | 1000 | 1000 | 1000 | 1000 | 1000 | 1000 | 2505 | 1000 | 1000 | 1000 | 21199 |
| 29 | 1000 | 1000 | 1000 | 1000 | 1000 | 1000 | 1000 | 1000 | 1000 | 431408 | 1000 | 1000 | 1000 | 1035932 |
| 30 | 1000 | 1000 | 1000 | 1000 | 17672 | 1000 | 1000 | 1000 | 1000 | 1000 | 1000 | 1000 | 1000 | 87052 |
| 31 | 5340 | 1000 | 1000 | 12050 | 28205 | 1000 | 1000 | 1000 | 1000 | 15779 | 1000 | 1000 | 1000 | 701560 |
| 32 | 2899238 | 448973 | 93930 | 411140 | 438729 | 942444 | 1000 | 1000 | 1000 | 15387 | 61650 | 1000 | 1000 | 4164905 |
| 33 | 1000 | 1000 | 1000 | 1000 | 32428 | 1000 | 124232 | 1000 | 1000 | 9624 | 282120 | 466143 | 35070 | 1150079 |
| 34 | 11560 | 1000 | 1000 | 1000 | 1000 | 1000 | 1000 | 1000 | 1000 | 1000 | 1000 | 1000 | 1000 | 18757 |
| 35 | 27589 | 14799 | 2814 | 6780 | 24690 | 1000 | 1000 | 1000 | 1000 | 1000 | 1000 | 1000 | 1000 | 43648 |
| 36 | 1000 | 11505 | 4069 | 28368 | 117242 | 1000 | 26563 | 1000 | 1000 | 6633 | 24910 | 1000 | 1000 | 511336 |
| 37 | 1000 | 1000 | 1000 | 1000 | 70541 | 1000 | 1000 | 1000 | 1000 | 29514 | 1000 | 1000 | 1000 | 148269 |
| 38 | 1000 | 1000 | 1000 | 1000 | 1000 | 1000 | 1000 | 1000 | 1000 | 22286 | 36667 | 1000 | 1000 | 51206 |
| 39 | 66951 | 77321 | 1000 | 1000 | 123882 | 1000 | 1000 | 1000 | 1000 | 10225 | 1000 | 1000 | 1000 | 259261 |
| 40 | 1000 | 1000 | 1000 | 1000 | 1000 | 1000 | 1000 | 1000 | 1000 | 1000 | 1000 | 1000 | 1000 | 10311 |
| 41 | 1000 | 1000 | 1000 | 38227 | 49660 | 1000 | 1000 | 1000 | 4183 | 4486 | 1000 | 1000 | 1000 | 1150004 |
| 42 | 1000 | 1000 | 1000 | 1000 | 1000 | 1000 | 1000 | 1000 | 1000 | 1000 | 1000 | 1000 | 1000 | 12733 |
| 43 | 1000 | 1000 | 1000 | 66477 | 88689 | 1000 | 140849 | 1000 | 1000 | 14492 | 18757 | 1000 | 1000 | 2790129 |
| 44 | 1000 | 32824 | 51685 | 58759 | 62857 | 1000 | 277147 | 1000 | 1000 | 32260 | 1000 | 1000 | 1000 | 2531732 |
| 45 | 1000 | 1000 | 1000 | 1000 | 15171 | 1000 | 1000 | 1000 | 1000 | 43392 | 1000 | 1000 | 3422 | 1197455 |
| 46 | 1000 | 1000 | 1000 | 1000 | 28923 | 1000 | 1000 | 1000 | 1000 | 33251 | 1000 | 1000 | 1000 | 1689997 |
| 47 | 3385 | 1000 | 1000 | 1000 | 1000 | 1000 | 13373 | 10388 | 1000 | 30877 | 1000 | 61283 | 1000 | 262531 |
| 48 | 1000 | 1000 | 1000 | 4837 | 1000 | 1000 | 1000 | 1000 | 1000 | 19298 | 1000 | 1000 | 1000 | 500480 |
| 49 | 1000 | 1000 | 1000 | 1000 | 1000 | 1000 | 1000 | 1000 | 1000 | 5751 | 1000 | 1000 | 1000 | 71083 |
| 50 | 1000 | 1000 | 1000 | 1000 | 1000 | 1000 | 1000 | 1000 | 1000 | 11751 | 1000 | 1000 | 1000 | 51754 |
| 51 | 1000 | 1000 | 1000 | 1000 | 1000 | 1000 | 1000 | 1000 | 1000 | 9783 | 1000 | 1000 | 1000 | 16256 |
| 52 | 1000 | 1000 | 1000 | 1000 | 1000 | 1000 | 1000 | 1000 | 1000 | 2209 | 1000 | 1000 | 1000 | 16330 |
| 53 | 1000 | 1000 | 1000 | 1000 | 1000 | 1000 | 1000 | 1000 | 1000 | 4279 | 1000 | 1000 | 21553 | 15416 |
| 54 | 49823 | 1000 | 1000 | 1000 | 15099 | 1000 | 1000 | 1000 | 1000 | 15280 | 1000 | 1000 | 5703 | 110689 |
| 55 | 96639 | 1000 | 1000 | 6822 | 41700 | 1000 | 1000 | 1000 | 1000 | 15893 | 32340 | 1000 | 1000 | 962305 |
| 56 | 101589 | 125241 | 1000 | 3181 | 519340 | 1000 | 42508 | 1000 | 1000 | 7938 | 1000 | 1000 | 1000 | 3786745 |
| 57 | 1000 | 1000 | 1000 | 1000 | 136867 | 1000 | 46203 | 1000 | 1000 | 122923 | 119781 | 1000 | 1000 | 973738 |
| 58 | 2791249 | 641285 | 10405 | 178634 | 692509 | 224510 | 1000 | 1000 | 1000 | 1000 | 86844 | 1000 | 1000 | 3461175 |
| 59 | 1000 | 213966 | 493148 | 58610 | 570862 | 1000 | 1000 | 1000 | 1000 | 1991 | 1000 | 1000 | 1000 | 2479648 |
| 60 | 1000 | 1000 | 132690 | 5389 | 128591 | 1000 | 1000 | 1000 | 1000 | 5381 | 1000 | 1000 | 1000 | 601150 |
| 61 | 2906079 | 1000 | 20562 | 55013 | 166556 | 1490523 | 1000 | 1000 | 1000 | 24519 | 172078 | 1000 | 1000 | 5623379 |

Determination was performed on two axes in the same manner as in Example 2-1. The results are shown in the two graphs in the upper half of Figure 15. The left side shows the results before treatment and the right side shows the results after treatment. When the positions of plots were observed before and after treatment, the plots entirely moved from the upper right ((a) at the level requiring retreatment) to the lower left ((d) mild), which allowed determination of effects of the treatment. Further, the two graphs in the lower half of Figure 15 are graphs of the periodontal pocket (vertical axis ) and the balance index (horisontal axis). The left side is before treatment and the right side is after treatment. From these results, it was possible to determine that the disease state was stable in each plot in which the balance index did not exceed the determination value even with a periodontal pocket depth of about 4 mm after the treatment. On the other hand, it was possible to determine that each plot in which the balance index exceeded the determination value even with a periodontal pocket depth of 3 mm should be considered for treatment.

### [Example 3]

### Determination based on Next-Generation Sequencer Data

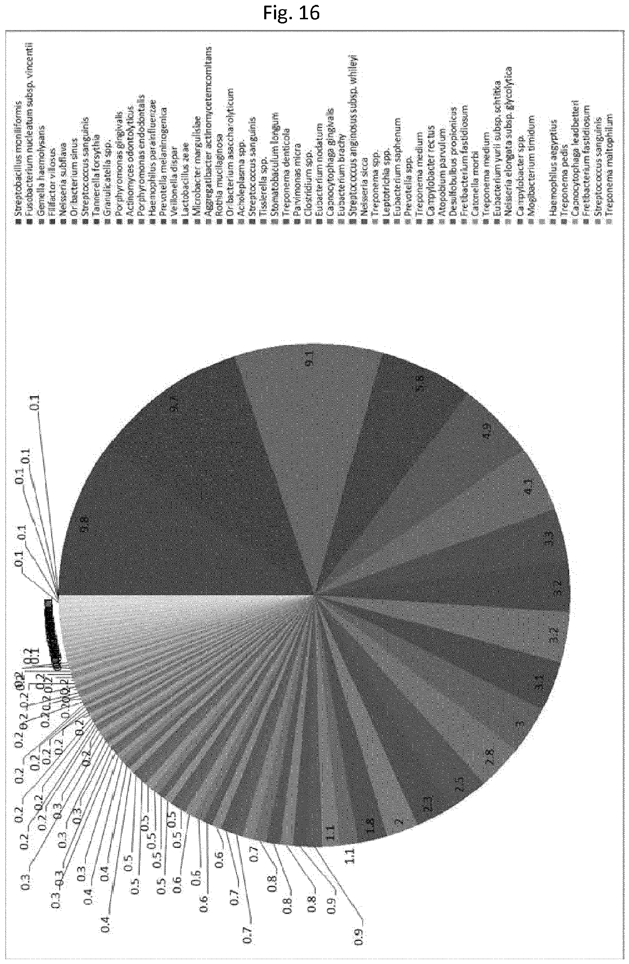

One of the samples in Example 1-1 was sent to the J-Bio21 Center (NIPPON STEEL Eco-Tech Corporation: Tsukuba Kouken Building 2F, 2-1-13 Umezono, Tsukuba City, Ibaraki Prefecture) for 16S rRNA next-generation sequencer analysis. From the obtained results, the relative ratio of each bacterium to the total bacterial count was calculated. The results are shown in Figure 16.

Next, the relative amount between the "bacterial species that increases as the periodontal pocket value increases" and the "bacterial species that decreases as the periodontal pocket value increases" shown in Example 1-4 was examined and found as shown in Table 11. Further, the balance index was calculated in the same manner as in Examples 1-4 and 2-2. The balance index was 3.739 (17.2%/4.6%) and it was about 0.5728 when converted by LOG10. Thus, it was possible to calculate the determination value. By determining the value on the X-axis in Figure 15, it was possible to determine "mild" as the balance index.

**[Table 11]**

| | Next-Generation Sequencer Results |
|---|---|
| Bacterial Species Name | Relative amount (%) |
| Porphyromonas gingivalis | 3.1 |
| Tannerella forsythensis | 3.2 |
| Treponema denticola | 0.7 |
| Campylobacter rectus | 0.5 |
| Fusobacterium nucleatum | 9.7 |
| Prevotella intermedia | N.D. |
| Capnocytophaga gingivalis | 0.6 |
| Streptococcus gordonii | 4 |
| Streptococcus intermedius | N.D. |
| Veillonella parvula | N.D. |
| Total bacteria | 100 |

### [Example 4]

In order to investigate bacterial species newly discussed in recent years, a DNA chip which is newly equipped with the bacterial probes shown in Table 12 was prepared in the same manner as in Example 1-1.

**[Table 12]**

| SEQ ID NO | Name | Probe Sequence |
|---|---|---|
| 35 | Absolute load index probe | CTATTCGACCAGCGATATCACTACGTAGGC |
| 34 | Total load index probe | CGTATTACCGCGGCTGCTGGCAC |
| 54 | Eubacterium nodatum probe | CCTACGCTTACTTAACCACCTA |
| 55 | Parvimonas micra probe | GTGCTTAATGAGGTTAAGCC |
| 56 | Filifactor alocis probe | CCCCTACTACAGAGTTTTACGA |
| 57 | Streptococcus sobrinus probe | TACACACGTTCTTCCCCTAC |
| 58 | Porphyromonas pasteri probe | ACACGTGACTCTTGTTATTC |
| 59 | Veillonella atypica probe | CGTCAAATCCTCGCACTATTC |
| 60 | Haemophilus parainfluenzae probe | AGTTAACGTCAATCACCTAG |
| 61 | Alloprevotella spp. (A. rava, OT 308) probe | TTCCCAACTAAAAGCAGTTTA |
| 62 | Streptococcus parasanguinis probe | CTGGTAAGTTACCGTCAC |
| 63 | Actinomyces israelii probe | GCGCTTCATAACCCGGCTAC |
| 64 | Prevotella pallens probe | CACGTGCATCAAATTATTCTCG |
| 65 | Prevotella loescheii probe | CCTACTTTCAGCGCACTCAA |
| 66 | Prevotella histicola probe | CACGTGACTGACTTTATCCC |
| 67 | Solobacterium moorei probe | CCAACAATTTAACCACTTAC |
| 68 | Prevotella melaninogenica probe | AATAGGGACACGTCCCTAAC |
| 69 | Selenomonas sputigena probe | GTACCGTCACCCAAACTCAATA |
| 70 | Rothia dentocariosa probe | ACCCACTGCAAAACCAGGGT |
| 71 | Rothia mucilaginosa probe | TCTCTTCTTCCCTGCTAACA |
| 72 | Veillonella rogosae probe | ACCGTCAATTCCTCTAACTATT |
| 73 | Peptostreptococcus stomatis probe | ACCACCGACTTGAAGGACCA |
| 74 | Prevotella denticola probe | AGTCAGACGTTGGGCGCCTA |
| 75 | Porphyromonas endodontalis probe | TACATGCATCTCAGCTACACGT |
| 76 | Streptococcus salivarius probe | CACACTCGTTCTTGACTTAC |
| 77 | Actinomyces graevenitzii probe | AAAAAGCAGTGCCTTGTTCC |
| 78 | Treponema medium probe | GTCGATTACCGTCATCAGATG |
| 79 | Treponema socranskii probe | TTCCTCCAAAACTTATTCCT |
| 80 | Gemella sanguinis probe | CCGTCTCTACTGTATATAGT |
| 81 | Porphyromonas catoniae probe | GGTACATTCACTATGGTACACG |
| 82 | Corynebacterium matruchotii probe | TCTTAACAAAGGTACCGTCACC |
| 83 | Eubacterium saphenum probe | CCCTAGGACAGAGGCTTACA |
| 84 | Neisseria flavescens probe | AGCTGTCGATATTAGCAACAG |
| 85 | Granulicatella adiacens probe | GTCAAGGCGCTAACAGTTAC |
| 86 | Eubacterium sulci probe | AAACCCTGCGCTTAAGGTGC |
| 87 | Megasphaera micronuciformis probe | TAACCACAAGATTATTCGTC |
| 88 | Prevotella shahii probe | ACGTGGGCTCTTTTATCCCC |
| 89 | SR1 sp. OT 345 probe | CGTCATTCGTCTTCTGCCAA |

Fluorescence intensity data were newly collected with a DNA chip shown in Table 12 for the 321 samples collected in the same manner as in Example 1-1. The experimental conditions were the same as in Example 1-1, but the following two points were changed.

The primers used for PCR were changed as follows.

R and Y represent mixed bases, R represents A and G, and Y represents C and T.
Forward primer (for bacterial amplification):
   5'-Cy5-TACGGGAGGCAGCAG-3' (SEQ ID NO: 90)
Reverse primer (for bacterial amplification):
   5'-CRGGGTATCTAATCCYGTT-3' (SEQ ID NO: 91)
Forward primer (for absolute load index amplification):
   5'-Cy5-GAGAAGCCTACACAAACGTAACGTC-3' (SEQ ID NO: 39)
Reverse primer (for absolute load index amplification):
   5'-CTCTAAAGACCGCTCTATCTCGG-3' (SEQ ID NO: 40)

The hybridization temperature and time were set to 50°C for 16 hours. Subsequently, the obtained fluorescence intensity was processed as follows. The fluorescence intensity of a spot with a probe mounted thereon for a detection target bacterium was subtracted by the background value (the median of the fluorescence intensities of spots without a probe), thereby calculating the signal intensity derived from hybridization. At this time, when the signal intensity was below a certain threshold, it was determined to be noise and was set to "0." Here, as the threshold value, a value three times the standard deviation of 20 values excluding the upper and lower 5 values out of the fluorescence intensities of 30 spots without a probe was used.

Further, the relative ratio of each bacterium to the total bacteria was calculated by dividing the signal intensity of the probe for a detection target bacterium by the signal intensity of the probe for the total microbial load index. For the subsequent analysis, the value obtained by converting the relative ratio to the total bacterial load by log10 was used. However, since the value "0" cannot be calculated, the value after log10 conversion was replaced with -4. Thus, data were obtained for all 321 specimens. Table 13 summarizes the results and periodontal pocket depth for each specimen (Table 13-1 to Table 13-16).

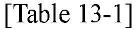

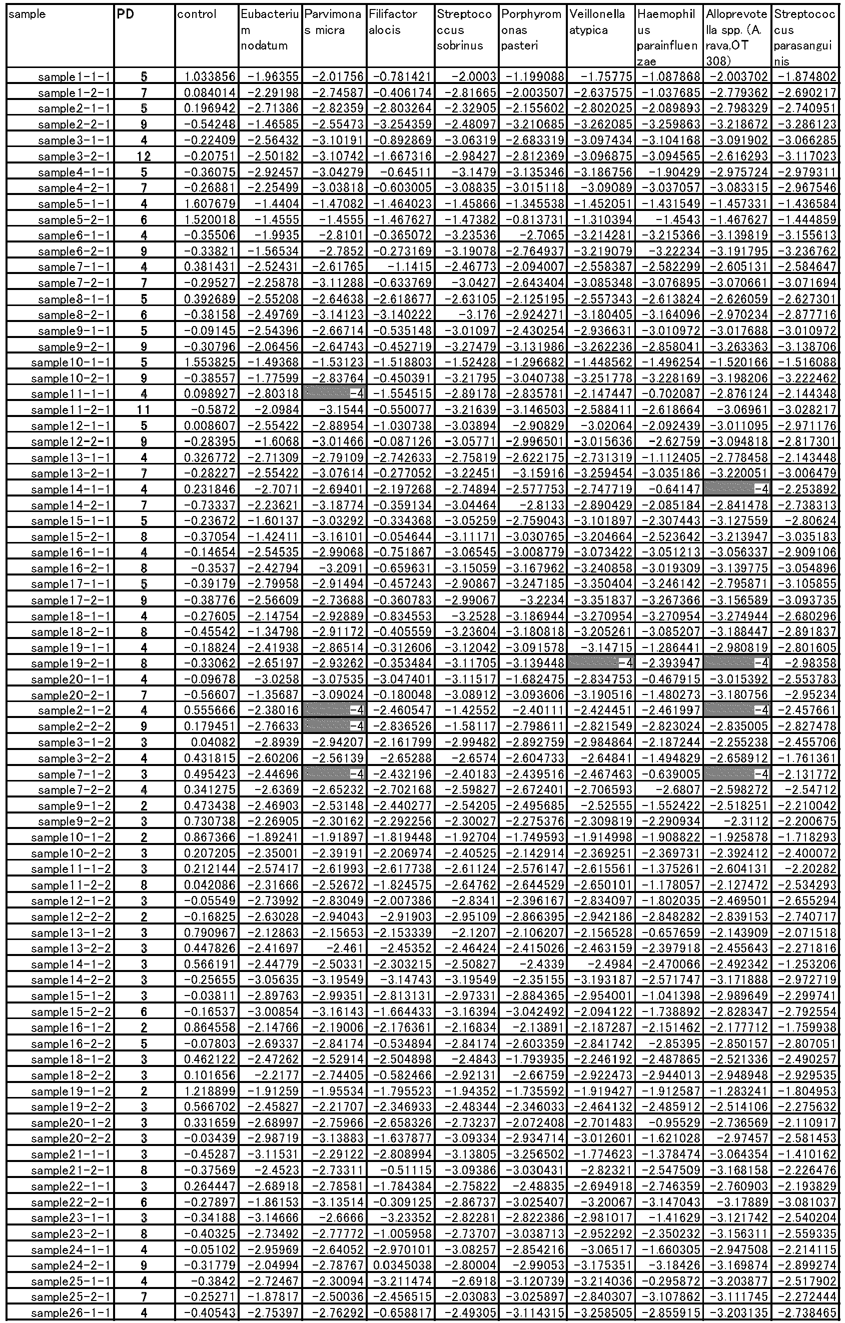

**[Table 13-2]**

| | | | | | | | | | | | |
|---|---|---|---|---|---|---|---|---|---|---|---|
| sample26-2-1 | 8 | -0.40818 | -2.35494 | -3.09677 | -0.129634 | -26225 | -2.978061 | -2.582334 | -3.143571 | -3.201427 | -2.777363 |
| sample27-1-1 | 4 | -0.21587 | -2.7411 | -2.42253 | -0.849843 | -3.15161 | -2.589554 | -3.191892 | -3.217042 | -3.151611 | -3.118488 |
| sample27-2-1 | 11 | -0.45698 | -1.67575 | -3.17285 | -0.360234 | -2.93578 | -3.140861 | -3.268377 | -3.239488 | -3.26723 | -3.220557 |
| sample28-1-1 | 4 | -0.30388 | -2.39999 | -2.62774 | -0.090914 | -2.87244 | -2.780667 | -3.176965 | -3.097004 | -3.168847 | -2.810328 |
| sample28-2-1 | 6 | -0.59781 | -1.9953 | -2.83217 | -0.044617 | -2.97035 | -2.926021 | -3.186831 | -3.126179 | -3.18897 | -2.944556 |
| sample29-1-1 | 3 | 0.076767 | -2.89546 | -3.00869 | -2822453 | -2.98912 | -2.923647 | -2.978934 | -1.805054 | -2.995048 | -2.399378 |
| sample29-2-1 | 6 | -0.52944 | -1.60903 | -2.82277 | -0.12704 | -3.08313 | -3.139961 | -3.231247 | -3.092253 | -3.201792 | -2.989299 |
| sample30-1-1 | 4 | -0.09103 | -2.33814 | -2.91424 | -0.257269 | -2.87179 | -2.685848 | -2.998491 | -1.295561 | -2.902759 | -2.537353 |
| sample30-2-1 | 6 | -0.17286 | -2.41529 | -2.95735 | -0.182348 | -3.18714 | -2.962547 | -3.211596 | -3.160443 | -3.168761 | -3.073293 |
| sample31-1-1 | 4 | -0.30666 | -2.89964 | -3.29953 | -1.582509 | -3.27642 | -2.612166 | -3.285787 | -3.141014 | -3.256998 | -3.023654 |
| sample31-2-1 | 6 | -0.28346 | -2.46208 | -3.33059 | -1.092486 | -3.31394 | -3.145629 | -3.322183 | -2.907637 | -3.295283 | -3.204943 |
| sample32-1-1 | 4 | -0.07219 | -2.13469 | -3.08181 | -1.986384 | -268652 | -2.975468 | -3.110544 | -3.061124 | -2.969293 | -2.829784 |
| sample32-2-1 | 9 | -0.4482 | -1.75812 | -3.19249 | -0.404194 | -3.04844 | -3.193606 | -3.273235 | -3.17821 1 | -3.244618 | -2.736333 |
| sampleHC01-1-1 | 3 | -0.39165 | -3.06456 | -2.93584 | -1.89922 | -3.25856 | -2.85678 | -3252668 | -2.730229 | -2.98035 | -3.011934 |
| sampleHC01-2-1 | 3 | -0.25694 | -3.09815 | -3.25589 | -3.137362 | -3.24994 | -2.942312 | -2.928706 | -2665208 | -0.890581 | -2.700002 |
| sampleHC01-3-1 | ? | -0.35202 | -3.14637 | -3.10122 | -3.224945 | -3.32246 | -2.258738 | -3.07382 | -3.14536 | -3.16078 | -2.712875 |
| sampleHC02-1-1 | 3 | -0.1337 | -2.98543 | -2.99175 | -1.581463 | -3.13498 | -2.599127 | -3.120043 | -0.882634 | -2.96698 | -2.697606 |
| sampleHC02-2-1 | 2 | 0.000234 | -2.95389 | -3.09451 | -1.746037 | -3.05897 | -2.443578 | -3.033815 | -1.625688 | -1.812078 | -2.43538 |
| sampleHC02-3-1 | 3 | -0.03205 | -285892 | -3.02217 | -3.112416 | -3.13343 | -2.446842 | -3.086959 | -2.432078 | -3.125139 | -2.661932 |
| sampleHC03-1-1 | 3 | 0.059711 | -2.89572 | -2.99901 | -2.969229 | -2.98973 | -2.837461 | -2.961784 | -1.283191 | -2.992363 | -2.221981 |
| sampleHC03-2-1 | 2 | 0.161561 | -2.82871 | -2.91233 | -2.856007 | -2.86073 | -2.639591 | -2.838748 | -0.191845 | -2.900462 | -2.164615 |
| sampleHC03-3-1 | 3 | -0.0527 | -2.90999 | -3.02749 | -2.995005 | -3.00158 | -2.88804 | -2.959942 | -0.248408 | -2902552 | -2.309546 |
| sampleHC04-1-1 | 3 | -0.18598 | -3.01978 | -2.85154 | -1.271193 | -3.13408 | -2.023566 | -2.379727 | -1.086673 | -3.170601 | -2.520219 |
| sampleHC04-2-1 | 2 | -0.19987 | -3.05369 | -2.879 | -3.130365 | -3.16554 | -3.097823 | -1.79051 | -3.16015 | -3.07963 | -2.608489 |
| sampleHC04-3-1 | 3 | -0.15706 | -3.06609 | -2.65237 | -3.165724 | -3.19686 | -3.111848 | -3.157606 | -1.991 | -3.188144 | -2.599223 |
| sampleHC05-1-1 | 3 | -0.32212 | -3.1035 | -2.56316 | -3.282741 | -3.30637 | -3.156789 | -3.2655 | -0.92486 | -3.125545 | -2.787945 |
| sampleHC05-2-1 | 2 | 0.131627 | -2.86927 | -3.03733 | -2.983534 | -3.03258 | -2.993486 | -2.960275 | -0.532409 | -1.972023 | -2.188837 |
| sampleHC05-3-1 | 3 | -0.02384 | -2.99827 | -3.15317 | -3.099075 | -3.13156 | -2.848464 | -3.070069 | -0.338324 | -3.012993 | -2.237883 |
| sampleHC06-1-1 | 2 | -0.24296 | -3.15647 | -3.33561 | -3.285134 | -3.33104 | -2.799475 | -3.296164 | -1.557888 | -2.61925 | -2.85241 1 |
| sampleHC06-2-1 | 2 | 0.297624 | -2.79697 | -2.87403 | -2.790362 | -2.87722 | -2.555077 | -2.840368 | -0.970202 | -1.475642 | -2.359825 |
| sampleHC06-3-1 | 2 | -0.24643 | -3.16061 | -3.32585 | -2.764656 | -3.31788 | -2.253555 | -3.261666 | -0.561739 | -2.394539 | -2.661692 |
| sampleHC07-1-1 | 3 | -0.06838 | -3.06021 | -3.17838 | -3.143499 | -3.20778 | -3.159833 | -3.150848 | -1.537197 | -3.191204 | -2.484176 |
| sampleHC07-2-1 | 3 | 0.430999 | -2.66884 | -2.75155 | -2.715157 | -2.73516 | -2.192977 | -2.583635 | -2.331699 | -2.745519 | -2.290937 |
| sampleHC07-3-1 | 3 | 0.20382 | -292227 | -3.04972 | -2.995961 | -3.0447 | -2.975623 | -3.001951 | -1.106717 | -3.025181 | -2.593876 |
| sampleHC08-1-1 | 3 | -0.21489 | -3.15059 | -3.14732 | -3255528 | -3.31372 | -3.106814 | -3.265414 | -0.94325 | -2.853418 | -2.766017 |
| sampleHC08-2-1 | ? | 0.284622 | -2.85051 | -2.94204 | -2.776089 | -2.94037 | -2.83457 | -2.900648 | -0.611744 | -1.603278 | -2.363137 |
| sampleHC08-3-1 | 3 | -0.01735 | -3.06264 | -2.7034 | -3.179499 | -3.23294 | -3.14019 | -3.193435 | -2.641349 | -3.044395 | -3.1388 |
| sampleHC09-1-1 | 3 | -0.00889 | -3.00251 | -2.99551 | -3.057653 | -3.09242 | -2.694475 | -3.073992 | -1.065528 | -2.150904 | -2286462 |
| sampleHC09-2-1 | 3 | 0.142051 | -2.87463 | -2.98637 | -2.874625 | -2.9667 | -2.807494 | -2.935777 | -2.111645 | -2.574894 | -2.322302 |
| sampleHC09-3-1 | 3 | -0.08863 | -3.05753 | -3.21862 | -3.151903 | -3.21535 | -2.888279 | -3.17057 | -1.354239 | -3.185496 | -2.651967 |
| sampleHC10-1-1 | 2 | 0.189471 | -2.91669 | -3.02566 | -2.935382 | -3.01177 | -2.84077 | -2.927807 | -1.914981 | -3.008744 | -2.084321 |
| sampleHC10-2-1 | 1 | 1.293267 | -1.97193 | -2.03204 | -1.798805 | -2.02698 | -1.994675 | -2.00571 | -0.448743 | -2.021978 | -1.825382 |
| sampleHC10-3-1 | 3 | 0.475011 | -2.62453 | -2.70709 | -2.699324 | -2.70709 | -2.665273 | -2.682706 | -1.358374 | -2.691691 | -2.63769 |
| sampleHC11-1-1 | 3 | 0.108632 | -3.0046 | -2.60451 | -3.112668 | -3.15919 | -3.092464 | -3.104473 | -3.107188 | -3.14858 | -1.955456 |
| sampeHC11-2-1 | 3 | 0.334428 | -2.80732 | -2.55776 | -2.874902 | -2.77531 | -2.876397 | -2.863124 | -2.874902 | -2.909043 | -1.847433 |
| sampleHC11-3-1 | 2 | 0.680228 | -2.46514 | -2.54915 | -2.476603 | -2.54234 | -2.525763 | -2.517705 | -2.491376 | -2.527392 | -1.296944 |
| sampleHC12-1-1 | 2 | 0.67361 | -2.53306 | -2.63066 | -2.597739 | -262895 | -2.586783 | -2.574592 | -1.520146 | -2.618849 | -1.676224 |
| sampleHC12-2-1 | 2 | 0.62704 | -2.54409 | -2.61545 | -2.362673 | -2.61545 | -2.565063 | -2.590338 | -1.416966 | -2.61891 | -2.21682 |
| sampleHC12-3-1 | 3 | 0.052254 | -3.01204 | -3.16573 | -3.063339 | -3.15889 | -3.095838 | -3.088563 | -0.649341 | -3.157197 | -2.375679 |
| sampeHC13-1-1 | 2 | 0.24351 | -2.8561 | -2.97988 | -2.743497 | -2.97377 | -2.654159 | -2.906387 | -2.935979 | -2.978347 | -2.43758 |
| sampleHC13-2-1 | 1 | 0.119637 | -2.89664 | -3.05512 | -2.896641 | -2.99731 | -2.235188 | -2.93634 | -0.228834 | -1.929718 | -2.515184 |
| sampleHC13-3-1 | 3 | 0.139825 | -2.82186 | -2.96858 | -2.81494 | -2.91728 | -2.309668 | -2.88156 | -1.307255 | -2.957553 | -2.460476 |
| sampleHC14-1-1 | 2 | -0.08179 | -2.98072 | -3.14502 | -3.091235 | -3.12723 | -2.539134 | -3.079069 | -3.097448 | -2.184154 | -2.370606 |
| sampleHC14-2-1 | 2 | 0.08208 | -2.71569 | -2.79243 | -2.740875 | -2.7496 | -2.403093 | -2.765318 | -1.661604 | -1.577648 | -2.373825 |
| sampleHC14-3-1 | 3 | -0.00953 | -2.92108 | -3.06446 | -3.027698 | -3.04307 | -2.859654 | -3.037887 | -1.850183 | -2.596568 | -2.465837 |
| sampleHC15-1-1 | 3 | 0.339464 | -2.60997 | -2.78433 | -2.77792 | -2.77538 | -2.5141 | -2.761671 | -1.386136 | -2.784335 | -2.522506 |
| sampleHC15-2-1 | 2 | 0.416785 | -2.61916 | -2.75211 | -2.580146 | -2.73698 | -2.611296 | -2.715222 | -0.45709 | -2.309709 | -2.114224 |
| sampleHC15-3-1 | 3 | -0.16102 | -3.08724 | -2.32976 | -3.297696 | -3.30935 | -3.0057 | -3.29388 | -1.478597 | -3.08254 | -3.058322 |
| sample21-1-2 | 3 | 0.18788 | -2.81092 | -2.663 | -2.910725 | -2.94791 | -2.673572 | -1.265506 | -1.975368 | -2.880146 | -0.663498 |
| sample21-2-2 | 4 | 0.076999 | -2.71051 | -2.75289 | 0.175236 | -3.0688 | -2.977715 | -2.239589 | -3.067481 | -3.067481 | -2.985249 |
| sample22-1-2 | 3 | -0.14226 | -3.00913 | -3.17166 | -3.116077 | -3.14223 | -3.120328 | -2.978287 | -2.543175 | -3.149783 | -2.496401 |
| sample22-2-2 | 5 | -0.24881 | -1.8431 | -2.95832 | -0.455958 | -2.69883 | -2.841009 | -3.175155 | -3.172788 | -3.144244 | -3.107193 |
| sample23-1-2 | 2 | -0.19429 | -3.0251 | -3.17142 | -3.071819 | -3.13894 | -2.353964 | -1.912044 | -1.339306 | -2.820737 | -1.76668 |
| sample23-2-2 | 4 | -0.18102 | -2.78 | -3.1316 | -3.080446 | -3.1084 | -2.931469 | -2.308021 | -2.164027 | -3.056407 | -2.128826 |
| sample24-1-2 | 3 | 0.043086 | -2.77024 | -2.92031 | -2.884001 | -2.87595 | -2.757903 | -2.864696 | -0.518063 | -1.89458 | -2269352 |
| sample24-2-2 | 4 | 0.012332 | -2.88066 | -29288 | -2.919158 | -2.82779 | -2.473516 | -2.909728 | -1.066857 | -2.000847 | -2.046647 |
| sample25-1-2 | 2 | 0.226923 | -2.77942 | -2.90333 | -2.709673 | -2.89513 | -2.737937 | -2.85757 | -1.282944 | -2.877892 | -2.070414 |
| sample28-1-2 | 2 | 0.390321 | -2.58501 | -2.67429 | -2.647728 | -266525 | -2.507423 | -2625023 | -0.705314 | -2.657655 | -2.38049 |
| sample28-2-2 | 3 | 1.089113 | -1.85578 | -1.92459 | -1.937873 | -1.93922 | -1.86709 | -1.927215 | -1.90791 | -1.935184 | -1.931181 |
| sample29-1-2 | 3 | 0.719668 | -2.28613 | -2.33494 | -2.255996 | -2.3185 | -2.254834 | -2.30266 | -2.29241 1 | -2.319849 | -2.043088 |
| sample29-2-2 | 3 | 0.054244 | -2.8399 | -2.94276 | -2.922045 | -2.92086 | -2.61218 2 | -2.054263 | -2.917312 | -2.913795 | -2.588364 |
| sample30-1-2 | 3 | -0.14972 | -3.00704 | -2.83842 | -3.067634 | -3.08906 | -2.922004 | -3.072304 | -1.531062 | -3.134 | -2.347364 |
| sample30-2-2 | 3 | -0.31961 | -3.09969 | -3.2824 | -2.821285 | -3.27955 | -3.079532 | -3.240229 | -2.51461 | -3.244135 | -2.8931 17 |
| sample33-1-1 | 4 | -0.14031 | -2.56649 | -2.76747 | -0.781852 | -3.18389 | -3.086692 | -3.169605 | -3.111789 | -3.145016 | -2.373556 |
| sample33-2-1 | 9 | -0.41166 | -3.03008 | -3.18159 | -3.151535 | -3.15153 | -2.540417 | -2.213877 | -2.844238 | -2.946154 | -2.946154 |
| sample33-3-1 | 7 | -0.19569 | -2.76262 | -2.75824 | -0.50619 | -2.98105 | -3.138157 | -3.216874 | -3.136998 | -3.20997 | -2.985934 |
| sample34-1-1 | 4 | 0.024936 | -2.8217 | -2.92805 | -2.793182 | -2.88963 | -2.885061 | -2.894252 | -1.919842 | -2.909617 | -2.138337 |
| sample34-2-1 | 6 | -0.45525 | -1.45857 | -3.15556 | 0.1732187 | -3.19307 | -3.093341 | -3.222336 | -3256226 | -3.195255 | -3256226 |
| sample34-3-1 | 10 | -0.41108 | -1.80088 | -3.19843 | -0.037692 | -3.18403 | -2.998596 | -3.212056 | -3.050905 | -3.161044 | -3.140281 |
| sample35-1-1 | 4 | -0.20166 | -3.05706 | -295682 | -2.528478 | -3.1862 | -3.145955 | -3.175095 | -1.541978 | -3.203398 | -2.494883 |

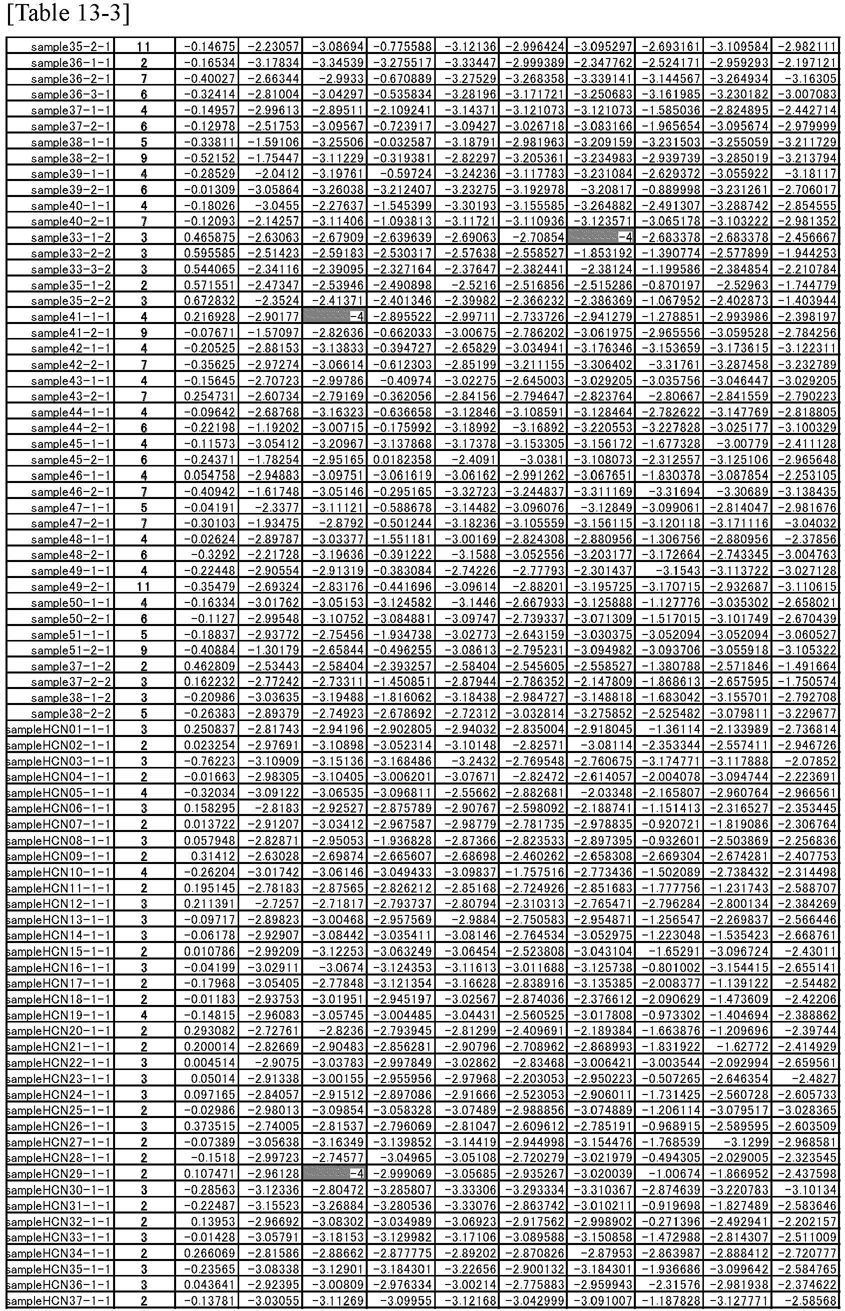

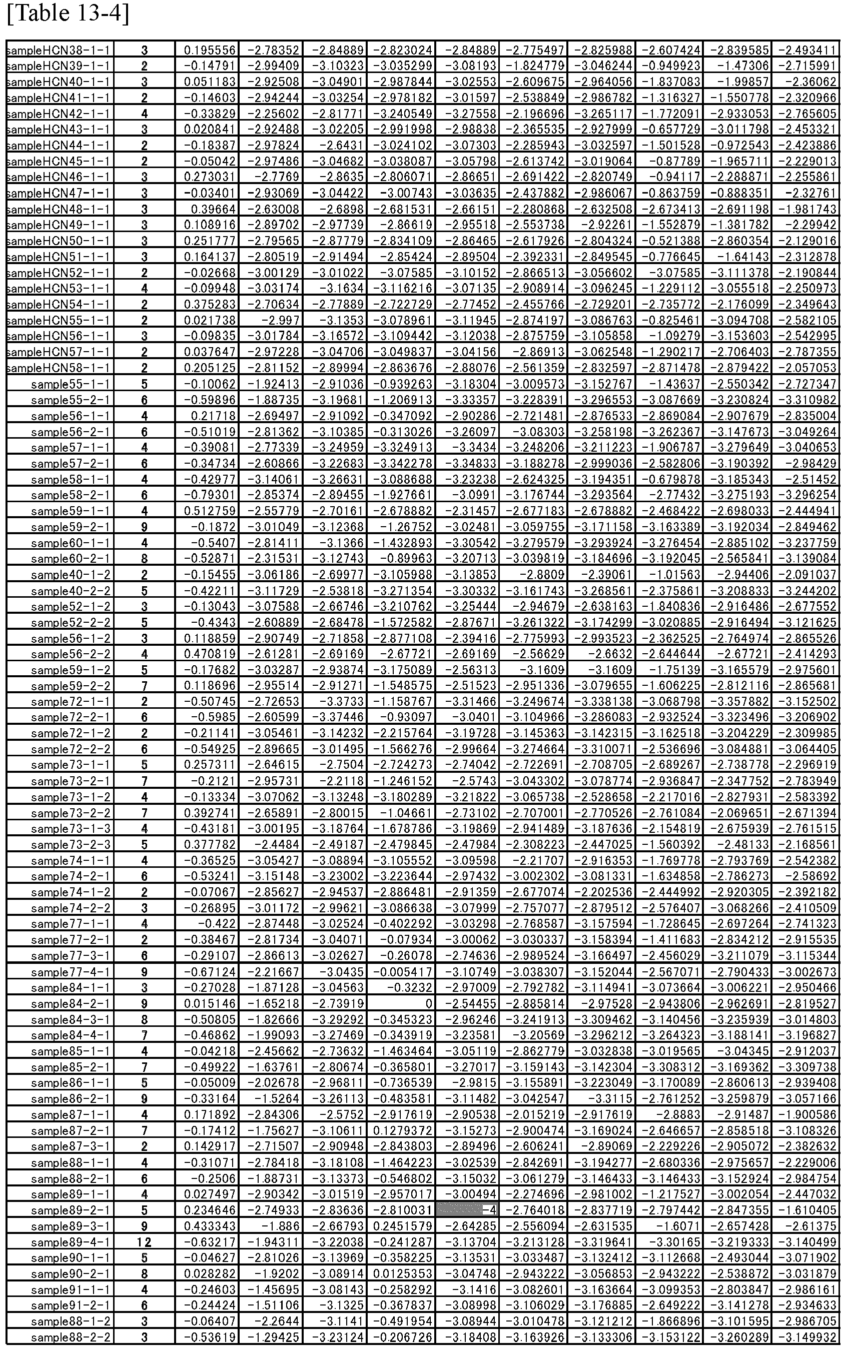

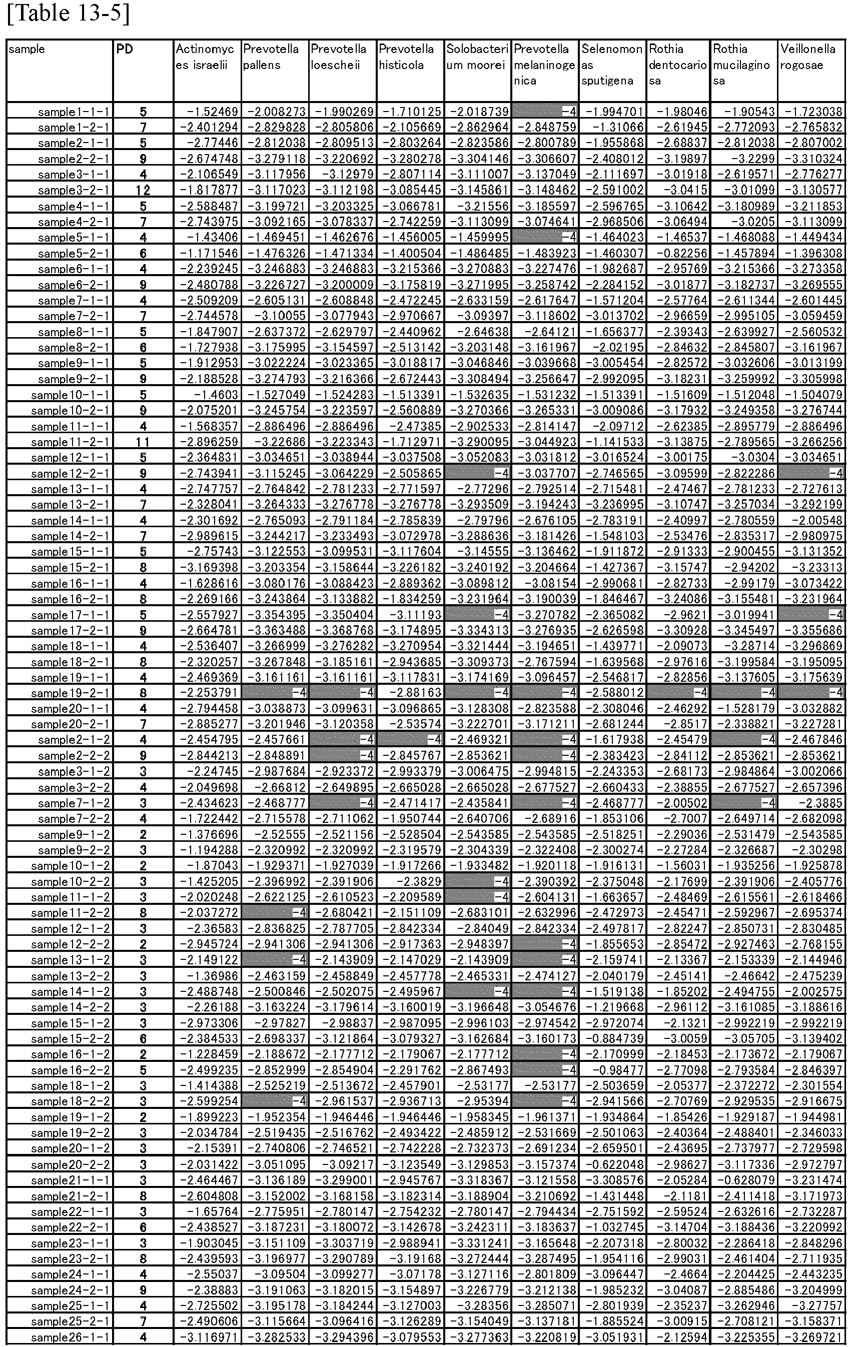

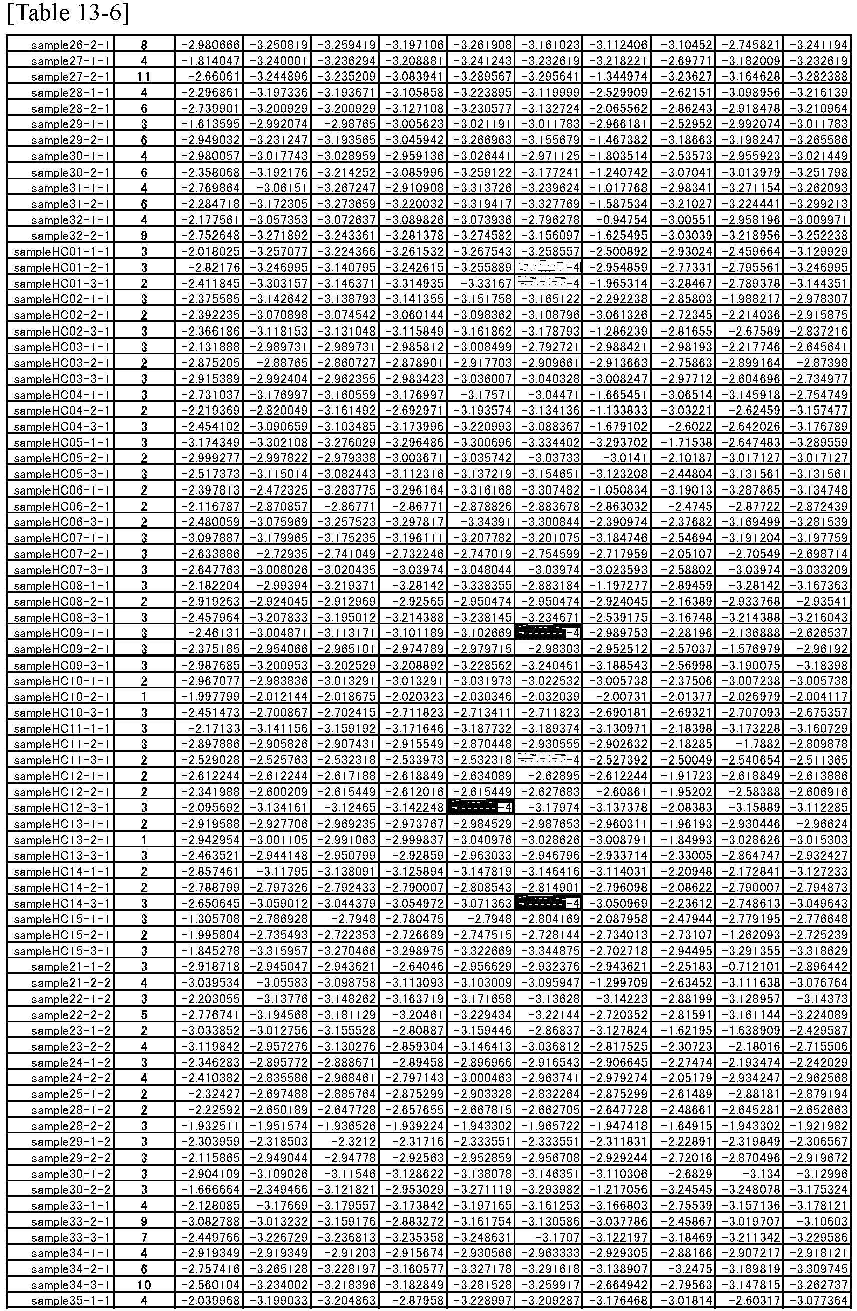

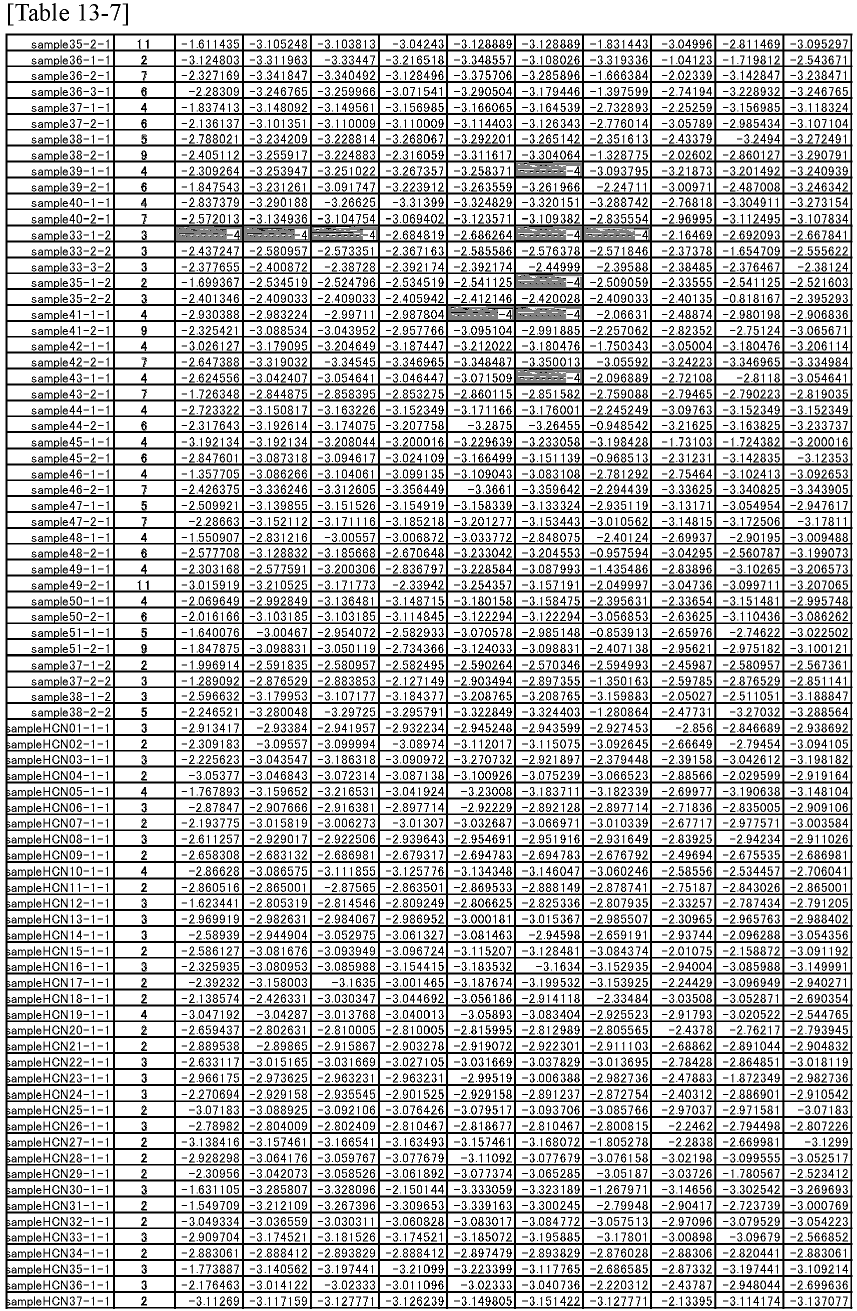

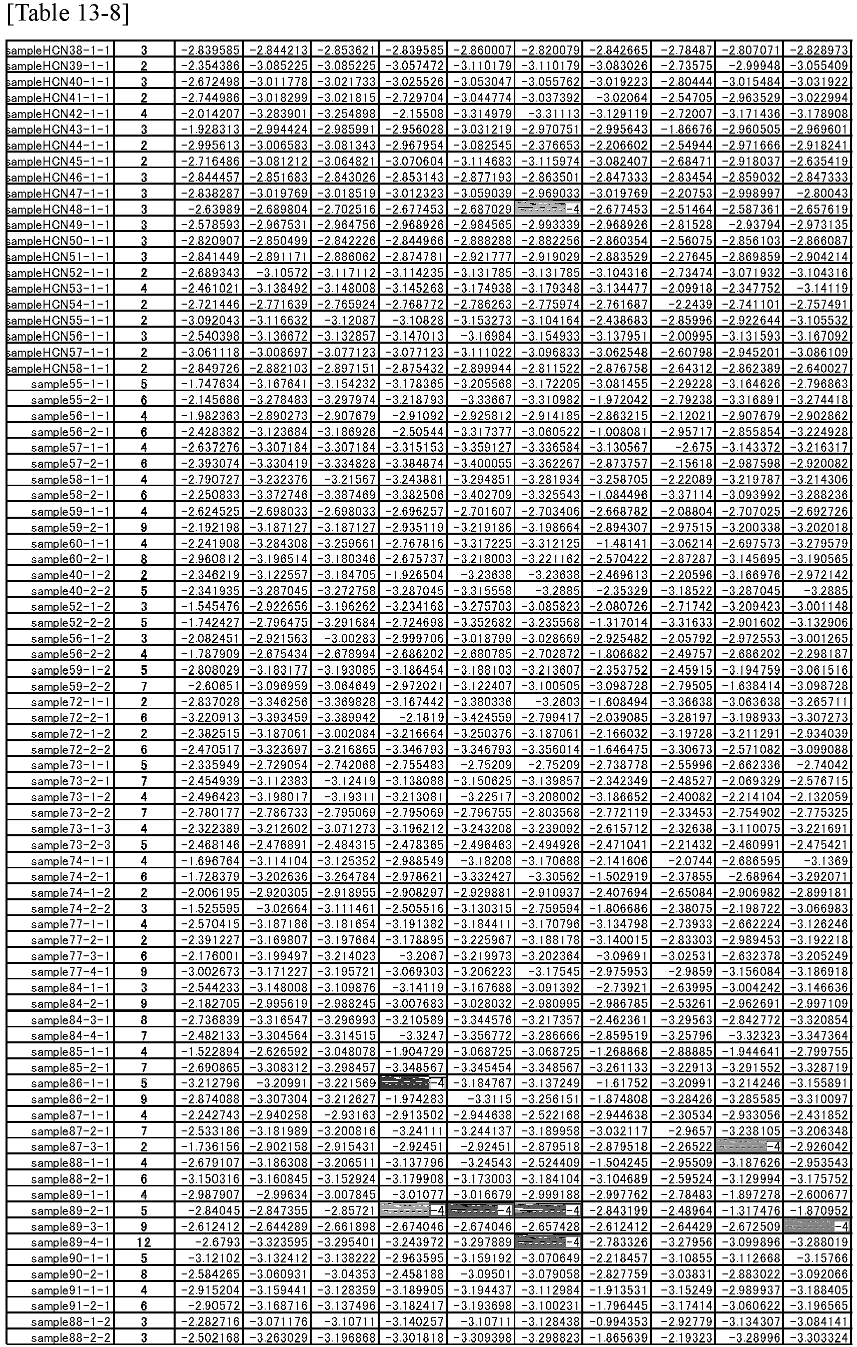

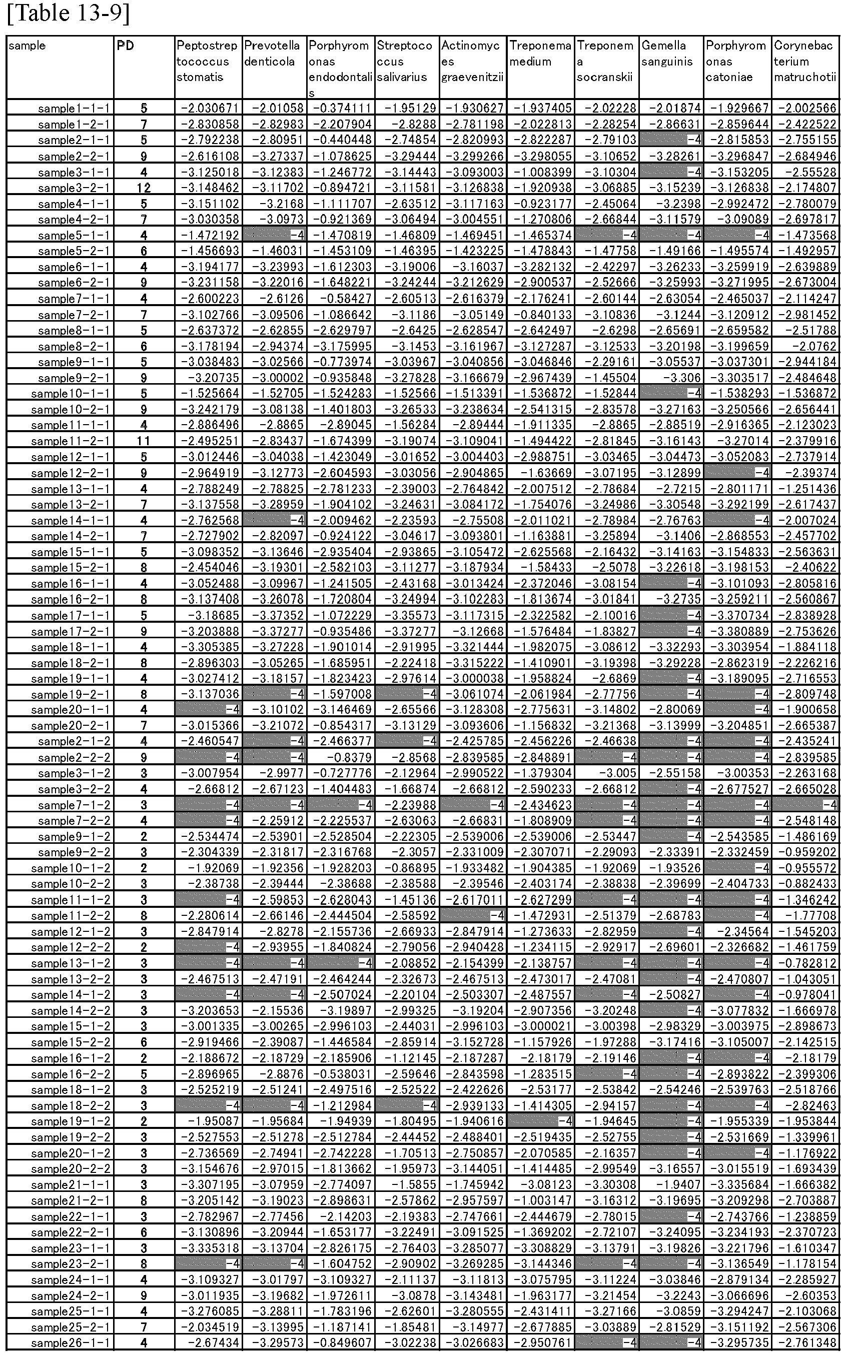

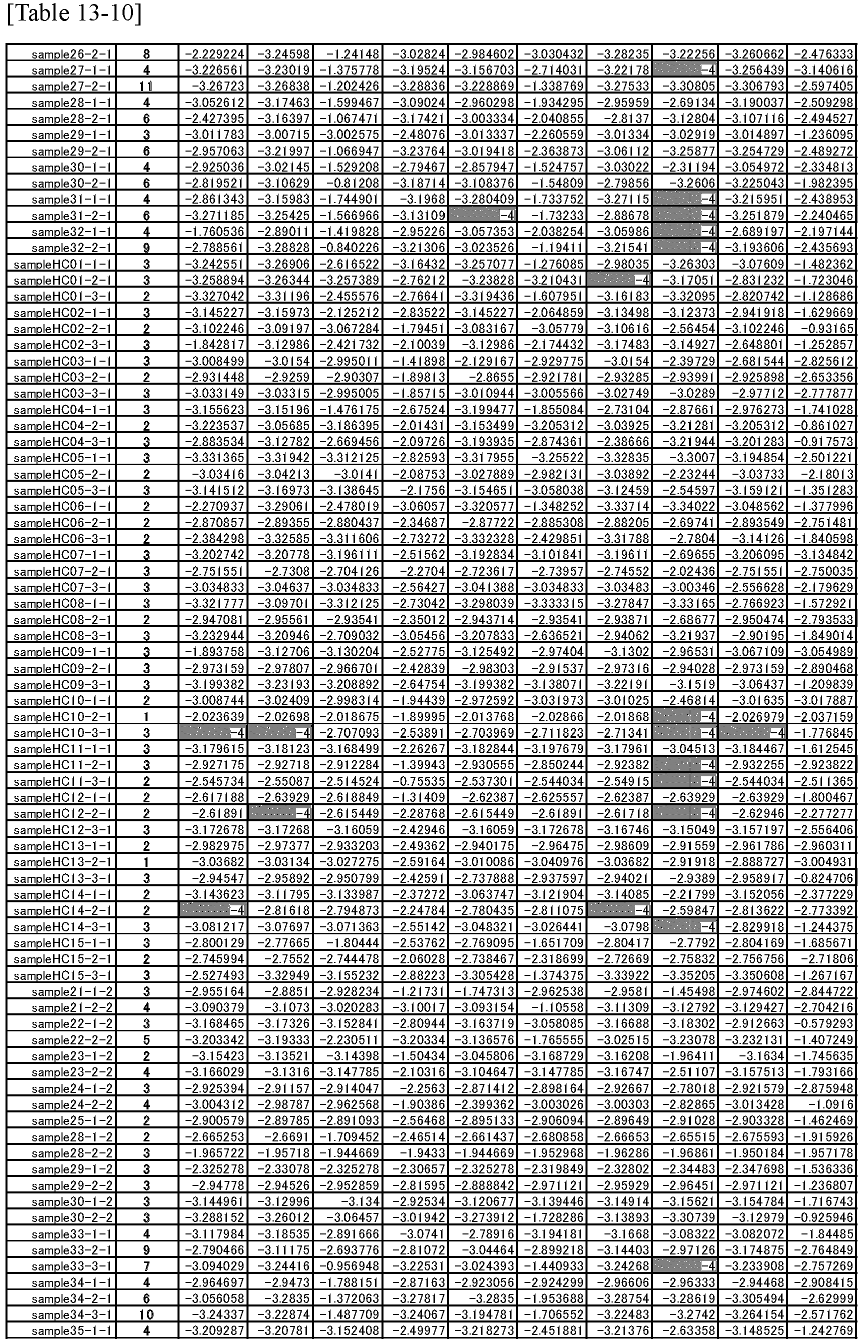

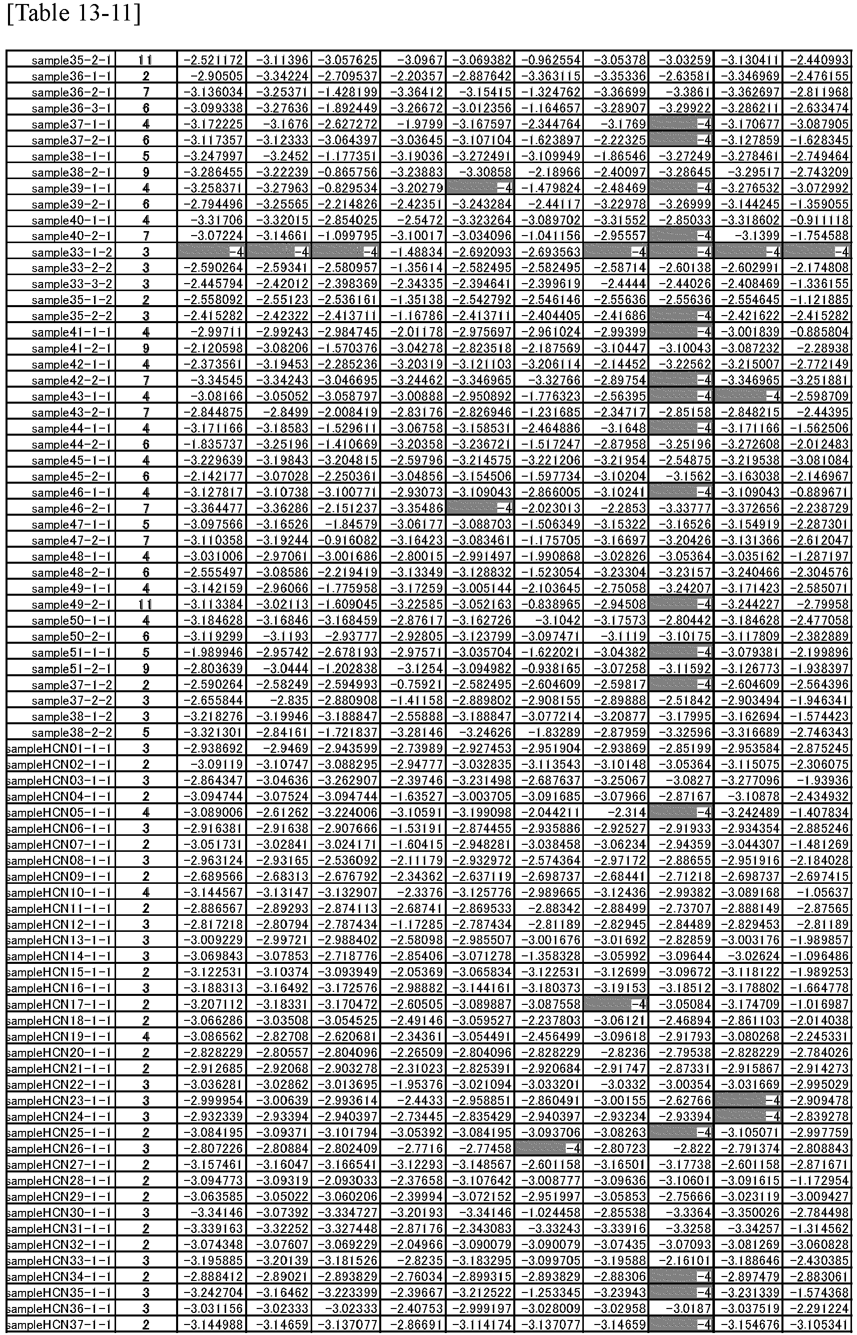

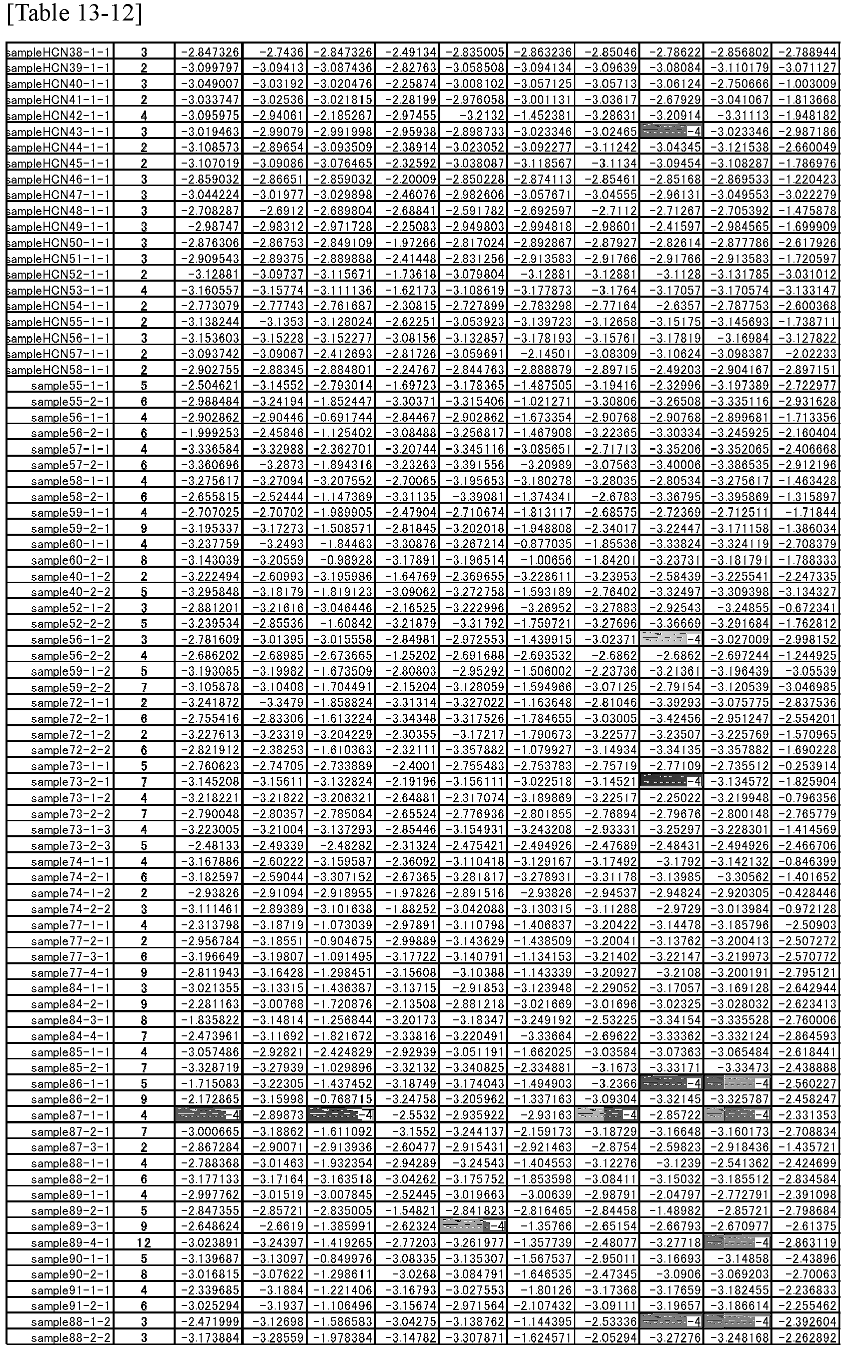

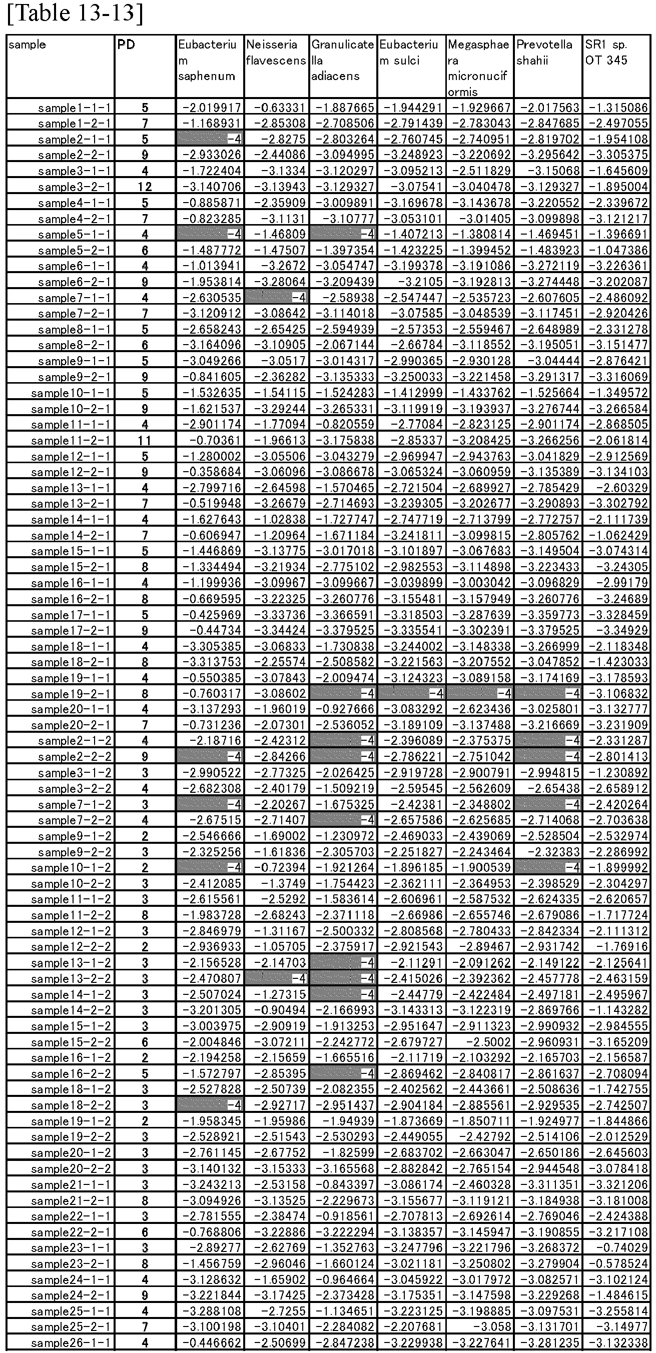

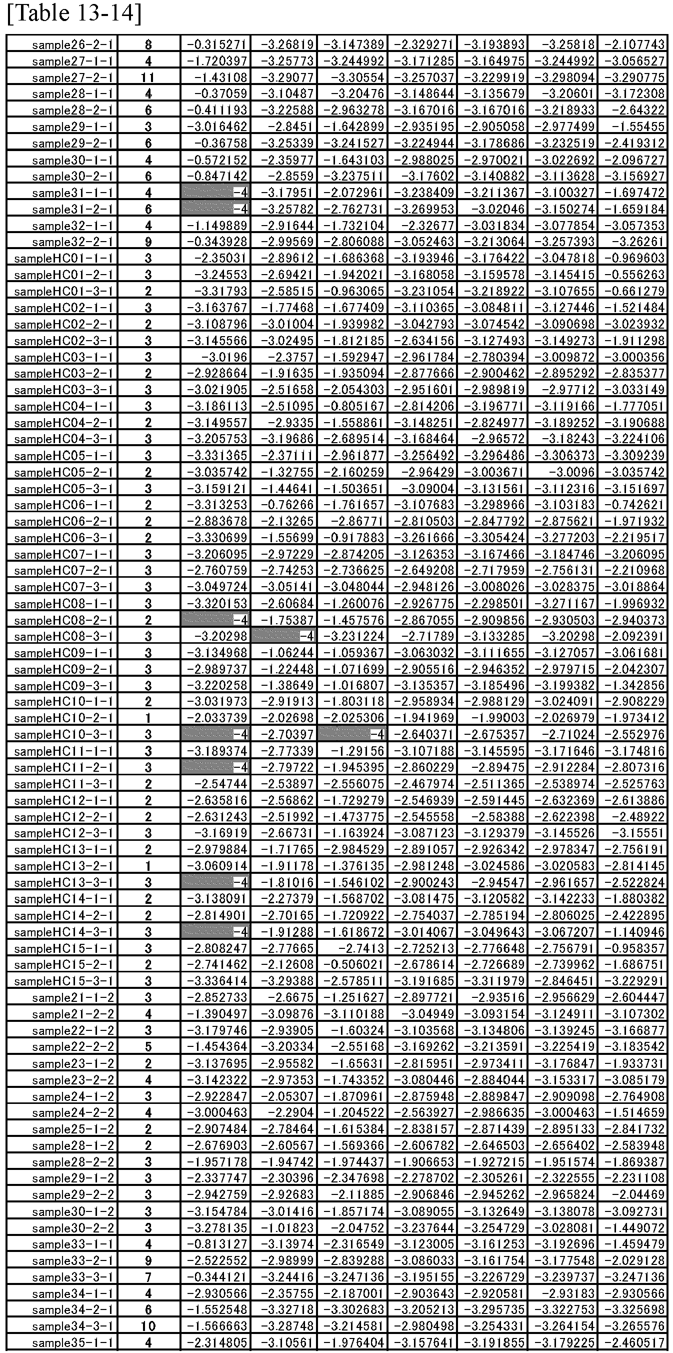

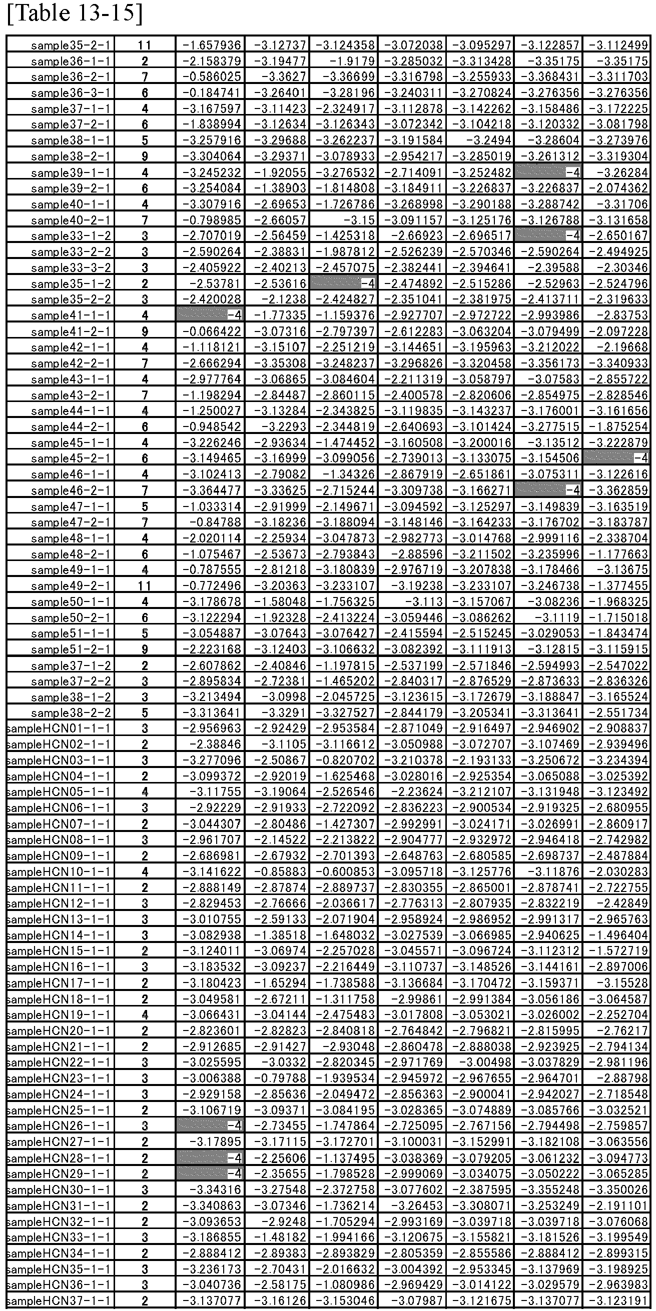

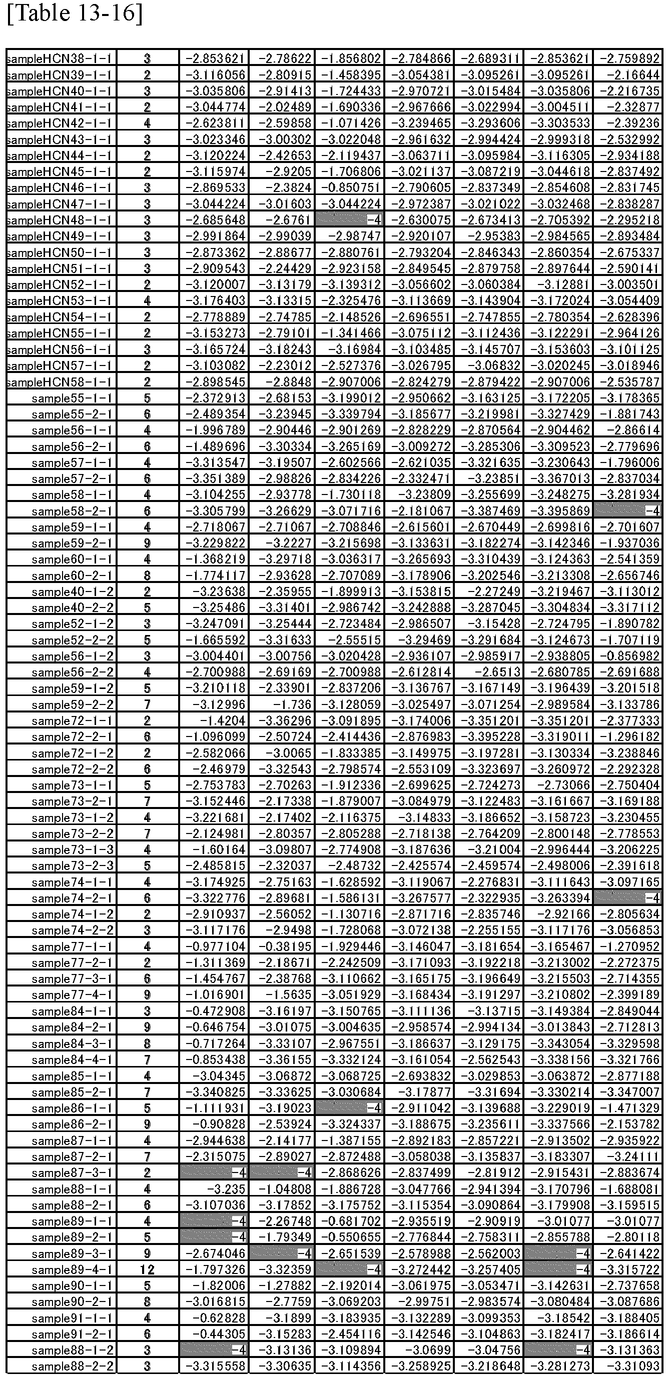

The correlation coefficient of periodontal pocket depth (Pd) and the value of log10 (relative ratio to the total bacterial load) of each bacterium was calculated for all 36 types of bacteria, and further, the p-value and q-value were calculated regarding the significance of these correlations (Table 14).

**[Table 14]**

| 6 species of bad bacteria, 23 species of good bacteria | | | |
|---|---|---|---|
| | Correlation coefficient | p value | q value |
| Filifactor alocis | 0.640 | 2.12E-38 | 7.63E-37 |
| Porphyromonas endodontalis | 0.556 | 1.94E-27 | 3.49E-26 |
| Eubacterium nodatum | 0.532 | 8.13E-25 | 9.76E-24 |
| Eubacterium saphenum | 0.468 | 7.74E-19 | 3.98E-18 |
| Treponema medium | 0.426 | 1.46E-15 | 5.84E-15 |
| Selenomonas sputigena | 0.203 | 0.000254 | 0.000366 |
| Prevotella denticola | -0.163 | 0.003427 | 0.004254 |
| Prevotella melaninogenica | -0.163 | 0.00334 | 0.004254 |
| Gemella sanguinis | -0.164 | 0.003243 | 0.004254 |
| Eubacterium sulci | -0.194 | 0.000475 | 0.000658 |
| Corynebacterium matruchotii | -0.208 | 0.000177 | 0.000265 |
| Rothia mucilaginosa | -0.209 | 0.000166 | 0.00026 |
| Porphvromonas catoniae | -0.227 | 3.96E-05 | 6.48E-05 |
| Solobacterium moorei | -0.260 | 2.29E-06 | 3.93E-06 |
| Neisseria flavescens | -0.262 | 1.91 E-06 | 3.44E-06 |
| Prevotella loescheii | -0.274 | 6.32E-07 | 1.2E-06 |
| Megasphaera micronuciformis | -0.274 | 6.1 E-07 | 1.2E-06 |
| Actinomyces graevenitzii | -0.276 | 5.13E-07 | 1.09E-06 |
| Veillonella atypica | -0.279 | 3.64E-07 | 8.19E-07 |
| Prevotella pallens | -0.306 | 2.09E-08 | 5.02E-08 |
| Prevotella shahii | -0.315 | 8.27E-09 | 2.13E-08 |
| Porphyromonas pasteri | -0.328 | 1.65E-09 | 4.57E-09 |
| Veillonella rogosae | -0.357 | 4.17E-11 | 1.25E-10 |
| Alloprevotella spp. (A. rava,OT 308) | -0.365 | 1.47E-11 | 4.81 E-11 |
| Rothia dentocariosa | -0.400 | 8.94E-14 | 3.22E-13 |
| Granulicatella adiacens | -0.433 | 4.59E-16 | 2.07E-15 |
| Streptococcus salivarius | -0.469 | 6.3E-19 | 3.78E-18 |
| Haemophilus parainfluenzae | -0.493 | 4.58E-21 | 3.3E-20 |
| Streptococcus parasanguinis | -0.519 | 1.7E-23 | 1.53E-22 |

These values were calculated using the statistical software "R" (R Development Core Team) with the cor function for the correlation coefficient, the cor.test function for the p value, and the p.adjust function for the q value ("BH" was specified in the "methods" option).

Regarding the significance of the correlation coefficients shown in Table 14, it was specified that a bacterium shows a significant correlation when the significance level is "q value < 0.05." Next, the bacteria having a significant correlation were roughly divided into the "bacterial species that increases as the periodontal pocket value increases" and the "bacterial species that decreases as the periodontal pocket value increases" based on the positive or negative correlation coefficient. The group of "bacterial species that increases as the periodontal pocket value increases" was set to consist of 6 bacterial species, namely Filifactor alocis, Porphyromonas endodontalis, Eubacterium nodatum, Eubacterium saphenum, Treponema medium, and Selenomonas sputigena.

The group of "bacterial species that decreases as the periodontal pocket value increases" was set to consist of 23 bacterial species, namely Prevotella denticola, Prevotella melaninogenica, Gemella sanguinis, Eubacterium sulci, Corynebacterium matruchotii, Rothia mucilaginosa, Porphyromonas catoniae, Solobacterium moorei, Neisseria flavescens, Prevotella loescheii, Megasphaera micronuciformis, Actinomyces graevenitzii, Veillonella atypica, Prevotella pallens, Prevotella shahii, Porphyromonas pasteri, Veillonella rogosae, Alloprevotella spp. (A. rava, OT 308), Rothia dentocariosa, Granulicatella adiacens, Streptococcus salivarius, Haemophilus parainfluenzae, and Streptococcus parasanguinis.

Subsequently, the average of the group of "bacterial species that increases as the periodontal pocket value increases" and the group of "bacterial species that decreases as the periodontal pocket value increases" was calculated using the value of log10 (relative ratio to total load). Lastly, regarding the average, the balance index of bacterial groups was calculated by subtracting the value of the group of "bacterial species that decreases as the periodontal pocket value increases" from the value of the group of "bacterial species that increases as the periodontal pocket value increases." The values are shown in Figure 15 (Table 15-1 to Table 15-7)

**[Table 15-1]**

| **Average of bad bacteria** | **Average of good bacteria** | **PD** | **Balanc e index** |
|---|---|---|---|
| -1.51185 | -1.90458 | **5** | **0.3927** |
| -1.56808 | -2.66196 | **7** | **1.0939** |
| -2.45595 | -2.78445 | **5** | **0.3285** |
| -2.40632 | -3.19651 | **9** | **0.7902** |
| -1.59108 | -3.04501 | **4** | **1.4539** |
| -2.11942 | -3.03354 | **12** | **0.9141** |
| -1.51453 | -3.01605 | **5** | **1.5015** |
| -1.47366 | -3.0526 | **7** | **1.5789** |
| -1.88411 | -2.00151 | **4** | **0.1174** |
| -1.46719 | -1.39532 | **6** | **-0.072** |
| -1.70827 | -3.15683 | **4** | **1.4486** |
| -1.77087 | -3.17876 | **9** | **1.4079** |
| -1.77134 | -2.60886 | **4** | **0.8375** |
| -1.82566 | -3.05628 | **7** | **1.2306** |
| -2.45961 | -2.58537 | **5** | **0.1258** |
| -2.85454 | -2.98057 | **6** | **0.126** |
| -2.15911 | -2.98192 | **5** | **0.8228** |
| -1.70904 | -3.1417 | **9** | **1.4327** |
| 1.51994 | -1.60888 | **5** | **0.0889** |
| -1.80002 | -3.19844 | **9** | **1.3984** |
| -2.35963 | -2.47637 | **4** | **0.1167** |
| -1.27707 | -2.99334 | **11** | **1.7163** |
| -2.04888 | -2.96425 | **5** | **0.9154** |
| -1.50674 | -3.11663 | **9** | **1.6099** |
| -2.62661 | -2.50493 | **4** | **-0.122** |
| -1.70773 | -3.17024 | **7** | **1.4625** |
| -2.22261 | -2.57405 | **4** | **0.3514** |
| -1.13973 | -2.78798 | **7** | **1.6482** |
| -1.80924 | -3.00287 | **5** | **1.1936** |
| -1.40118 | -3.07771 | **8** | **1.6765** |
| -1.85023 | -3.04879 | **4** | **1.1986** |
| -1.52302 | -3.1659 | **8** | **1.6429** |
| -1.57378 | -3.32301 | **5** | **1.7492** |
| -1.4188 | -3.31282 | **9** | **1.894** |
| -1.93506 | -3.03919 | **4** | **1.1041** |
| -1.63395 | -2.9855 | **8** | **1.3516** |
| -1.60191 | -2.9818 | **4** | **1.3799** |
| -1.6688 | -3.71625 | **8** | **2.0475** |
| -2.90677 | -2.5598 | **4** | **-0.347** |
| -1.16009 | -2.94042 | **7** | **1.7803** |

**[Table 15-2]**

| | | | |
|---|---|---|---|
| -2.2614 | -3.11524 | **4** | **0.8538** |
| -2.61218 | -3.18576 | **9** | **0.5736** |
| -2.06611 | -2.73513 | **3** | **0.669** |
| -2.43207 | -2.50549 | **4** | **0.0734** |
| -2.96376 | -2.95071 | **3** | **-0.013** |
| -2.31696 | -2.8179 | **4** | **0.5009** |
| -2.50696 | -2.36912 | **2** | **-0.138** |
| -2.30178 | -2.2123 | **3** | **-0.089** |
| -2.24343 | -1.93077 | **2** | **-0.313** |
| -2.35569 | -2.30188 | **3** | **-0.054** |
| -2.45441 | -2.55975 | **3** | **0.1053** |
| -2.0859 | -2.6802 | **8** | **0.5943** |
| -2.25358 | -2.62815 | **3** | **0.3746** |
| -2.23614 | -2.73713 | **2** | **0.501** |
| -2.45617 | -2.49485 | **3** | **0.0387** |
| -2.38646 | -2.5784 | **3** | **0.1919** |
| 2.29529 | -2.57963 | **3** | **0.2843** |
| -2.78851 | -2.84604 | **3** | **0.0575** |
| -2.94716 | -2.74888 | **3** | **-0.198** |
| -1.69451 | -2.78791 | **6** | **1.0934** |
| -2.17616 | -2.32099 | **2** | **0.1448** |
| -1.2679 | -2.95606 | **5** | **1.6882** |
| -2.50638 | -2.40763 | **3** | **-0.099** |
| -2.0615 | -3.1845 | **3** | **1.123** |
| -2.25845 | -1.97274 | **2** | **-0.286** |
| -2.4779 | -2.48399 | **3** | **0.0061** |
| -2.59696 | -2.53369 | **3** | **-0.063** |
| -1.9359 | -2.84744 | **3** | **0.9115** |
| -3.05524 | -2.40253 | **3** | **-0.653** |
| -1.8986 | -2.90775 | **8** | **1.0092** |
| -2.43224 | -2.57159 | **3** | **0.1394** |
| -1.16576 | -3.14264 | **6** | **1.9769** |
| -2.93588 | -2.81319 | **3** | **-0.123** |
| -1.98347 | -2.95364 | **8** | **0.9702** |
| -3.05666 | -2.61738 | **4** | **-0.439** |
| -1.85972 | -3.07762 | **9** | **1.2179** |
| -2.7068 | -2.837 | **4** | **0.1302** |
| -2.19757 | -2.8783 | **7** | **0.6807** |
| -1.78529 | -3.10267 | **4** | **1.3174** |
| -1.69736 | -3.04882 | **8** | **1.3515** |
| -2.10323 | -3.19105 | **4** | **1.0878** |
| -1.22554 | -3.22888 | **11** | **2.0033** |
| -1.48753 | -3.04874 | **4** | **1.5612** |
| -1.27083 | -3.08447 | **6** | **1.8136** |
| -2.82728 | -2.72762 | **3** | **-0.1** |
| -1.16697 | -3.16362 | **6** | **1.9966** |
| -1.33751 | -2.71029 | **4** | **1.3728** |
| -1.17428 | -3.09345 | **6** | **1.9192** |
| -2.1631 | -3.1223 | **4** | **0.9592** |
| -2.07357 | -3.2192 | **6** | **1.1456** |

**[Table 15-3]**

| | | | |
|---|---|---|---|
| -1.61276 | -2.90396 | **4** | **1.2912** |
| -1.02768 | -3.15445 | **9** | **2.1268** |
| -2.2846 | -2.95095 | **3** | **0.6664** |
| -3.15062 | -2.85584 | **3** | **-0.295** |
| -2.61968 | -2.91849 | **2** | **0.2988** |
| -2.36883 | -2.70867 | **3** | **0.3398** |
| -2.83252 | -2.62098 | **2** | **-0.212** |
| -2.49988 | -2.77529 | **3** | **0.2754** |
| -2.96629 | -2.57006 | **3** | **-0.396** |
| -2.89198 | -2.59028 | **2** | **-0.302** |
| -2.98929 | -2.6883 | **3** | **-0.301** |
| -2.07897 | -2.68571 | **3** | **0.6067** |
| -2.80986 | -2.78343 | **2** | **-0.026** |
| -2.77675 | -2.86664 | **3** | **0.0899** |
| -3.26311 | -2.9469 | **3** | **-0.316** |
| -2.98315 | -2.56114 | **2** | **-0.422** |
| -3.09606 | -2.63255 | **3** | **-0.464** |
| -2.43866 | -2.80828 | **2** | **0.3696** |
| -2.84996 | -2.60525 | **2** | **-0.245** |
| -2.89807 | -2.71355 | **2** | **-0.185** |
| -3.14875 | -2.97835 | **3** | **-0.17** |
| -2.71774 | -2.58371 | **3** | **-0.134** |
| -3.0102 | -2.82419 | **3** | **-0.186** |
| -2.92817 | -2.77711 | **3** | **-0.151** |
| -3.07024 | -2.55146 | **2** | **-0.519** |
| -2.88831 | -3.10258 | **3** | **0.2143** |
| -3.04819 | -2.64473 | **3** | **-0.403** |
| -2.92893 | -2.6115 | **3** | **-0.317** |
| -3.16087 | -2.76068 | **3** | **-0.4** |
| -2.98668 | -2.75448 | **2** | **-0.232** |
| -1.97652 | -2.01959 | **1** | **0.0431** |
| -2.90549 | -2.81259 | **3** | **-0.093** |
| -3.13396 | -2.85051 | **3** | **-0.283** |
| -3.0579 | -2.70823 | **3** | **-0.35** |
| -2.51252 | -2.52236 | **2** | **0.0098** |
| -2.60388 | -2.36278 | **2** | **-0.241** |
| -2.5635 | -2.54631 | **2** | **-0.017** |
| -3.1192 | -2.82523 | **3** | **-0.294** |
| -2.90629 | -2.79928 | **2** | **-0.107** |
| -2.98854 | -2.58961 | **1** | **-0.399** |
| -3.07648 | -2.55902 | **3** | **-0.517** |
| -3.09666 | -2.73125 | **2** | **-0.365** |
| -2.77892 | -2.54189 | **2** | **-0.237** |
| -3.18293 | -2.75794 | **3** | **-0.425** |
| -2.29004 | -2.62234 | **3** | **0.3323** |
| -2.62299 | -2.36626 | **2** | **-0.257** |
| -2.82561 | -3.01902 | **3** | **0.1934** |
| -2.90146 | -2.30125 | **3** | **-0.6** |
| -1.55856 | -3.01099 | **4** | **1.4524** |
| -3.10969 | -2.85991 | **3** | **-0.25** |

**[Table 15-4]**

| | | | |
|---|---|---|---|
| -1.74497 | -3.05369 | **5** | **1.3087** |
| -3.11252 | -2.44418 | **2** | **-0.668** |
| -3.01931 | -2.68253 | **4** | **-0.337** |
| -2.88266 | -2.51429 | **3** | **-0.368** |
| -2.95753 | -2.45588 | **4** | **-0.502** |
| -2.84484 | -2.61565 | **2** | **-0.229** |
| -2.49128 | -2.45287 | **2** | **-0.038** |
| -1.93265 | -1.92646 | **3** | **-0.006** |
| -2.30614 | -2.26629 | **3** | **-0.04** |
| -2.92632 | -2.74299 | **3** | **-0.183** |
| -3.1022 | -2.8651 | **3** | **-0.237** |
| -2.53484 | -2.87738 | **3** | **0.3425** |
| -2.23569 | -2.98626 | **4** | **0.7506** |
| -2.88916 | -2.94229 | **9** | **0.0531** |
| -1.52217 | -3.21019 | **7** | **1.688** |
| -2.69787 | -2.78428 | **4** | **0.0864** |
| -1.55043 | -3.23491 | **6** | **1.6845** |
| -1.54407 | -3.15384 | **10** | **1.6098** |
| -2.78018 | -2.85494 | **4** | **0.0748** |
| -1.75262 | -3.03157 | **11** | **1.279** |
| -3.0007 | -2.75612 | **2** | **-0.245** |
| -1.38995 | -3.21445 | **7** | **1.8245** |
| -1.33089 | -3.17929 | **6** | **1.8484** |
| -2.66298 | -2.9425 | **4** | **0.2795** |
| -2.09079 | -3.01419 | **6** | **0.9234** |
| -1.92008 | -3.18087 | **5** | **1.2608** |
| -1.62702 | -3.13566 | **9** | **1.5086** |
| -1.88114 | -3.24502 | **4** | **1.3639** |
| -2.73804 | -2.79818 | **6** | **0.0601** |
| -2.85521 | -2.96281 | **4** | **0.1076** |
| -1.50198 | -3.05984 | **7** | **1.5579** |
| -3.11181 | -3.05561 | **3** | **-0.056** |
| -2.56168 | -2.31301 | **3** | **-0.249** |
| -2.37802 | -2.29161 | **3** | **-0.086** |
| -2.51559 | -2.43086 | **2** | **-0.085** |
| -2.40015 | -2.23606 | **3** | **-0.164** |
| -2.96823 | -2.71291 | **4** | **-0.255** |
| -1.38574 | -2.93776 | **9** | **1.552** |
| -1.93934 | -3.11132 | **4** | **1.172** |
| -2.6136 | -3.33697 | **7** | **0.7234** |
| -2.17112 | -3.07174 | **4** | **0.9006** |
| -1.69448 | -2.79605 | **7** | **1.1016** |
| -1.80235 | -3.04831 | **4** | **1.246** |
| -1.03217 | -3.09124 | **6** | **2.0591** |
| -3.17378 | -2.81264 | **4** | **-0.361** |
| -1.62173 | -2.98534 | **6** | **1.3636** |
| -2.97682 | -2.80163 | **4** | **-0.175** |
| -1.95763 | -3.30036 | **7** | **1.3427** |
| -1.70783 | -3.00866 | **5** | **1.3008** |
| -1.3977 | -3.13119 | **7** | **1.7335** |

**[Table 15-5]**

| | | | |
|---|---|---|---|
| -2.31049 | -2.741 | **4** | **0.4305** |
| -1.39734 | -3.02262 | **6** | **1.6253** |
| -1.56521 | -3.00479 | **4** | **1.4396** |
| -1.40091 | -3.1692 | **11** | **1.7683** |
| -2.99819 | -2.77954 | **4** | **-0.219** |
| -3.04912 | -2.85876 | **6** | **-0.19** |
| -2.18025 | -2.94096 | **5** | **0.7607** |
| -1.42823 | -3.0238 | **9** | **1.5956** |
| -2.55502 | -2.38668 | **2** | **-0.168** |
| -2.37639 | -2.53834 | **3** | **0.162** |
| -2.91531 | -2.85716 | **3** | **-0.058** |
| -2.28695 | -3.13837 | **5** | **0.8514** |
| -2.91669 | -2.79558 | **3** | **-0.121** |
| -2.95203 | -2.94623 | **2** | **-0.006** |
| -2.98078 | -2.77888 | **3** | **-0.202** |
| -3.05693 | -2.72108 | **2** | **-0.336** |
| -2.95936 | -2.90014 | **4** | **-0.059** |
| -2.89294 | -2.65514 | **3** | **-0.238** |
| -2.99949 | -2.60494 | **2** | **-0.395** |
| -2.62822 | -2.65248 | **3** | **0.0243** |
| -2.67253 | -2.63783 | **2** | **-0.035** |
| -3.06522 | -2.51848 | **4** | **-0.547** |
| -2.85541 | -2.71419 | **2** | **-0.141** |
| -2.79269 | -2.63886 | **3** | **-0.154** |
| -2.97369 | -2.69696 | **3** | **-0.277** |
| -2.63062 | -2.59346 | **3** | **-0.037** |
| -3.08003 | -2.75807 | **2** | **-0.322** |
| -3.14048 | -2.89029 | **3** | **-0.25** |
| -3.12796 | -2.67981 | **2** | **-0.448** |
| -2.75991 | -2.62619 | **2** | **-0.134** |
| -2.83907 | -2.70043 | **4** | **-0.139** |
| -2.79717 | -2.58412 | **2** | **-0.213** |
| -2.88845 | -2.73347 | **2** | **-0.155** |
| -2.99859 | -2.88185 | **3** | **-0.117** |
| -2.95209 | -2.59232 | **3** | **-0.36** |
| -2.90339 | -2.78669 | **3** | **-0.117** |
| -3.07107 | -3.01945 | **2** | **-0.052** |
| -3.18989 | -2.61681 | **3** | **-0.573** |
| -2.82469 | -2.98091 | **2** | **0.1562** |
| -3.03747 | -2.63461 | **2** | **-0.403** |
| -3.17074 | -2.66961 | **2** | **-0.501** |
| -2.56325 | -3.14549 | **3** | **0.5822** |
| -3.206 | -2.78731 | **2** | **-0.419** |
| -3.05206 | -2.75452 | **2** | **-0.298** |
| -3.139 | -2.79872 | **3** | **-0.34** |
| -2.87429 | -2.91526 | **2** | **0.041** |
| -2.77786 | -2.91134 | **3** | **0.1335** |
| -2.86878 | -2.74124 | **3** | **-0.128** |
| -3.11152 | -3.00032 | **2** | **-0.111** |
| -2.83556 | -2.73102 | **3** | **-0.105** |

**[Table 15-6]**

| | | | |
|---|---|---|---|
| -3.06834 | -2.7368 | **2** | **-0.332** |
| -3.00759 | -2.67558 | **3** | **-0.332** |
| -3.0015 | -2.60423 | **2** | **-0.397** |
| -2.48119 | -2.88136 | **4** | **0.4002** |
| -2.99187 | -2.82763 | **3** | **-0.164** |
| -2.91916 | -2.65907 | **2** | **-0.26** |
| -3.06773 | -2.67588 | **2** | **-0.392** |
| -2.83883 | -2.50211 | **3** | **-0.337** |
| -3.01495 | -2.70258 | **3** | **-0.312** |
| -2.67618 | -2.68039 | **3** | **0.0042** |
| -2.94842 | -2.6725 | **3** | **-0.276** |
| -2.85091 | -2.64894 | **3** | **-0.202** |
| -2.876 | -2.57074 | **3** | **-0.305** |
| -3.09099 | -2.97411 | **2** | **-0.117** |
| -3.12464 | -2.82697 | **4** | **-0.298** |
| -2.75244 | -2.61944 | **2** | **-0.133** |
| -2.98928 | -2.78854 | **2** | **-0.201** |
| -3.12691 | -2.96833 | **3** | **-0.159** |
| -2.79091 | -2.81374 | **2** | **0.0228** |
| -2.8707 | -2.7613 | **2** | **-0.109** |
| -2.09971 | -2.83773 | **5** | **0.738** |
| -1.73823 | -3.23771 | **6** | **1.4995** |
| -1.71119 | -2.79827 | **4** | **1.0871** |
| -1.36962 | -3.09606 | **6** | **1.7264** |
| -2.99846 | -3.08676 | **4** | **0.0883** |
| -2.88005 | -3.07857 | **6** | **0.1985** |
| -3.16335 | -2.82608 | **4** | **-0.337** |
| -1.9489 | -3.11594 | **6** | **1.167** |
| -2-40442 | -2.59622 | **4** | **0.1918** |
| -2.30992 | -3.06421 | **9** | **0.7543** |
| -1.63638 | -3.19198 | **4** | **1.5556** |
| -1.59255 | -3.05101 | **8** | **1.4585** |
| -3.04974 | -2.61031 | **2** | **-0.439** |
| -2.56818 | -3.20144 | **5** | **0.6333** |
| -2.9884 | -2.81402 | **3** | **-0.174** |
| -1.75537 | -3.08338 | **5** | **1.328** |
| -2.69499 | -2.9355 | **3** | **0.2405** |
| -2.52748 | -2.5124 | **4** | **-0.015** |
| -2.49189 | -2.99176 | **5** | **0.4999** |
| -2.33864 | -2.80545 | **7** | **0.4668** |
| -1.65611 | -3.25413 | **2** | **1.598** |
| -1.67834 | -3.11246 | **6** | **1.4341** |
| -2.50223 | -2.95617 | **2** | **0.4539** |
| -1.87825 | -3.00886 | **6** | **1.1306** |
| -2.72511 | -2.54465 | **5** | **-0.18** |
| -2.64227 | -2.81457 | **7** | **0.1723** |
| -3.17591 | -2.65342 | **4** | **-0.522** |
| -2.36493 | -2.7181 | **7** | **0.3532** |
| -2.54643 | -2.936 | **4** | **0.3896** |
| -2-47714 | -2.39077 | **5** | **-0.086** |

**[Table 15-7]**

| | | | |
|---|---|---|---|
| -2.96085 | -2.64602 | **4** | **-0.315** |
| -2.96448 | -2.7951 | **6** | **-0.169** |
| -2.81977 | -2.57271 | **2** | **-0.247** |
| -2.8757 | -2.66374 | **3** | **-0.212** |
| -1.64476 | -2.79419 | **4** | **1.1494** |
| -1.61521 | -2.92719 | **2** | **1.312** |
| -1.65071 | -3.05397 | **6** | **1.4033** |
| -1.44279 | -3.01932 | **9** | **1.5765** |
| -1.66116 | -3.04903 | **3** | **1.3879** |
| -1.67138 | -2.90231 | **9** | **1.2309** |
| -1.64294 | -3.19588 | **8** | **1.5529** |
| -1.86769 | -3.23059 | **7** | **1.3629** |
| -2.05321 | -2.90902 | **4** | **0.8558** |
| -1.99502 | -3.23821 | **7** | **1.2432** |
| -1.40419 | -3.23032 | **5** | **1.8261** |
| -1.14982 | -3.1628 | **9** | **2.013** |
| -3.09693 | -2.67017 | **4** | **-0.427** |
| -1.79096 | -3.07276 | **7** | **1.2818** |
| -3.04563 | -2.81047 | **2** | **-0.235** |
| -2.05409 | -2.80733 | **4** | **0.7532** |
| -2.27716 | -3.1028 | **6** | **0.8256** |
| -3.14541 | -2.55656 | **4** | **-0.589** |
| -3.00901 | -2.49424 | **5** | **-0.515** |
| -1.61182 | -2.82829 | **9** | **1.2165** |
| -1.59034 | -3.35001 | **12** | **1.7597** |
| -1.60409 | -2.93721 | **5** | **1.3331** |
| -1.7829 | -2.9896 | **8** | **1.2067** |
| -1.21329 | -3.07967 | **4** | **1.8664** |
| -1.22205 | -3.04508 | **6** | **1.823** |
| -1.74695 | -3.11769 | **3** | **1.3707** |
| -1.71419 | -3.14869 | **3** | **1.4345** |

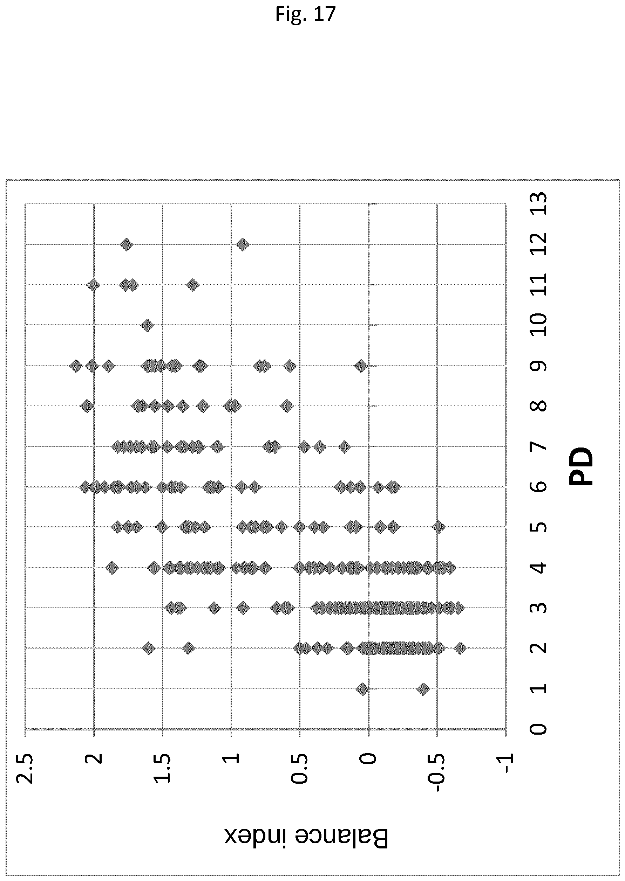

Figure 17 shows a scatter diagram in which the vertical axis represents the balance index and the horizontal axis represents the periodontal pocket depth (Pd). It was decided to determine the disease state/disease state by creating a discriminant model using data with a periodontal pocket depth of 1 mm to 3 mm and data with a periodontal pocket depth of 5 mm or more from the data shown in Figure 17 and using data with a periodontal pocket depth of 4 mm as test data.

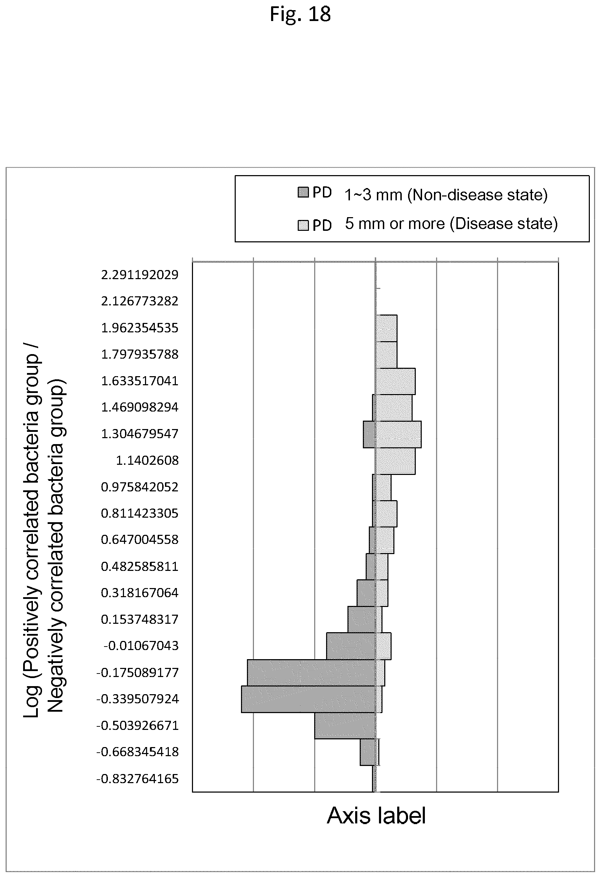

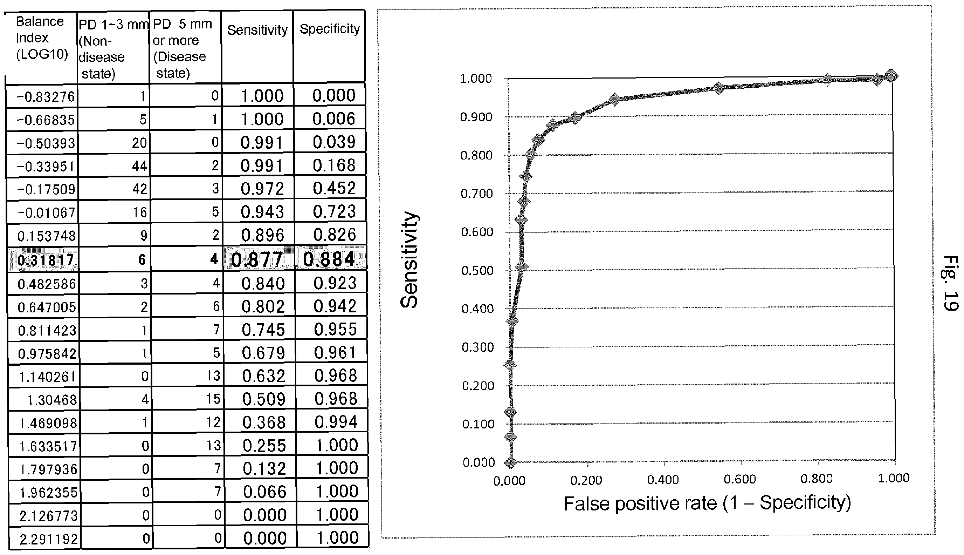

A histogram was created for the data with a periodontal pocket depth of 1 mm to 3 mm and the data with a periodontal pocket depth of 5 mm or more from the data shown in Figure 17 (Figure 18). The vertical axis of Figure 18 represents a value obtained by converting the balance index of Figure 17 with LOG10. The horizontal axis represents frequency.
ROC analysis was performed based on the data in Figure 18 (Figure 19), and the point near the upper left (balance index (LOG10) = 0.3182) was taken as the cut-off value. In this case, it was found from the analysis that a test was performed with a sensitivity of 0.877 and a specificity of 0.884.

Next, using this test, data with a periodontal pocket depth (Pd) of 4 mm were determined. The 4-mm data were the data with a pocket depth of 4 mm in Figure 17, and there were data for 60 individuals. When these data were determined with a cut-off value of 0.3182, 31 subjects had a balance index value larger than the cut-off value. It was determined that these subjects had a periodontal disease state as advanced as the disease state with a periodontal pocket depth of 5 mm or more.

### Sequence Listing Free Text

SEQ ID NOS: 1 to 91: Synthetic DNAs

### Sequence Listing

SEQUENCE LISTING

<110>  Mitsubishi Chemical Corporation

<120>  Intraoral examination method utilizing information on bacterial
       community related to clinical indicators

<130>  G1908WO

<150>  JP2017-212488
<151>  2017-11-02

<160>  91

<170>  PatentIn version 3.5

<210>  1
<211>  20
<212>  DNA
<213>  Artificial

<220>
<223>  Synthetic DNA

<400>  1
ttcaatgcaa tactcgtatc                                                   20


<210>  2
<211>  20
<212>  DNA
<213>  Artificial

<220>
<223>  Synthetic DNA

<400>  2
cacgtatctc attttattcc                                                   20


<210>  3
<211>  21
<212>  DNA
<213>  Artificial

<220>
<223>  Synthetic DNA

<400>  3
cctcttcttc ttattcttca t                                                 21


<210>  4
<211>  20
<212>  DNA
<213>  Artificial

<220>
<223>  Synthetic DNA

<400>  4
ctcttcttct tattcttcat                                                   20

<210>  5
<211>  18
<212>  DNA
<213>  Artificial

<220>
<223>  Synthetic DNA

<400>  5
gccttcgcaa taggtatt                                                    18


<210>  6
<211>  20
<212>  DNA
<213>  Artificial

<220>
<223>  Synthetic DNA

<400>  6
attctttccc aagaaaagga                                                  20


<210>  7
<211>  20
<212>  DNA
<213>  Artificial

<220>
<223>  Synthetic DNA

<400>  7
gtcataattc tttcccaaga                                                  20


<210>  8
<211>  20
<212>  DNA
<213>  Artificial

<220>
<223>  Synthetic DNA

<400>  8
caatgggtat tcttcttgat                                                  20


<210>  9
<211>  20
<212>  DNA
<213>  Artificial

<220>
<223>  Synthetic DNA

<400>  9
tagttataca gtttccaacg                                                  20


<210>  10
<211>  19

<212>  DNA
<213>  Artificial

<220>
<223>  Synthetic DNA

<400>  10
ccagtactct agttacaca                                                    19


<210>  11
<211>  20
<212>  DNA
<213>  Artificial

<220>
<223>  Synthetic DNA

<400>  11
tttctttctt cccaactgaa                                                   20


<210>  12
<211>  20
<212>  DNA
<213>  Artificial

<220>
<223>  Synthetic DNA

<400>  12
tacattccga aaaacgtcat                                                   20


<210>  13
<211>  19
<212>  DNA
<213>  Artificial

<220>
<223>  Synthetic DNA

<400>  13
tatgcagttt ccaacgcaa                                                    19


<210>  14
<211>  19
<212>  DNA
<213>  Artificial

<220>
<223>  Synthetic DNA

<400>  14
gggtaaatgc aaaaaggca                                                    19


<210>  15
<211>  20
<212>  DNA
<213>  Artificial

<220>
<223>  Synthetic DNA

<400>  15
ctttattccc acataaaagc                                                    20


<210>  16
<211>  18
<212>  DNA
<213>  Artificial

<220>
<223>  Synthetic DNA

<400>  16
aagtaccgtc actgtgtg                                                      18


<210>  17
<211>  20
<212>  DNA
<213>  Artificial

<220>
<223>  Synthetic DNA

<400>  17
gtcaatttgg catgctatta                                                    20


<210>  18
<211>  18
<212>  DNA
<213>  Artificial

<220>
<223>  Synthetic DNA

<400>  18
cccaagcagt tctatggt                                                      18


<210>  19
<211>  20
<212>  DNA
<213>  Artificial

<220>
<223>  Synthetic DNA

<400>  19
tacacgtaca ccttattctt                                                    20


<210>  20
<211>  19
<212>  DNA
<213>  Artificial

<220>
<223>  Synthetic DNA

<400>  20
caaccattca agaccaaca                                                    19


<210>  21
<211>  20
<212>  DNA
<213>  Artificial

<220>
<223>  Synthetic DNA

<400>  21
tacacgtaca ccttattctt                                                   20


<210>  22
<211>  19
<212>  DNA
<213>  Artificial

<220>
<223>  Synthetic DNA

<400>  22
ctctagctat ccagttcag                                                    19


<210>  23
<211>  19
<212>  DNA
<213>  Artificial

<220>
<223>  Synthetic DNA

<400>  23
cacccgttct tctcttaca                                                    19


<210>  24
<211>  18
<212>  DNA
<213>  Artificial

<220>
<223>  Synthetic DNA

<400>  24
cacccgttct tctcttac                                                     18


<210>  25
<211>  21
<212>  DNA
<213>  Artificial

<220>
<223>  Synthetic DNA

<400>  25
acagtatgaa ctttccattc t                                                 21

<210>  26
<211>  20
<212>  DNA
<213>  Artificial

<220>
<223>  Synthetic DNA

<400>  26
cagtatgaac tttccattct                                                    20


<210>  27
<211>  18
<212>  DNA
<213>  Artificial

<220>
<223>  Synthetic DNA

<400>  27
tctcccctct tgcactca                                                      18


<210>  28
<211>  19
<212>  DNA
<213>  Artificial

<220>
<223>  Synthetic DNA

<400>  28
tcccctcttg cactcaagt                                                     19


<210>  29
<211>  17
<212>  DNA
<213>  Artificial

<220>
<223>  Synthetic DNA

<400>  29
aagtcagccc gtaccca                                                       17


<210>  30
<211>  18
<212>  DNA
<213>  Artificial

<220>
<223>  Synthetic DNA

<400>  30
tattcgcaag aaggcctt                                                      18


<210>  31
<211>  17

<212>  DNA
<213>  Artificial

<220>
<223>  Synthetic DNA

<400>  31
ccacccacaa ggagcag                                                    17


<210>  32
<211>  18
<212>  DNA
<213>  Artificial

<220>
<223>  Synthetic DNA

<400>  32
ttcgcattag gcacgttc                                                   18


<210>  33
<211>  18
<212>  DNA
<213>  Artificial

<220>
<223>  Synthetic DNA

<400>  33
cacacgttct tgacttac                                                   18


<210>  34
<211>  23
<212>  DNA
<213>  Artificial

<220>
<223>  Synthetic DNA

<400>  34
cgtattaccg cggctgctgg cac                                             23


<210>  35
<211>  30
<212>  DNA
<213>  Artificial

<220>
<223>  Synthetic DNA

<400>  35
ctattcgacc agcgatatca ctacgtaggc                                      30


<210>  36
<211>  488
<212>  DNA
<213>  Artificial

<220>
<223>  Synthetic DNA

<400>  36
gtgagaagcc tacacaaacg taacgtcagg gctaagacaa acgctaacgg tacaccctag      60

atgggagctt gtagctagat cgctaagtcc taccgacatg taggcatact cacgaaggca     120

attccctgaa agcctcgtct tatcccgaac ttggcatctg ctgatacgtc aggttgaacg     180

cgtacattta cctgtcatgc gtgggccttc tccgaatagc ctacgtagtg atatcgctgg     240

tcgaataggc ggattgctca taaatgcaca ttggctaagg cccacggaac acgaatcacg     300

tgagatcact tactattcga cggaactact atacgcaccg ggacatgcaa gtagcgtccc     360

acaagcataa ggaactctat actcgccatc tacgcagcta caggggatac acgtatgagc     420

ggttacgaag taaagccgag atagagcggt ctttagagaa aaaacaggat tagataccct     480

ggtagtcc                                                              488


<210>  37
<211>  19
<212>  DNA
<213>  Artificial

<220>
<223>  Synthetic DNA

<400>  37
tcctacggga ggcagcagt                                                   19


<210>  38
<211>  26
<212>  DNA
<213>  Artificial

<220>
<223>  Synthetic DNA

<400>  38
cagggtatct aatcctgttt gctacc                                           26


<210>  39
<211>  25
<212>  DNA
<213>  Artificial

<220>
<223>  Synthetic DNA

<400>  39
gagaagccta cacaaacgta acgtc                                            25


<210>  40
<211>  23
<212>  DNA
<213>  Artificial

<220>
<223>  Synthetic DNA

<400>  40
ctctaaagac cgctctatct cgg                                              23


<210>  41
<211>  19
<212>  DNA
<213>  Artificial

<220>
<223>  Synthetic DNA

<400>  41
tcctacggga ggcagcagt                                                   19


<210>  42
<211>  18
<212>  DNA
<213>  Artificial

<220>
<223>  Synthetic DNA

<400>  42
tcctacggga ggcagcag                                                    18


<210>  43
<211>  17
<212>  DNA
<213>  Artificial

<220>
<223>  Synthetic DNA

<400>  43
tcctacggga ggcagca                                                     17


<210>  44
<211>  16
<212>  DNA
<213>  Artificial

<220>
<223>  Synthetic DNA

<400>  44
tcctacggga ggcagc                                                      16


<210>  45
<211>  15
<212>  DNA
<213>  Artificial

<220>
<223>  Synthetic DNA

<400>  45
cctacgggag gcagc                                                        15


<210>  46
<211>  16
<212>  DNA
<213>  Artificial

<220>
<223>  Synthetic DNA

<400>  46
ctacgggagg cagcag                                                       16


<210>  47
<211>  15
<212>  DNA
<213>  Artificial

<220>
<223>  Synthetic DNA

<400>  47
tacgggaggc agcag                                                        15


<210>  48
<211>  26
<212>  DNA
<213>  Artificial

<220>
<223>  Synthetic DNA

<400>  48
ggactaccag ggtatctaat cctgtt                                            26


<210>  49
<211>  26
<212>  DNA
<213>  Artificial

<220>
<223>  Synthetic DNA

<400>  49
cagggtatct aatcctgttt gctacc                                            26


<210>  50
<211>  21
<212>  DNA
<213>  Artificial

<220>
<223>  Synthetic DNA

<400>  50
cagggtatct aatcctgtty g                                                 21

<210>  51
<211>  19
<212>  DNA
<213>  Artificial

<220>
<223>  Synthetic DNA

<400>  51
cagggtatct aatcctgtt                                                    19


<210>  52
<211>  17
<212>  DNA
<213>  Artificial

<220>
<223>  Synthetic DNA

<400>  52
gggtatctaa tccygtt                                                      17


<210>  53
<211>  19
<212>  DNA
<213>  Artificial

<220>
<223>  Synthetic DNA

<400>  53
crgggtatct aatccygtt                                                    19


<210>  54
<211>  22
<212>  DNA
<213>  Artificial

<220>
<223>  Synthetic DNA

<400>  54
cctacgctta cttaaccacc ta                                                22


<210>  55
<211>  20
<212>  DNA
<213>  Artificial

<220>
<223>  Synthetic DNA

<400>  55
gtgcttaatg aggttaagcc                                                   20


<210>  56

<211>  22
<212>  DNA
<213>  Artificial

<220>
<223>  Synthetic DNA

<400>  56
cccctactac agagttttac ga                                                22


<210>  57
<211>  20
<212>  DNA
<213>  Artificial

<220>
<223>  Synthetic DNA

<400>  57
tacacacgtt cttcccctac                                                   20


<210>  58
<211>  20
<212>  DNA
<213>  Artificial

<220>
<223>  Synthetic DNA

<400>  58
acacgtgact cttgttattc                                                   20


<210>  59
<211>  21
<212>  DNA
<213>  Artificial

<220>
<223>  Synthetic DNA

<400>  59
cgtcaaatcc tcgcactatt c                                                 21


<210>  60
<211>  20
<212>  DNA
<213>  Artificial

<220>
<223>  Synthetic DNA

<400>  60
agttaacgtc aatcacctag                                                   20


<210>  61
<211>  21
<212>  DNA
<213>  Artificial

<220>
<223>  Synthetic DNA

<400>  61
ttcccaacta aaagcagttt a                                                21


<210>  62
<211>  18
<212>  DNA
<213>  Artificial

<220>
<223>  Synthetic DNA

<400>  62
ctggtaagtt accgtcac                                                    18


<210>  63
<211>  20
<212>  DNA
<213>  Artificial

<220>
<223>  Synthetic DNA

<400>  63
gcgcttcata acccggctac                                                  20


<210>  64
<211>  22
<212>  DNA
<213>  Artificial

<220>
<223>  Synthetic DNA

<400>  64
cacgtgcatc aaattattct cg                                               22


<210>  65
<211>  20
<212>  DNA
<213>  Artificial

<220>
<223>  Synthetic DNA

<400>  65
cctactttca gcgcactcaa                                                  20


<210>  66
<211>  20
<212>  DNA
<213>  Artificial

<220>
<223>  Synthetic DNA

<400>  66
cacgtgactg actttatccc                                                   20


<210>  67
<211>  20
<212>  DNA
<213>  Artificial

<220>
<223>  Synthetic DNA

<400>  67
ccaacaattt aaccacttac                                                   20


<210>  68
<211>  20
<212>  DNA
<213>  Artificial

<220>
<223>  Synthetic DNA

<400>  68
aatagggaca cgtccctaac                                                   20


<210>  69
<211>  22
<212>  DNA
<213>  Artificial

<220>
<223>  Synthetic DNA

<400>  69
gtaccgtcac ccaaactcaa ta                                                22


<210>  70
<211>  20
<212>  DNA
<213>  Artificial

<220>
<223>  Synthetic DNA

<400>  70
acccactgca aaaccagggt                                                   20


<210>  71
<211>  20
<212>  DNA
<213>  Artificial

<220>
<223>  Synthetic DNA

<400>  71
tctcttcttc cctgctaaca                                                   20

<210>  72
<211>  22
<212>  DNA
<213>  Artificial

<220>
<223>  Synthetic DNA

<400>  72
accgtcaatt cctctaacta tt                                                22


<210>  73
<211>  20
<212>  DNA
<213>  Artificial

<220>
<223>  Synthetic DNA

<400>  73
accaccgact tgaaggacca                                                   20


<210>  74
<211>  20
<212>  DNA
<213>  Artificial

<220>
<223>  Synthetic DNA

<400>  74
agtcagacgt tgggcgccta                                                   20


<210>  75
<211>  22
<212>  DNA
<213>  Artificial

<220>
<223>  Synthetic DNA

<400>  75
tacatgcatc tcagctacac gt                                                22


<210>  76
<211>  20
<212>  DNA
<213>  Artificial

<220>
<223>  Synthetic DNA

<400>  76
cacactcgtt cttgacttac                                                   20


<210>  77

<211>  20
<212>  DNA
<213>  Artificial

<220>
<223>  Synthetic DNA

<400>  77
aaaaagcagt gccttgttcc                                                    20


<210>  78
<211>  21
<212>  DNA
<213>  Artificial

<220>
<223>  Synthetic DNA

<400>  78
gtcgattacc gtcatcagat g                                                  21


<210>  79
<211>  20
<212>  DNA
<213>  Artificial

<220>
<223>  Synthetic DNA

<400>  79
ttcctccaaa acttattcct                                                    20


<210>  80
<211>  20
<212>  DNA
<213>  Artificial

<220>
<223>  Synthetic DNA

<400>  80
ccgtctctac tgtatatagt                                                    20


<210>  81
<211>  22
<212>  DNA
<213>  Artificial

<220>
<223>  Synthetic DNA

<400>  81
ggtacattca ctatggtaca cg                                                 22


<210>  82
<211>  22
<212>  DNA
<213>  Artificial

<220>
<223>  Synthetic DNA

<400>  82
tcttaacaaa ggtaccgtca cc                                              22


<210>  83
<211>  20
<212>  DNA
<213>  Artificial

<220>
<223>  Synthetic DNA

<400>  83
ccctaggaca gaggcttaca                                                 20


<210>  84
<211>  21
<212>  DNA
<213>  Artificial

<220>
<223>  Synthetic DNA

<400>  84
agctgtcgat attagcaaca g                                               21


<210>  85
<211>  20
<212>  DNA
<213>  Artificial

<220>
<223>  Synthetic DNA

<400>  85
gtcaaggcgc taacagttac                                                 20


<210>  86
<211>  20
<212>  DNA
<213>  Artificial

<220>
<223>  Synthetic DNA

<400>  86
aaaccctgcg cttaaggtgc                                                 20


<210>  87
<211>  20
<212>  DNA
<213>  Artificial

<220>
<223>  Synthetic DNA

<400>  87
taaccacaag attattcgtc                                                    20


<210>  88
<211>  20
<212>  DNA
<213>  Artificial

<220>
<223>  Synthetic DNA

<400>  88
acgtgggctc ttttatcccc                                                    20


<210>  89
<211>  20
<212>  DNA
<213>  Artificial

<220>
<223>  Synthetic DNA

<400>  89
cgtcattcgt cttctgccaa                                                    20


<210>  90
<211>  15
<212>  DNA
<213>  Artificial

<220>
<223>  Synthetic DNA

<400>  90
tacgggaggc agcag                                                         15


<210>  91
<211>  19
<212>  DNA
<213>  Artificial

<220>
<223>  Synthetic DNA

<400>  91
crgggtatct aatccygtt                                                     19

## Claims

1. An intraoral examination method for measuring a signal intensity of a nucleic acid from an oral bacterial group present in an oral sample, calculating an abundance of the bacterial group from a measured value of the signal intensity, and determining a state of periodontal disease using the obtained calculated value as an index, wherein
an abundance ratio of bacterial groups shows a correlation between a bacterial load of a bacterial species that increases as a periodontal pocket value increases and a bacterial load of a bacterial species that decreases as a periodontal pocket value increases.

2. The method according to claim 1, wherein the state of periodontal disease is determined by comparing the obtained calculated value with a cut-off value of the abundance ratio of bacterial groups.

3. The method according to claim 1, wherein the abundance ratio of bacterial groups is a ratio of the bacterial load of the bacterial species that increases as the periodontal pocket value increases and the bacterial load of the bacterial species that decreases as the periodontal pocket value increases.

4. The method according to claim 2, wherein the cut-off value is determined based on an ROC curve created from a calculated value obtained by calculating the abundance ratio of bacterial groups from the measured value of the signal intensity of the nucleic acid from the oral bacterial group present in the oral sample for standardization.

5. The method according to claim 1, wherein the abundance ratio of bacterial groups shows a correlation between the bacterial load of Fusobacterium nucleatum species and the bacterial load of the bacterial species that decreases as the periodontal pocket value increases.

6. The method according to claim 1, wherein the following (a) and (b) are used as the abundance ratio of bacterial groups:
(a) a correlation between the bacterial load of the bacterial species that increases as the periodontal pocket value increases (including at least one bacterial species other than Fusobacterium nucleatum species) and the bacterial load of the bacterial species that decreases as the periodontal pocket value increases; and
(b) a correlation between the bacterial load of Fusobacterium nucleatum species and the bacterial load of the bacterial species that decreases as the periodontal pocket value increases.

7. The method according to claim 1, wherein the bacterial species that increases as the periodontal pocket value increases is at least one selected from the group consisting of Porphyromonas gingivalis, Tannerella forsythia, Treponema denticola, Campylobacter gracilis, Campylobacter rectus, Campylobacter showae, Fusobacterium nucleatum subsp. vincentii, Fusobacterium nucleatum subsp. polymorphum, Fusobacterium nucleatum subsp. animalis, Fusobacterium nucleatum subsp. nucleatum, Fusobacterium periodonticum, Prevotella intermedia, Streptococcus constellatus, Aggregatibacter actinomycetemcomitans, Eikenella corrodens, Filifactor alocis, Porphyromonas endodontalis, Eubacterium nodatum , Eubacterium saphenum, Treponema medium, and Selenomonas sputigena.

8. The method according to claim 1, wherein bacterial species that decreases as the periodontal pocket value increases is at least one selected from the group consisting of Prevotella nigrescens, Campylobacter concisus, Capnocytophaga gingivalis, Capnocytophaga ochracea, Capnocytophaga sputigena, Streptococcus gordonii, Streptococcus intermedius, Streptococcus mitis, Streptococcus mitis bv 2, Actinomyces odontolyticus, Veillonella parvula, Actinomyces naeslundii II, Selenomonas noxia, Prevotella denticola, Prevotella melaninogenica, Gemella sanguinis, Eubacterium sulci, Corynebacterium matruchotii, Rothia mucilaginosa, Porphyromonas catoniae, Solobacterium moorei, Neisseria flavescens, Prevotella loescheii, Megasphaera micronuciformis, Actinomyces graevenitzii, Veillonella atypica, Prevotella pallens, Prevotella shahii, Porphyromonas pasteri, Veillonella rogosae, Alloprevotella spp. (A. rava, OT 308), Rothia dentocariosa, Granulicatella adiacens, Streptococcus salivarius, Haemophilus parainfluenzae, and Streptococcus parasanguinis.

9. The method according to claim 5, wherein the Fusobacterium nucleatum species is at least one selected from the group consisting of Fusobacterium nucleatum subsp. vincentii, Fusobacterium nucleatum subsp. polymorphum, Fusobacterium nucleatum subsp. animalis, and Fusobacterium nucleatum subsp. nucleatum.
